(19) **Europäisches Patentamt**
**European Patent Office**
**Office européen des brevets**

(11) **EP 4 491 723 A1**

(12) **EUROPEAN PATENT APPLICATION**
published in accordance with Art. 153(4) EPC

(43) Date of publication:
**15.01.2025 Bulletin 2025/03**

(21) Application number: 23765988.3

(22) Date of filing: **07.03.2023**

(51) International Patent Classification (IPC):
**C12N 9/78** (2006.01)　　**C07K 19/00** (2006.01)
**C12N 15/113** (2010.01)　　**C12N 7/00** (2006.01)
**C12N 5/10** (2006.01)　　**C12N 15/79** (2006.01)

(52) Cooperative Patent Classification (CPC):
**A61K 38/50; A61K 48/00; A61P 1/00; A61P 1/16;**
**A61P 3/10; A61P 7/06; A61P 9/00; A61P 11/00;**
**A61P 21/00; A61P 25/00; A61P 25/08; A61P 25/18;**
**A61P 25/28; A61P 25/30; A61P 27/02;**　　(Cont.)

(86) International application number:
**PCT/CN2023/080052**

(87) International publication number:
**WO 2023/169410 (14.09.2023 Gazette 2023/37)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **08.03.2022　CN 202210220832**

(71) Applicant: **INSTITUTE OF GENETICS AND**
**DEVELOPMENTAL BIOLOGY,**
**CHINESE ACADEMY OF SCIENCES**
**Chaoyang District**
**Beijing 100101 (CN)**

(72) Inventors:
　• **GAO, Caixia**
　**Beijing 100101 (CN)**

　• **LIN, Qiupeng**
　**Beijing 100101 (CN)**
　• **HUANG, Jiaying**
　**Beijing 100101 (CN)**
　• **ZHAO, Kevin T·**
　**Beijing 102206 (CN)**

(74) Representative: **Michalski Hüttermann & Partner**
**Patentanwälte mbB**
**Kaistraße 16A**
**40221 Düsseldorf (DE)**

Remarks:
The complete document including Reference
Table(s) and the Sequence Listing(s) can be
downloaded from the EPO website

(54) **CYTOSINE DEAMINASE AND USE THEREOF IN BASE EDITING**

(57)　The present invention relates to the field of genetic engineering. Specifically, the present invention relates to cytosine deaminase and use thereof in base editing. More specifically, the present invention relates to a base editing system based on a newly identified cytosine deaminase, a method for base editing a target sequence in the genome of an organism (e.g., a plant) using the base editing system, and a genetically modified organism (e.g., a plant) produced by the method and progenies thereof.

EP 4 491 723 A1

(52) Cooperative Patent Classification (CPC): (Cont.)
**A61P 27/16; A61P 31/22; A61P 35/00;**
**A61P 39/02; C07K 19/00; C12N 5/10; C12N 7/00;**
**C12N 9/14; C12N 9/22; C12N 9/78; C12N 15/10;**
**C12N 15/113; C12N 15/62; C12N 15/79;**
**C12N 15/82; C12N 15/864; G16B 15/20;**
**G16B 20/00**

# EP 4 491 723 A1

## Description

### Technical field

The present invention relates to the field of genetic engineering. Specifically, the present invention relates to cytosine deaminase and use thereof in base editing. More specifically, the present invention relates to a base editing system based on a newly identified cytosine deaminase, a method for base editing a target sequence in the genome of an organism (e.g., a plant) using the base editing system, and a genetically modified organism (e.g., a plant) produced by the method and progenies thereof.

### Background

Sequence-specific modifications to an organism's genome can confer new, stably heritable traits to the organism. Among them, single nucleotide variation at a specific site may lead to changes in the amino acid sequence of the gene or early termination, or may lead to changes in the regulatory sequence, thereby leading to the production of elite traits. Genome editing technologies, such as the CRISPR/Cas9 system, can achieve the function of targeting genome target sequences. The base editing system developed by taking advantage of the ability of the genome editing system to bind to target sequence and its combination with deaminase, can accurately deaminate target nucleotides on the genome. Among them, the cytosine base editing system can achieve the conversion of cytosine (C) to uracil (U) at the target site by fusion with APOBEC/AID family and APOBEC/AID family-like deaminase, and then conversion of cytosine to thymine (T) is achieved through related repair pathways in the cell. In addition, the efficiency of base editing can be significantly improved by introducing a nick into the single strand that has not undergone deamination on the opposite side to allow its cleavage.

Based on the structural comparison of deaminases, Iyer *et al.* searched for proteins with potential deamination functions and classified the proteins into at least 20 clades (Iyer, L. M., Zhang, D., Rogozin, I. B., & Aravind, L. (2011). Evolution of the deaminase fold and multiple origins of eukaryotic editing and mutagenic nucleic acid deaminases from bacterial toxin systems. Nucleic acids research, 39(22), 9473-9497.). They found that the deaminases of different clades were very different in structure and sequence. Among them, the functions of some clades have been resolved, including the "dCMP deaminase and ComE" clade that can convert dCMP into dUMP, the "Guanine deaminase" clade that can convert guanine (G) into xanthine (I), the "RibD-like" clade with diaminohydroxyphosphoribosylamidopyrimidine deaminase function, the "Tad1/ADAR" clade with RNA editing enzyme function that converts RNA adenine (A) into xanthine (I), and the "PurH/AICAR transformylase" clade with formyl transferase activity. However, the functions of some clades, such as whether they have deamination activity or what kind of substrates they can deaminize, have not yet been resolved or confirmed, such as the SCP1.201 clade, XOO2897 clade, MafB19 clade, and Pput_2613 clade from bacteria. Currently, only a few types of deaminases, including APOBEC1, APOBEC3, CDA, AID, CDA1L1 and CDA1L2, from the APOBEC/AID clade have been shown to act on single-stranded DNA and can therefore be applied to cytosine base editing systems.

There is still a need in the art for more deaminases that can be used in base editing systems, so as to expand the base editing systems and enhance the ability to precisely manipulate target DNA sequences.

### Brief description of the drawings

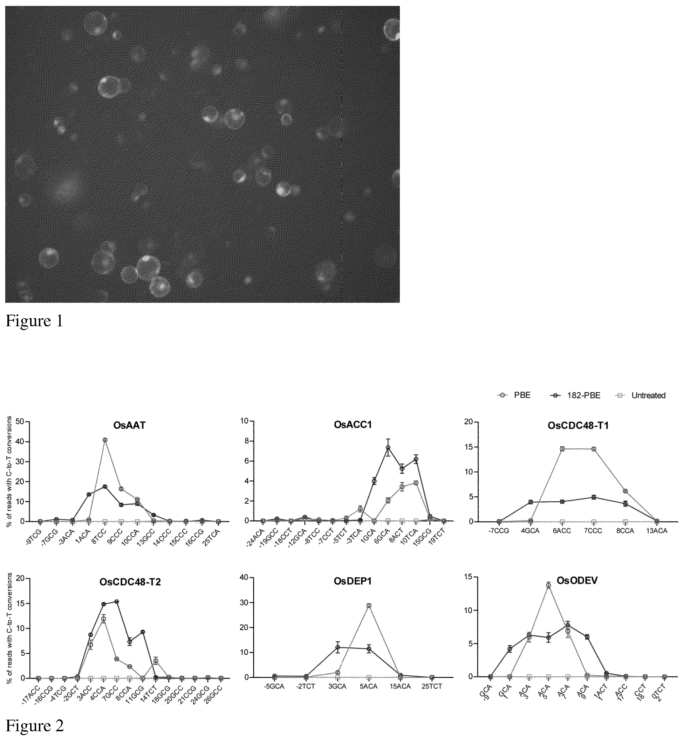

Figure 1: Potential deaminase No.182 (SEQ ID NO:1) in the APOBEC/AID clade achieved cytosine base editing in the reporter system.

Figure 2: Potential deaminase No.182 (SEQ ID NO:1) in the APOBEC/AID clade achieved cytosine base editing in the endogenous sites.

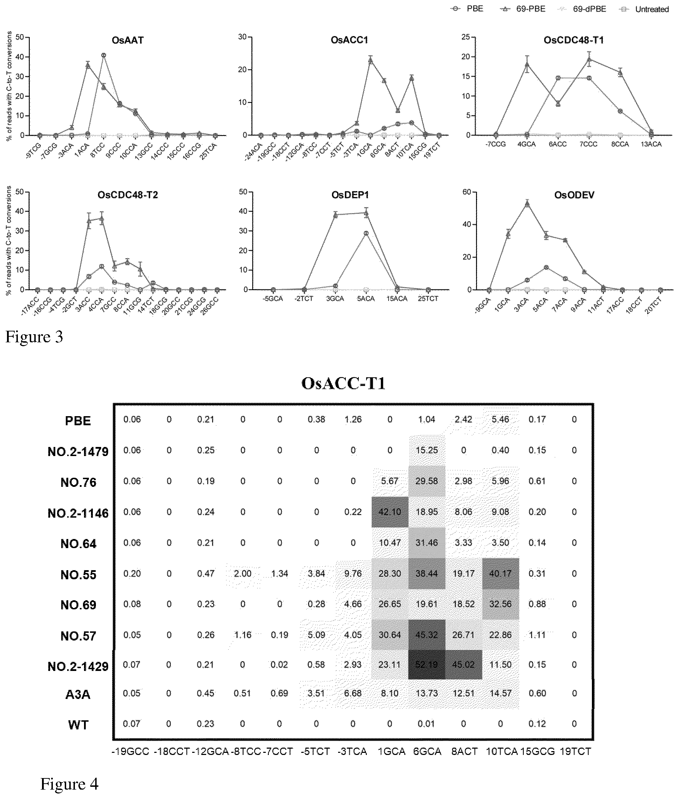

Figure 3: Potential deaminase No.69 (SEQ ID NO:2) in the SCP1.201 clade achieved cytosine base editing in the endogenous sites.

Figure 4: Cytosine base editing efficiency of 8 deaminases with high editing efficiency at the endogenous site of rice OsACC-T1.

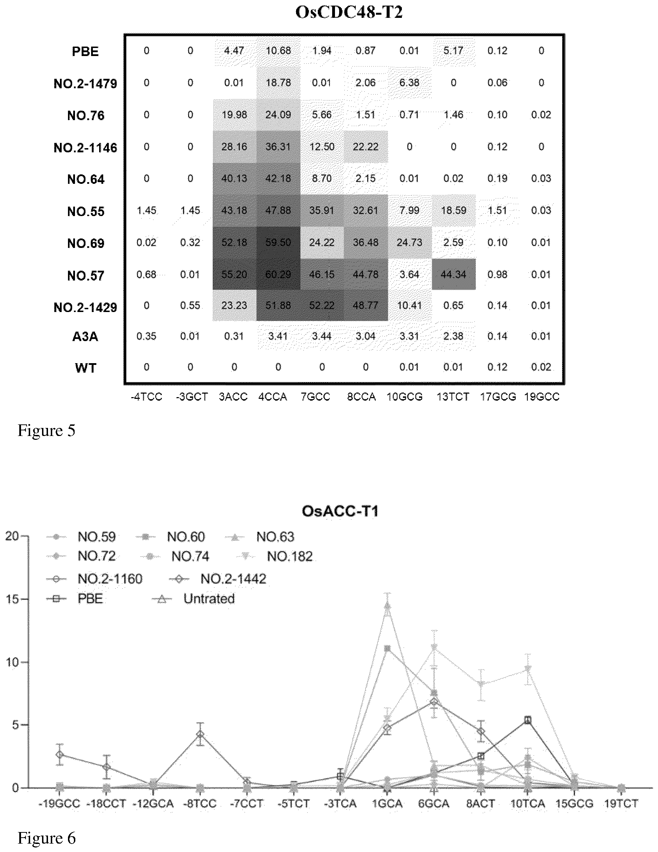

Figure 5: Cytosine base editing efficiency of 8 deaminases with high editing efficiency at the endogenous site of rice CDC48-T2.

Figure 6: Cytosine base editing efficiency of 8 deaminases with medium editing efficiency at the endogenous site of rice OsACC-T1.

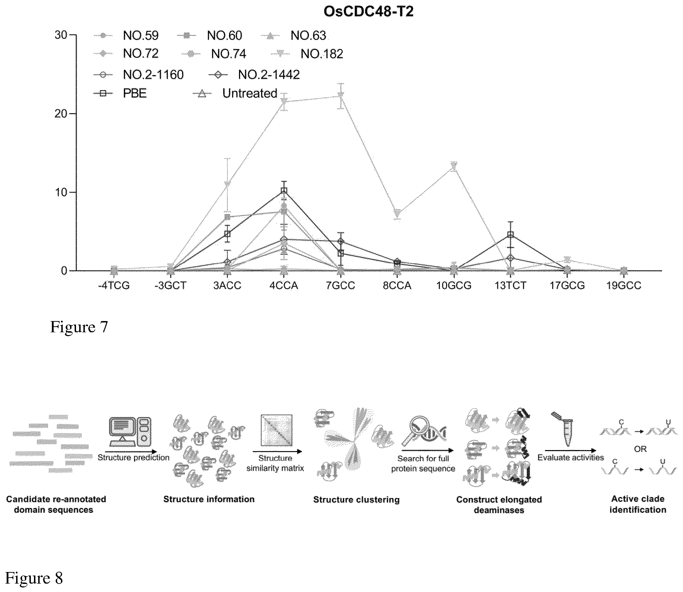

Figure 7: Cytosine base editing efficiency of 8 deaminases with medium editing efficiency at the endogenous site of rice CDC48-T2.

3

Figure 8: Protein clustering process based on AlphaFold2 predicted structures. AlphaFold2 was used to predict the structure of candidate sequences and then clustering was performed based on structural similarity. The cytosine deamination activity of proteins in each structural clade on ssDNA and dsDNA was then experimentally tested in plants and human cells.

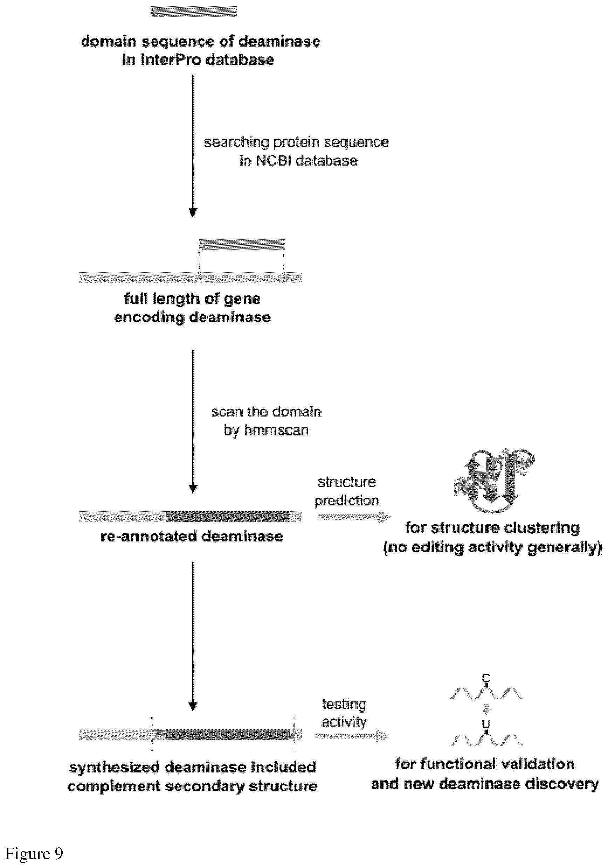

Figure 9: Re-annotation and synthesis process of candidate deaminases. We used Protein BLAST from the NCBI database (https://blast.ncbi.nlm.nih.gov/Blast.cgi) to obtain the full-length genes encoding deaminases, and then re-annotated the deaminase domain sequences using hmmscan (https://www.ebi.ac.uk/Tools/hmmer/search/hmmscan). The resulting domain sequences were used for structural classification. To confirm that they have deaminase activity, we synthesized some candidate deaminases with extended N-terminal and C-terminal sequences, and then evaluated their cytosine deaminase activity using a reporter system or at endogenous sites.

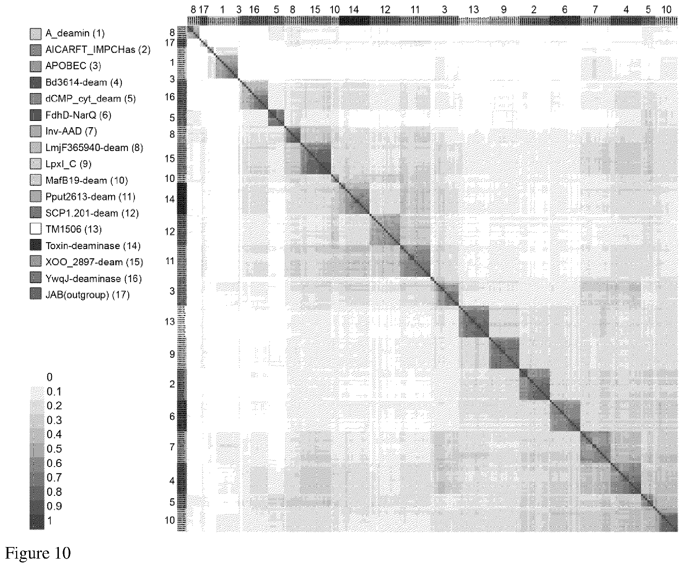

Figure 10: Structural similarity matrix, reflecting the similarity between 242 predicted protein structures of 16 deaminase families (238) and one outgroup JAB (4). Proteins from different families are distinguished by different numbers; the color depth of the heat map indicates the degree of similarity.

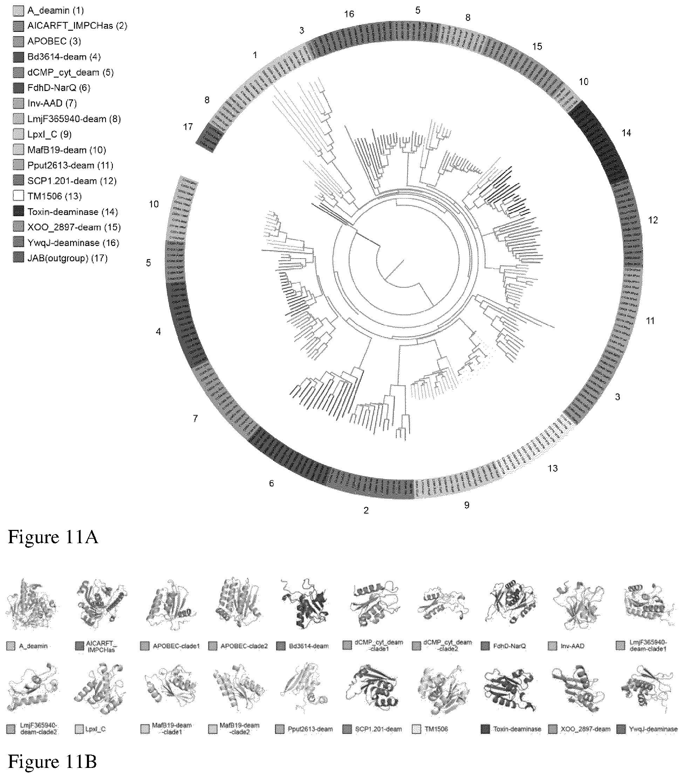

Figure 11: (A) Proteins are classified into different deaminase families based on protein structure, and different families are distinguished by different numbers; (B) Representative predicted structures of each of the 16 deaminase clades.

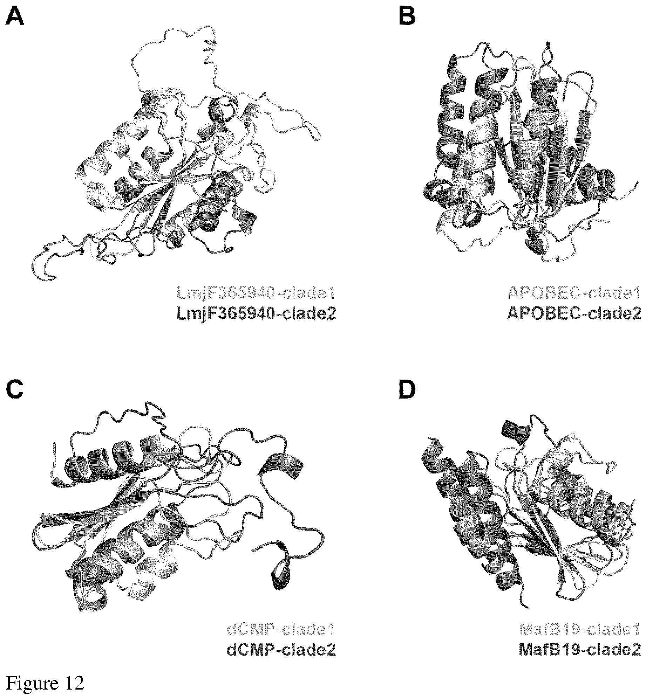

Figure 12: Alignment of representative structures of two clades of the LmjF365940, APOBEC, dCMP and MafB19 families corresponding to Figure 11. Although the two clades of each of these four families have partially similar structures, the overall structures of the two clades show relatively large differences, leading to their classification into different clades.

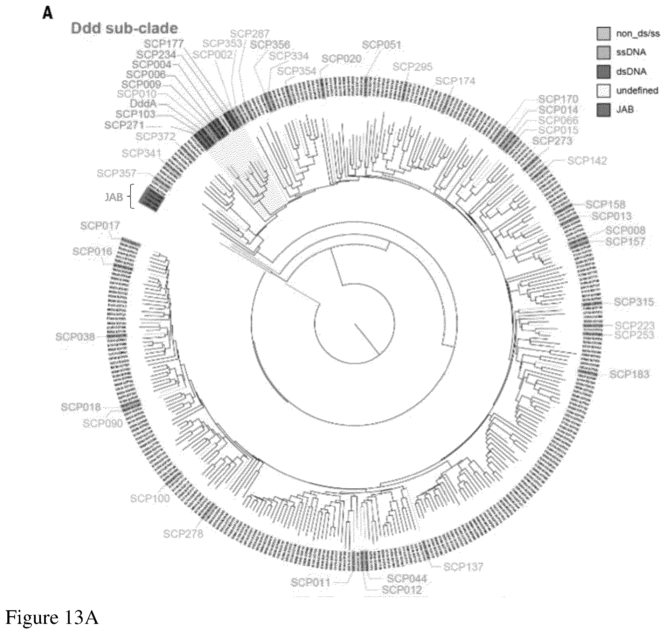

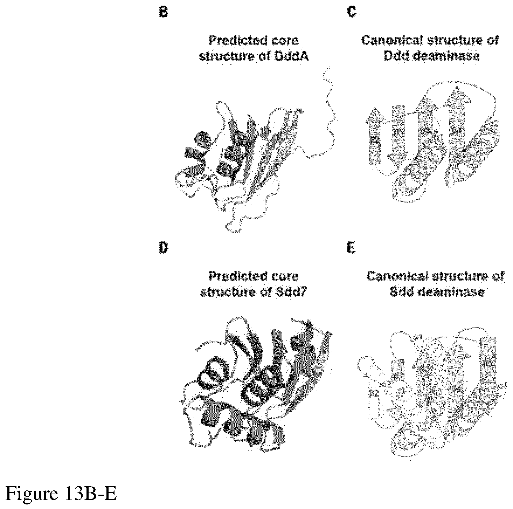

Figure 13: (A) Classification of SCP1.201 deaminases based on protein structure. The JAB family was considered as an outgroup, and the tested deaminases were shown as single-strand editing (ssDNA), double-strand editing (dsDNA), or no signle/double-stand editing (non-ds/ss) based on their functions. Deaminases in light grey are undefined and need further functional analysis. The deaminase domains with single-strand editing in the figure are: SCP356, SCP020, SCP051, SCP170, SCP014, SCP273, SCP158, SCP013, SCP008, SCP157, SCP315, SCP183, SCP044, SCP012, SCP011, SCP018, SCP038, SCP016, SCP017; the deaminase domains with double-strand editing are: SCP271, SCP103, SCP009, SCP006, SCP004, SCP234, SCP177; the other annotated deaminases are deaminases that have no editing. (B) The core structure of DddA predicted by AlphaFold2. (C) Typical structural features of Ddd protein (protein with double-strand deaminase activity). (D) The core structure of Sdd7 predicted by AlphaFold2. (E) Features of the typical structure of Sdd protein (protein with single-strand deaminase activity).

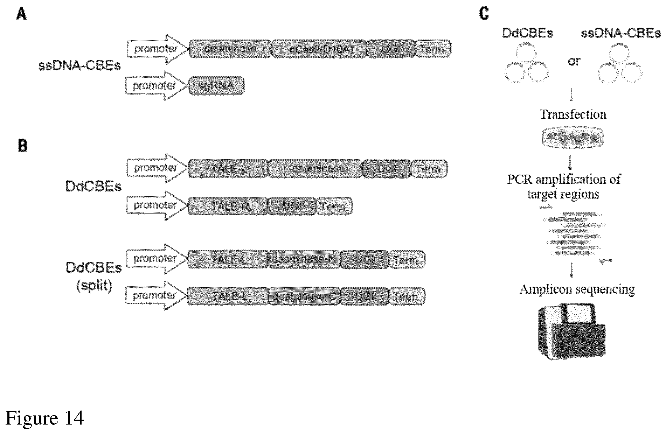

Figure 14: Identification of cytosine deamination activity of ssDNA and dsDNA at endogenous sites in animal cells. (A) Schematic diagram of ssDNA base editing vector for editing endogenous sites. (B) Schematic diagram of DdCBE vector and its split form. (C) Detecting the activity of DdCBEs on dsDNA as well as ssDNA CBEs on ssDNA in HEK293T cells, respectively, followed by high-throughput sequencing.

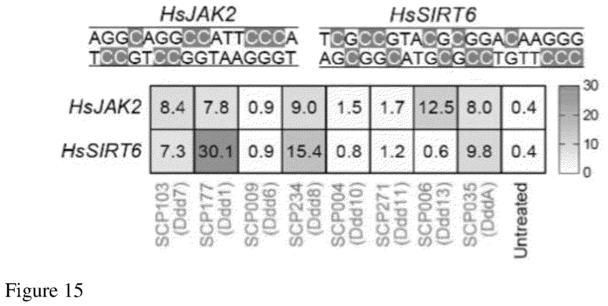

Figure 15: Experimental evaluation of dsDNA deamination activity of Ddd at two endogenous sites in HEK293T cells. For the base editing sites used for calculation, the color depth represents the editing efficiency.

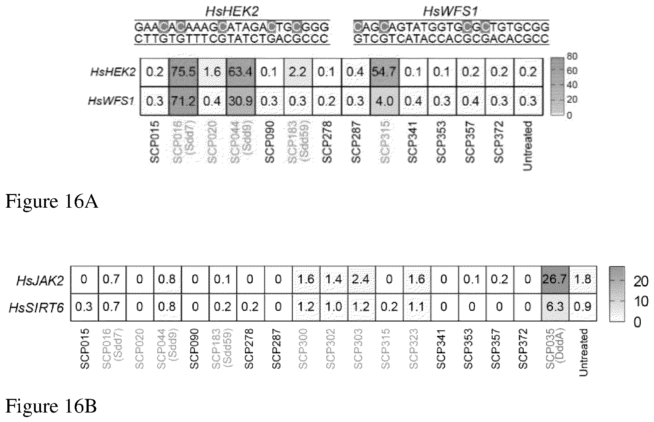

Figure 16: (A) Experimental evaluation of ssDNA deamination activity of Sdd at two endogenous sites in HEK293T cells. For the base editing sites used for calculation, the color depth represents the editing efficiency. (B) Experimental evaluation of ssDNA deaminase activity of Sdd at HsJAK2 and HsSIRT6 sites. Data are represented by the average of three independent experiments.

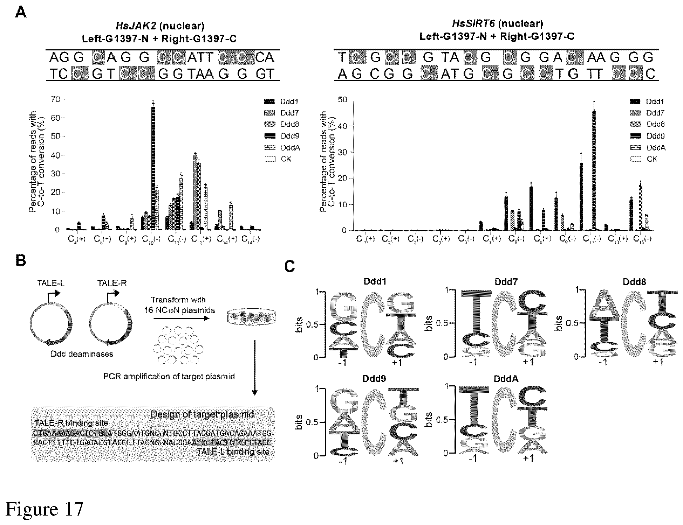

Figure 17: Evaluation of the editing properties of newly discovered Ddd proteins for use as base editors. (A) Editing efficiencies and editing windows of dsDNA deaminases Ddd1, Ddd7, Ddd8, Ddd9, and DddA of SCP1.201 at two genomic targets in HEK293T cells. (B) Plasmid library assay to profile context preferences of each Ddd protein in mammalian cells. Candidate proteins target and edit the "$NC_{10}N$" motif. (C) Sequence motif logos summarizing the context preferences of Ddd1,Ddd7, Ddd8, Ddd9, and DddA, as determined by the plasmid library assay. In the figure, dots represent individual biological replicates, bars represent mean values of editing efficiency, and error bars represent the SD of three independent biological replicates..

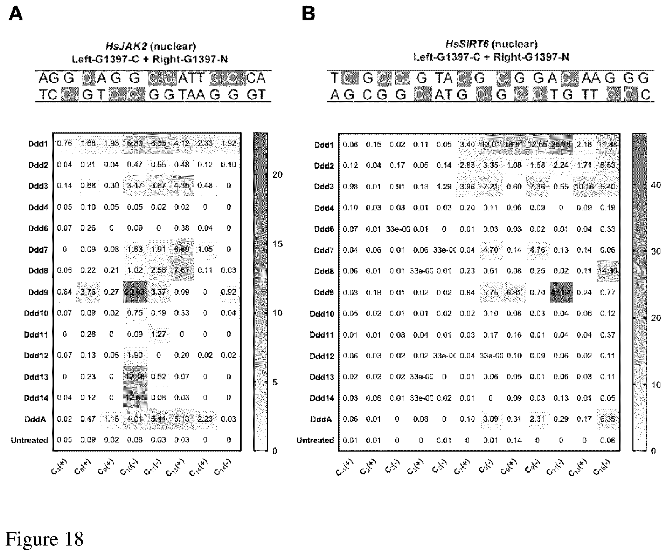

Figure 18: Heatmap of editing efficiencies and editing windows of SCP1.201 dsDNA deaminases at two target sites in HEK293T cells..

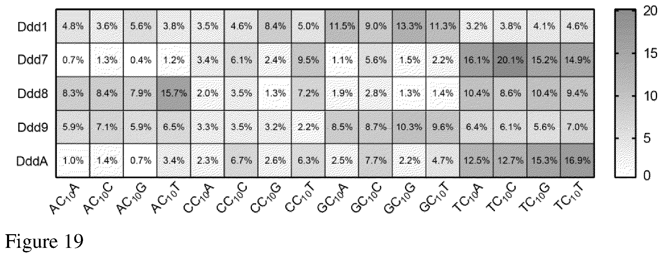

Figure 19: The proportion of editing efficiencies of each context preference among 16 plasmid libraries of different Ddds. Data are represented by the average of three independent experiments.

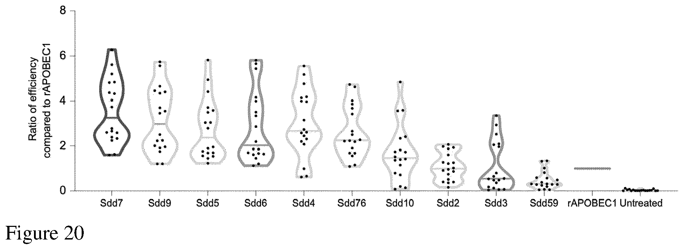

Figure 20: Evaluation of the newly discovered Sdd proteins as base editors in plants. Overall editing efficiency of ten Sdd proteins and rAPOBEC1 across six endogenous target sites in rice protoplasts. The average editing frequency of APOBEC1 at each target site was set to 1, and the editing efficiency observed for each Sdd was normalized accordingly.

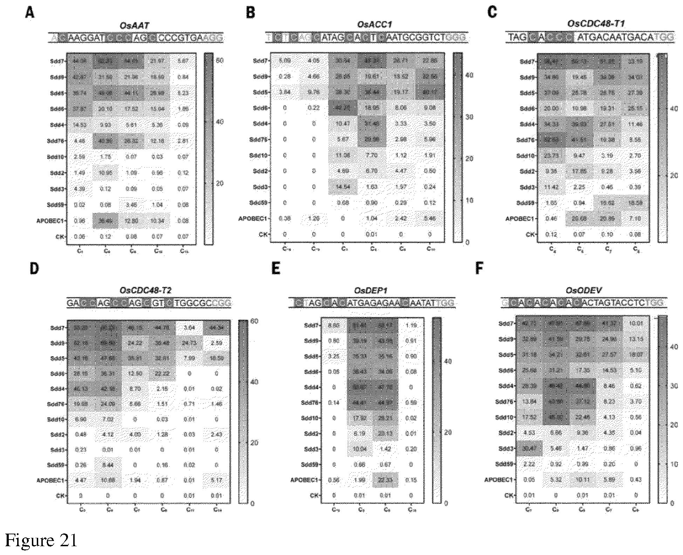

Figure 21: Editing behavior of Sdd deaminase and APOBEC1 at six endogenous target sites in rice protoplasts. (A-F) Heatmap shows the editing efficiencies and editing windows of 10 Sdd deaminase and APOBEC1 in rice protoplasts at OsAAT (A), OsACC1 (B), OsCDC48-T1 (C), OsCDC48-T2 (D), OsDEP1 (E), and OsODEV (F) sites. The values given in the heatmap cells represent C-to-T editing efficiencies., and the color depth represents the high and low editing efficiency. The target sequences are listed above the heatmap, the dark box marks the position of C-to-T editing, and the last three letters in light fonts represent PAMs. The data are represented by the average of three independent experiments.

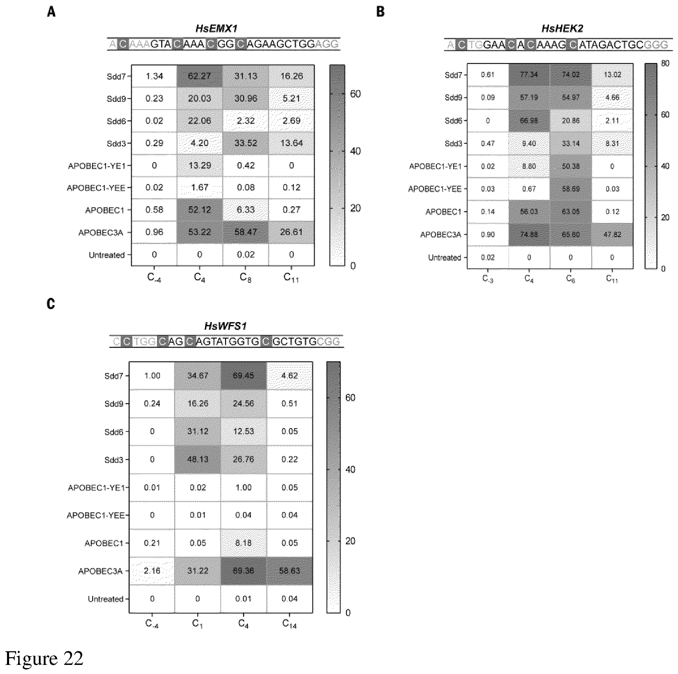

Figure 22: Editing behavior of SCP1.201 ssDNA deaminase and APOBEC deaminase at three endogenous target sites in HEK293T cells. (A-C) Heatmap shows the editing efficiencies and editing windows of four Sdd deaminases and APOBEC1, APOBEC3A, APOBEC1-YE1 and APOBEC1-YEE at HsEMX1 (A), HsHEK2 (B) and HsWFS1 (C) sites in HEK293T cells. The values given in the heatmap cells represent the C-to-T editing efficiency, and the color depth represents the editing efficiency. The target sequences are listed above the heatmap, the dark box marks the position of C-to-T editing, and the last three letter in light fonts mark PAM. The data are represented by the average of three independent experiments.

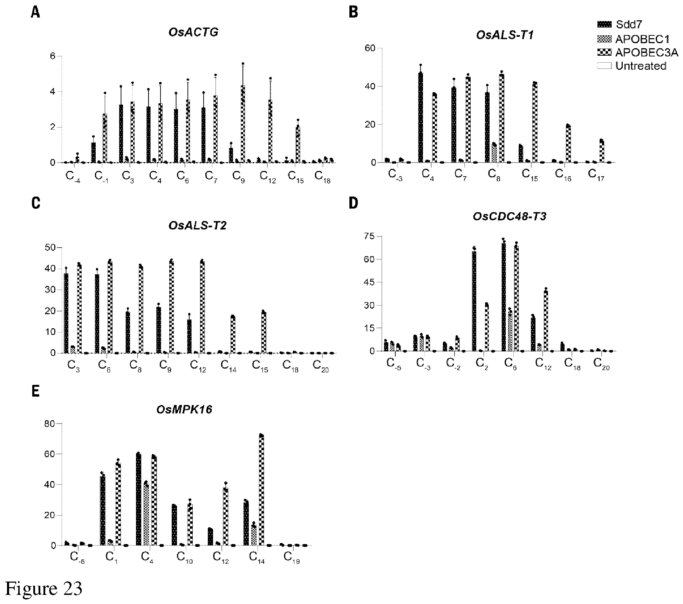

Figure 23: Comparison of the efficiencies of Sdd7, APOBEC1, and APOBEC3A at five sites in rice protoplasts. (A-E) The efficiency of Sdd7, APOBEC1 and APOBEC3A base editors at five endogenous target sites, (A) OsACTG, (B) OsALS-T1, (C) OsALS-T2, (D) OsCDC48-T3 and (E) OsMPK16 were compared. are represented by the average of three independent experiments, the bars represent the mean values of editing efficiency, and the error bars represent the standard deviation of three independent biological experiments.

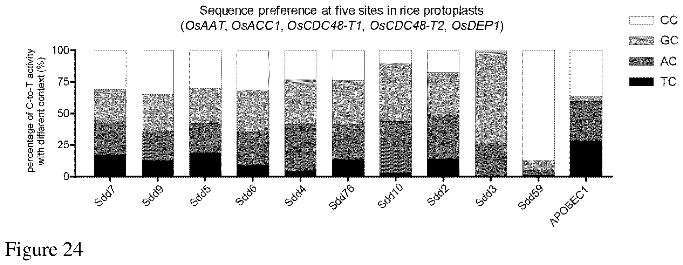

Figure 24: Sequence preferences of Sdd deaminases and APOBEC1 at five endogenous targets in rice protoplasts. The stacked graph shows the context preferences of 10 Sdd deaminases and APOBEC1 at five endogenous targets, OsAAT, OsACC1, OsCDC48-T1, OsCDC48-T2, and OsDEP1. The bars represent the C-to-T editing preferences of TC, AC, GC, and CC from bottom to top, respectively. The data are the results of three independent experiments.

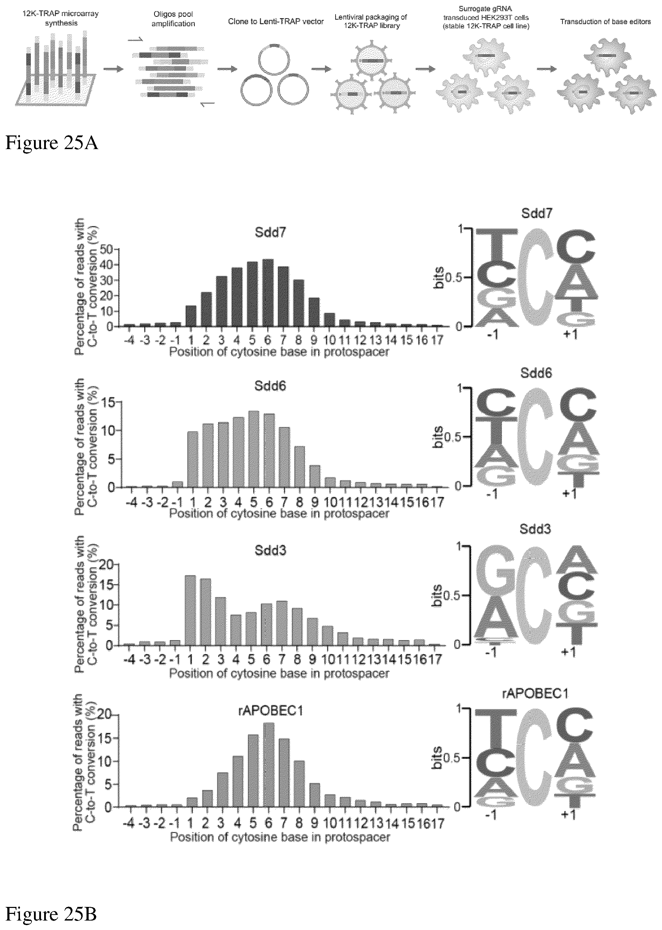

Figure 25: (A) Overview of high-throughput quantification of the activities and properties of Sdd and rAPOBEC1 in HEK293T cells using the 12K-TRAPseq library. (B) Evaluation of Sdd and rAPOBEC1 editing preferences and patterns by the 12K-TRAP library. The left shows the editing efficiencies and editing windows of the deaminases. The sequence motif logo on the right reflects the context preference of the deaminases.

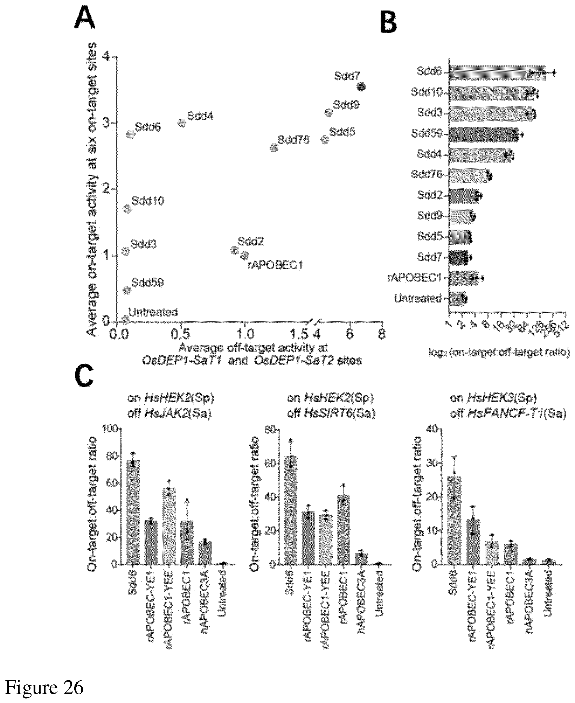

Figure 26: (A) Evaluation of off-target effects using an orthogonal R-loop assay in rice protoplasts. Dots represent the average frequency of on-target C-to-T conversions for each base editor at six rice target sites (Figure 20) and the frequency of off-target C-to-T conversions that were independent of sgRNAs at two ssDNAs (OsDEP1-SaT1 and OsDEP1-SaT2). (B) On-target:off-target editing ratios for each base editor in Figure 26A. (C) On-target:off-target editing ratios for Sdd6, rAPOBEC1-YE1, rAPOBEC1-YEE, rAPOBEC1, and hAPOBEC3A tested at two on-target and three off-target sites in HEK293T cells. Dots in the figure represent individual biological replicates, bars represent mean values, and error bars represent standard deviations for three independent biological replicates.

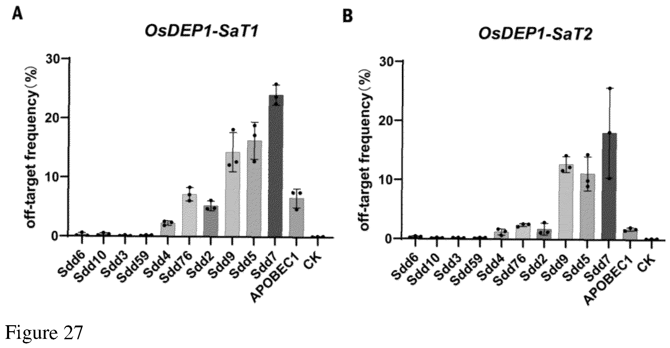

Figure 27: Specific off-target frequencies of Sdd deaminase and APOBEC1 at two endogenous targets in rice protoplasts (Figures 26A and 26B). Off-targets were assessed using an orthogonal R-loop assay. (A, B) Off-target frequencies of Sdd deaminase and APOBEC1 at OsDEP1-SaT1 (A) and OsDEP1-SaT2 (B) sites in rice protoplasts. Data are the results of three independent experiments.

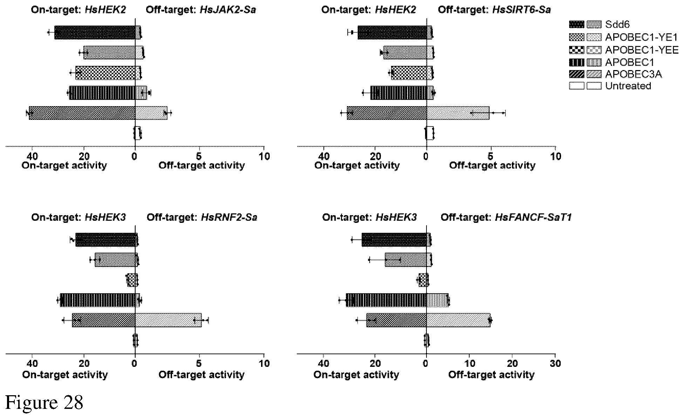

Figure 28: Specific on-target and off-target editing efficiencies of Sdd6 and APOBEC base editors were tested at two on-target sites and four off-target sites in HEK293T cells (Figure 26C). The on-target and off-target efficiencies of Sdd6, APOBEC1-YE1, APOBEC1-YEE, APOBEC1, APOBEC1, and APOBEC3A at the on-target site of HsHEK2 corresponding to the off-target sites of HsJAK2-Sa and HsSIRT6-Sa, and the on-target and off-target editing efficiencies at the on-target site of HsHEK3 corresponding to the off-target sites of HsRNF2-Sa and HsFANCF-SaT1. The data are the results of three independent experiments.

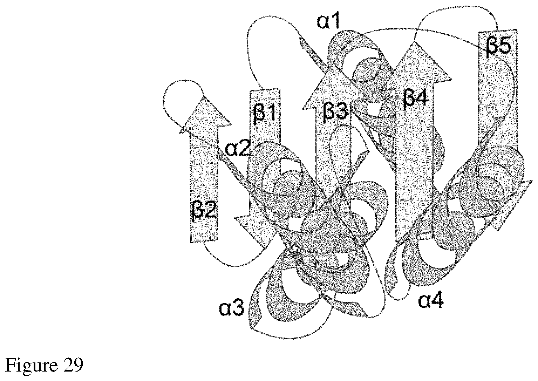

Figure 29: Conserved protein structure of highly active Sdd deaminases predicted by AlphaFold2. The core structure of Sdd deaminase with high deamination activity is shown. For some active deaminases, $\alpha 4$ is not a necessary structure.

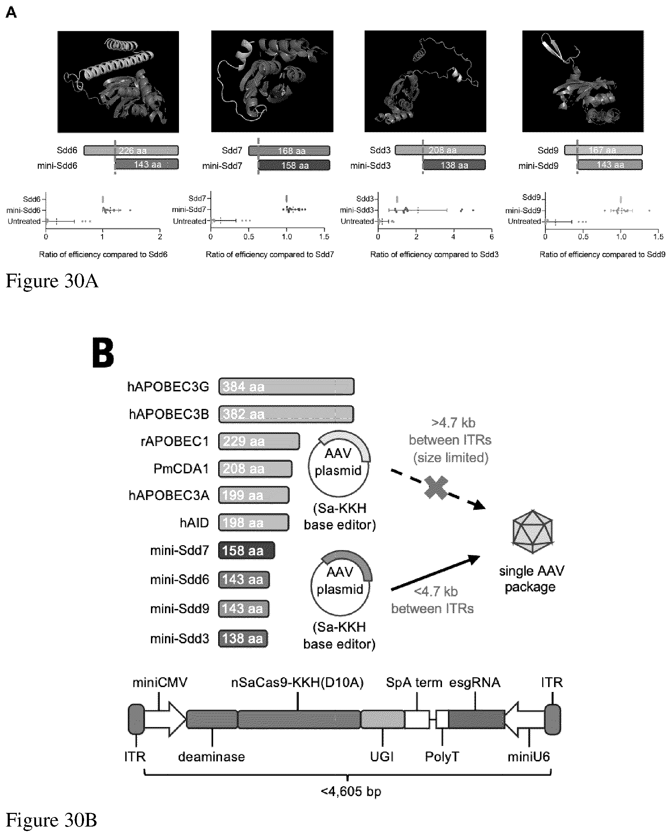

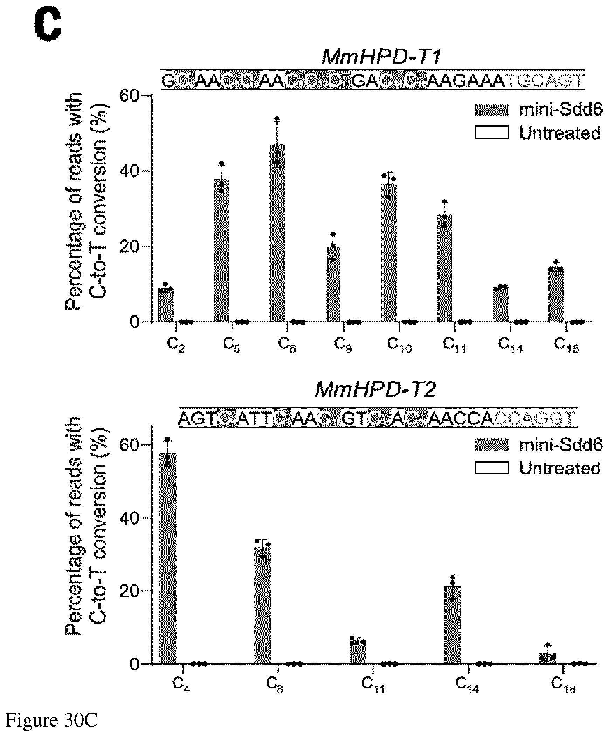

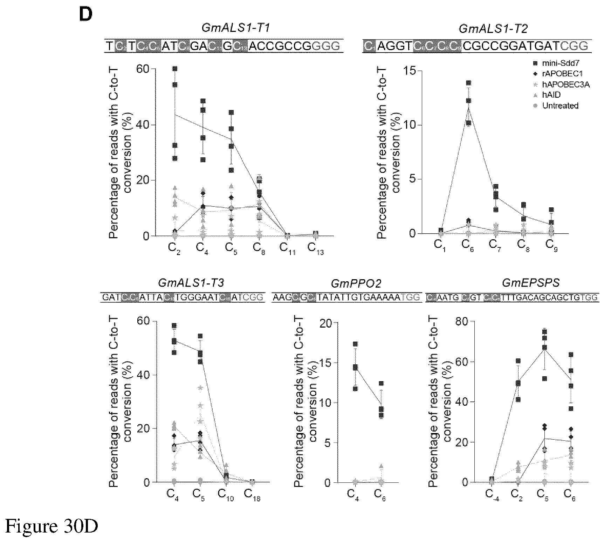

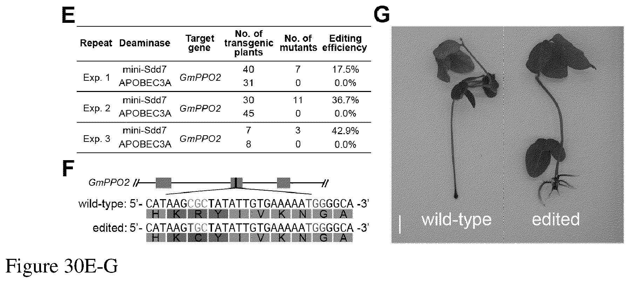

Figure 30: Engineering truncated Sdd proteins for use in animals and plants. (A) Engineering truncated Sdd proteins. The top panel shows the structures of Sdd6, Sdd7, Sdd3, and Sdd9 predicted by AlphaFold2. Conserved regions are represented by dark colors, and truncated regions are represented by light colors. The bottom panel shows the editing efficiency of Sdds and their minimized versions at two endogenous sites in rice protoplasts and HEK293T cells relative to the Sdd protein of original length. (B) Theoretical packaging of a SaCas9-based CBE vector for packaging into a single AAV. The top panle shows a schematic diagram of APOBEC/AID-like deaminases, Sdd minimized versions, and their AAV vectors. Among them, APOBEC3G, hAPOBEC3B, rAPOBEC1, PmCDA1, APOBEC3A, and hAID deaminase are too large for packaging using a single AAV. The bottom panel shows a schematic diagram of an AAV vector based on the Sdd minimized mini versions. (C) Editing efficiency of mini-Sdd6 at two endogenous targets of the MmHPD gene in mouse N2a cells. (D) Editing efficiency of mini-Sdd7, rAPOBEC1, hAPOBECA, and human AID base editors at five endogenous targets in soybean hairy roots. (E) Frequency of mutations induced by mini-Sdd7 in T0 generation soybean plants. (F) Genotypes of base-edited soybean plants. (G) Phenotypes of soybean plants treated with carfentrazone ethyl for 10 days. The left panel shows a wild-type soybean plant (R98). The right panel shows a base-edited soybean plant (C98). For panels A, C, and D, dots represent individual biological replicates, bara and line points represent the mean values, and error bars represent the standard deviation of three independent biological experiments.

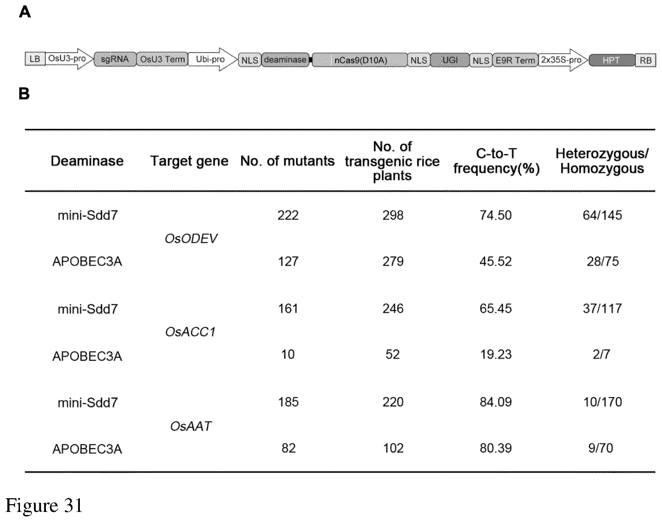

Figure 31: Frequencies of base-edited regenerated rice plants. (A) Schematic diagram of base editing binary vector for Agrobacterium-mediated transformation of rice. (B) Efficiency of mini-Sdd7 and hAPOBEC3A base editors in inducing mutations in T0 rice plants.

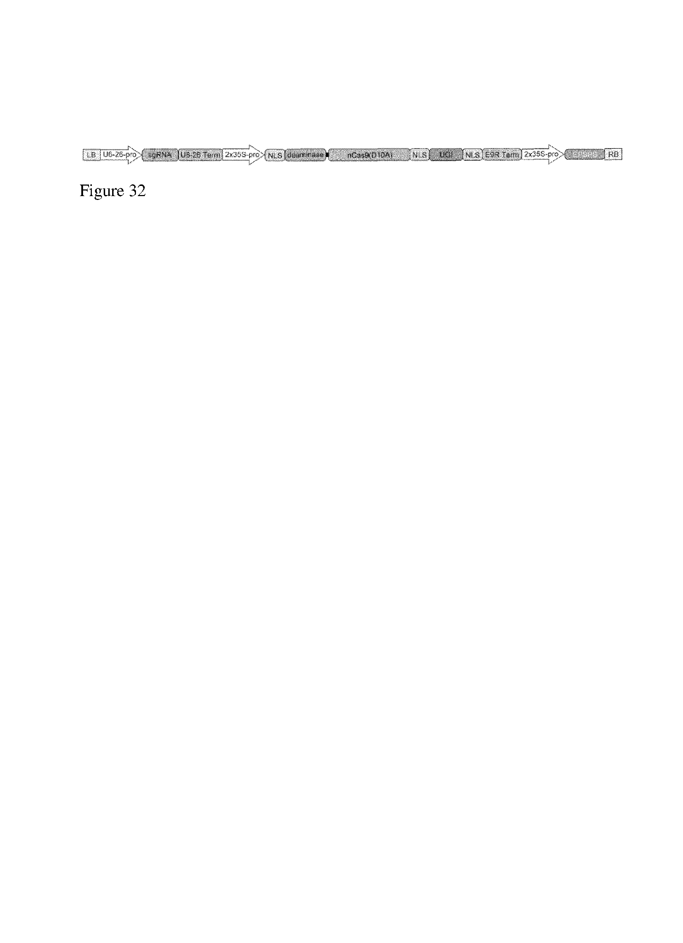

Figure 32: Schematic diagram of base editing binary vector for Agrobacterium-mediated transformation in soybean.

**Description of the invention**

**I. Definition**

In the present invention, unless indicated otherwise, the scientific and technological terminologies used herein refer to meanings commonly understood by a person skilled in the art. Also, the terminologies and experimental procedures used herein relating to protein and nucleotide chemistry, molecular biology, cell and tissue cultivation, microbiology, immunology, all belong to terminologies and conventional methods generally used in the art. In the meantime, in order to better understand the present invention, definitions and explanations for the relevant terminologies are provided below.

As used herein, the term "and/or" encompasses all combinations of items connected by the term, and each combination should be regarded as individually listed herein. For example, "A and/or B" covers "A", "A and B", and "B". For example, "A, B, and/or C" covers "A", "B", "C", "A and B", "A and C", "B and C", and "A and B and C".

"Cytosine deaminase" refers to a deaminase that can accept a nucleic acid, such as single-stranded DNA, as a substrate and can catalyze the deamination of cytidine or deoxycytidine to uracil or deoxyuracil, respectively.

"Genome" as used herein encompasses not only chromosomal DNA present in the nucleus, but also organelle DNA present in the subcellular components (e.g., mitochondria, plastids) of the cell.

As used herein, an "organism" includes any organism suitable for genome editing, preferably, a eukaryote. An example of an organism includes but is not limited to, a mammal such as human, mouse, rat, monkey, dog, pig, sheep, cattle, cat; poultry such as chicken, duck, goose; a plant, including a monocotyledonous plant or a dicotyledonous plant such as rice, corn, wheat, sorghum, barley, soybean, peanut, Arabidopsis and the like.

A "genetically modified organism" or a "genetically modified cell" means an organism or a cell which comprises an exogenous polynucleotide or comprises a modified gene or expression regulatory sequence within its genome. For example, the exogenous polynucleotide can be stably integrated into the genome of the organism or cell and inherited in successive generations. The exogenous polynucleotide may be integrated into the genome alone or as a part of a recombinant DNA construct. The modified gene or expression regulatory sequence is a gene or expression regulatory sequence comprising one or more nucleotide substitutions, deletions and additions in the genome of the organism or cell.

The term "exogenous" with respect to sequence means a sequence that originates from a foreign species, or, if from the same species, is substantially modified from its native form in composition and/or genomic locus by deliberate human intervention.

"Polynucleotide", "nucleic acid sequence", "nucleotide sequence", or "nucleic acid fragment" are used interchangeably to refer to a polymer of RNA or DNA that is single- or double-stranded, optionally containing synthetic, non-natural or altered nucleotide bases. Nucleotides are referred to by their single letter designation as follows: "A" for adenosine or deoxyadenosine (for RNA or DNA, respectively), "C" for cytidine or deoxycytidine, "G" for guanosine or deoxyguanosine, "U" for uridine, "T" for deoxythymidine, "R" for purines (A or G), "Y" for pyrimidines (C or T), "K" for G or T, "H" for A or C or T, "I" for inosine, and "N" for any nucleotide.

"Polypeptide", "peptide", "amino acid sequence" and "protein" are used interchangeably herein to refer to a polymer of amino acid residues. The terms apply to amino acid polymers in which one or more amino acid residue is an artificial chemical analogue of a corresponding naturally occurring amino acid, as well as to polymers of naturally occurring amino acids. The terms "polypeptide", "peptide", "amino acid sequence" and "protein" are also inclusive of modifications including, but not limited to, glycosylation, lipid attachment, sulfation, gamma-carboxylation of glutamic acid residues, hydroxylation and ADP-ribosylation.

Sequence "identity" has recognized meaning in the art, and the percentage of sequence identity between two nucleic acids or polypeptide molecules or regions can be calculated using the disclosed techniques. Sequence identity can be measured along the entire length of a polynucleotide or polypeptide or along a region of the molecule. (See, for example, Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). Although there are many methods for measuring the identity between two polynucleotides or polypeptides, the term "identity" is well known to the skilled person (Carrillo, H. & Lipman, D., SIAM J Applied Math 48: 1073 (1988)).

When the term "comprise" is used herein to describe the sequence of a protein or nucleic acid, the protein or nucleic acid may consist of the sequence or may have additional amino acids or nucleotides at one or both ends of the protein or nucleic acid, but still have the activity described in this invention. In addition, those skilled in the art know that the methionine encoded by the start codon at the N-terminus of the polypeptide will be retained under certain practical conditions (for example, when expressed in a specific expression system), but does not substantially affect the function of the polypeptide. Therefore, when describing the amino acid sequence of a specific polypeptide in the specification and claims of the present application, although it may not include the methionine encoded by the start codon at the N-terminus, the sequence containing the methionine is also encompassed, correspondingly, its coding nucleotide sequence may also contain a start codon; vice versa.

Suitable conserved amino acid substitutions in peptides or proteins are known to those skilled in the art and can generally be carried out without altering the biological activity of the resulting molecule. In general, one skilled in the art recognizes that a single amino acid substitution in a non-essential region of a polypeptide does not substantially alter the biological activity (See, for example, Watson et al., Molecular Biology of the Gene, 4th Edition, 1987, The Benjamin/Cummings Pub. co., p.224).

As used herein, an "expression construct" refers to a vector suitable for expression of a nucleotide sequence of interest in an organism, such as a recombinant vector. "Expression" refers to the production of a functional product. For example, the expression of a nucleotide sequence may refer to transcription of a nucleotide sequence (such as transcribing to produce an mRNA or a functional RNA) and/or translation of RNA into a protein precursor or a mature protein.

"Expression construct" of the invention may be a linear nucleic acid fragment, a circular plasmid, a viral vector, or, in some embodiments, an RNA that can be translated (such as an mRNA) .

"Expression construct" of the invention may comprise regulatory sequences and nucleotide sequences of interest that are derived from different sources, or regulatory sequences and nucleotide sequences of interest derived from the same source but arranged in a manner different from that normally found in nature.

"Regulatory sequence" or "regulatory element" are used interchangeably and refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to, promoters, translation leader sequences, introns, and polyadenylation recognition sequences.

"Promoter" refers to a nucleic acid fragment capable of controlling the transcription of another nucleic acid fragment. In some embodiments of the present invention, the promoter is a promoter capable of controlling the transcription of a gene in a cell, whether or not it is derived from the cell. The promoter may be a constitutive promoter or a tissue-specific promoter or a developmentally regulated promoter or an inducible promoter.

"Constitutive promoter" refers to a promoter that may cause expression of a gene in most circumstances in most cell types. "Tissue-specific promoter" and "tissue-preferred promoter" are used interchangeably and refer to a promoter that is expressed predominantly but not necessarily exclusively in one tissue or organ, but that may also be expressed in one specific cell or cell type. "Developmentally regulated promoter" refers to a promoter whose activity is determined by developmental events. "Inducible promoter" selectively expresses a DNA sequence operably linked to it in response to an endogenous or exogenous stimulus (environment, hormones, or chemical signals, and so on).

Examples of promoters include, but are not limited to, polymerase (pol) I, pol II or pol III promoters. Examples of pol I promoters include chicken RNA pol I promoter. Examples of pol II promoters include, but are not limited to, cytomegalovirus immediate early (CMV) promoter, Rous sarcoma virus long terminal repeat (RSV-LTR) promoter, and simian virus 40 (SV40) immediate early promoter. Examples of pol III promoters include U6 and H1 promoters. Inducible promoters such as metallothionein promoters can be used. Other examples of promoters include T7 phage promoter, T3 phage promoter, β-galactosidase promoter, and Sp6 phage promoter. When used for plants, the promoter can be a cauliflower mosaic virus 35S promoter, a corn Ubi-1 promoter, a wheat U6 promoter, a rice U3 promoter, a corn U3 promoter, a rice actin promoter.

As used herein, the term "operably linked" means that a regulatory element (for example but not limited to, a promoter sequence, a transcription termination sequence, and so on) is associated to a nucleic acid sequence (such as a coding sequence or an open reading frame), such that the transcription of the nucleotide sequence is controlled and regulated by the transcriptional regulatory element. Techniques for operably linking a regulatory element region to a nucleic acid molecule are known in the art.

"Introduction" of a nucleic acid molecule (e.g., plasmid, linear nucleic acid fragment, RNA, etc.) or protein into an organism means that the nucleic acid or protein is used to transform a cell of the organism such that the nucleic acid or protein functions in the cell. As used in the present invention, "transformation" includes both stable transformation and transient transformation.

"Stable transformation" refers to the introduction of an exogenous nucleotide sequence into the genome, resulting in the stable inheritance of the exogenous nucleotide sequence. Once stably transformed, the exogenous nucleotide sequence is stably integrated into the genome of the organism and any of its successive generations.

"Transient transformation" refers to the introduction of a nucleic acid molecule or protein into a cell, executing its function without the stable inheritance of an exogenous nucleotide sequence. In transient transformation, the exogenous nucleotide sequence is not integrated into the genome.

## II. Protein clustering and function prediction methods based on three-dimensional structure

In one aspect, the present invention provides a protein clustering method, which comprises:

(1) obtaining the sequences of a plurality of candidate proteins from a database;
(2) predicting the three-dimensional structure of each of the plurality of candidate proteins using a protein prediction program;
(3) performing multiple structural alignment on the three-dimensional structures of the plurality of candidate proteins using a scoring function, thereby obtaining a structural similarity matrix;
(4) clustering the plurality of candidate proteins based on the structural similarity matrix using a phylogenetic tree construction method.

In some embodiments, in step (1), the sequences of the plurality of candidate proteins are obtained through the annotation information in the database. For example, if deaminases are to be clustered, the sequences of a plurality of candidate proteins annotated as "deaminase" can be selected from the database.

In some embodiments, in step (1), the sequences of the plurality of candidate proteins are obtained by searching in a database based on sequence identity/similarity using the sequence of a reference protein. For example, the sequences of the plurality of candidate proteins can be obtained by searching in a database based on the sequence of a reference protein with known function using a BLAST program. In some embodiments, the plurality of candidate proteins have at least 20%, at least 30%, at least 40%, at least 50%, at least 60%, at least 70%, at least 80%, at least 90%, at least 95% sequence identity with the sequence of the reference protein.

In some embodiments, the candidate protein is a deaminase. In some preferred embodiments, the candidate protein is a cytosine deaminase.

In some embodiments, the database is the InterPro database. In some embodiments, the protein structure prediction program in step (2) is selected from AlphaFold2, RoseTT or other programs that can predict protein structures. (John Jumper and others, 'Highly Accurate Protein Structure Prediction with AlphaFold', Nature, 596.7873 (2021), 583-89)

In some embodiments, the scoring function used in step (3) includes TM-score, RMSD, LDDT, GDT score, QSC, FAPE or other scoring functions that can score for protein structure similarity. (John Jumper and others, 'Highly Accurate Protein Structure Prediction with AlphaFold', Nature, 596.7873 (2021), 583-89)

In some embodiments, when the scoring function is TM-score, the TM-score is at least 0.6, at least 0.7, at least 0.75, at least 0.8, at least 0.85 or higher. The calculation of TM-score can refer to the formula and method described in the "Materials and Methods" section of the examples of the present application.

In some embodiments, the phylogenetic tree construction method in step (4) is the Unweighted Pair Group Method with Arithmetic Mean (UPGMA) (C. P. Kurtzman, Jack W. Fell, and T. Boekhout, The Yeasts: A Taxonomic Study, 5th ed (Amsterdam: Elsevier, 2011); 'A Statistical Method for Evaluating Systematic Relationships - Robert Reuven Sokal, Charles Duncan Michener - Google Books').

In some embodiments, a clustering dendrogram of the plurality of candidate proteins is obtained in step (4).

In one aspect, the present invention provides a method for predicting the function of a protein based on three-dimensional structure, the method comprising clustering a plurality of candidate proteins according to the protein clustering method of the present invention, and then predicting the function of the candidate proteins according to the clustering result.

In some embodiments, the plurality of candidate proteins includes at least one reference protein with known function.

In some embodiments, the functions of other candidate proteins in the same clade or subclade are predicted by the position of the reference protein with known function in the cluster (dendrogram). In some embodiments, other candidate proteins located in the same clade or subclade as the reference protein are predicted to have the same or similar function as the reference protein. In some embodiments, the TM-score between different candidate proteins in the same clade or subclade is at least 0.6, at least 0.7, at least 0.75, at least 0.8, at least 0.85 or higher. In some embodiments, the TM-score between candidate proteins in different clades or subclade is less than 0.85, less than 0.8, less than 0.75, less than 0.7, less than 0.6 or less.

In some embodiments, the reference protein is a deaminase. In some preferred embodiments, the reference protein is a cytosine deaminase. In some embodiments, the reference protein is a reference cytosine deaminase, the reference cytosine deaminase is rAPOBEC1 having a sequence shown in SEQ ID No: 64 or DddA having a sequence shown in SEQ No: 65. In some embodiments, the TM-score between a different candidate protein in the same clade or subclade as the reference protein or the TM-score between a different candidate protein and the reference protein is at least 0.7. In some embodiments, the TM-score of a candidate protein in a different clade or subclade from the reference protein is less than 0.7.

In another aspect, the present invention provides a method for identifying a minimal functional domain of a protein based on the three-dimensional structure, comprising:

 a) comparing the structures of a plurality of candidate proteins clustered together by the method of the present invention, for example, clustered in the same clade or subclade, to determine the conserved core structure;
 b) identifying the conserved core structure as the minimal functional domain.

As used herein, "minimal functional domain" refers to the smallest portion of a protein that can substantially maintain the function of the full-length protein.

In some embodiments, the plurality of candidate proteins includes at least one reference protein with known function.

In some embodiments, the reference protein is a deaminase. In some preferred embodiments, the reference protein is a cytosine deaminase. In some embodiments, the reference protein is a reference cytosine deaminase, and the reference cytosine deaminase is rAPOBEC1 having a sequence shown in SEQ ID No: 64 or DddA having a sequence shown in SEQ No: 65.

In another aspect, the present invention provides a cytosine deaminase identified by the method of the present invention for predicting the function of a protein.

In another aspect, the present invention provides a truncated cytosine deaminase comprising or consisting of the minimal functional domain of cytosine deaminase identified by the method of the present invention.

In one aspect, the present invention also provides use of the cytosine deaminase or truncated cytosine deaminase for gene editing, such as base editing, in an organism or a cell of an organism.

### III. Cytosine deaminase and base editing fusion protein containing the same

In one aspect, the present invention provides a cytosine deaminase, wherein the cytosine deaminase is capable of deaminating the cytosine base of deoxycytidine in DNA. In some embodiments, the cytosine deaminase is from bacteria.

In some embodiments, the TM-score of the AlphaFold2 three-dimensional structure between the cytosine deaminase and the reference cytosine deaminase is not less than 0.6, not less than 0.7, not less than 0.75, not less than 0.8, not less than 0.85, and the cytosine deaminase comprises an amino acid sequence having 20-70%, 20-60%, 20-50%, 20-45%, 20-40%, 20-35% sequence identity or at least 20%, at least 30%, at least 40%, at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99% sequence identity with the amino acid sequence of the reference cytosine deaminase; the cytosine deaminase has the function of deaminating the cytosine base of the deoxycytidine of DNA.

In some embodiments, the reference cytosine deaminase is:

 (a) rAPOBEC1 having a sequence shown in SEQ ID No: 64; or
 (b) DddA having a sequence shown in SEQ ID No: 65; or
 (c) Sdd7 having a sequence shown in SEQ ID No: 4.

In some embodiments, the TM-score of the AlphaFold2 three-dimensional structure between the cytosine deaminase and rAPOBEC1 having a sequence shown in SEQ ID No: 64 is not less than 0.6, not less than 0.7, not less than 0.75, not less than 0.8, not less than 0.85, and the cytosine deaminase comprises an amino acid sequence having 20-70%, 20-60%, 20-50%, 20-45%, 20-40%, 20-35% sequence identity or at least 20%, at least 30%, at least 40%, at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99% sequence identity with SEQ ID No: 64; the cytosine deaminase has the function of deaminating the cytosine base of deoxycytidine in DNA.

In some embodiments, the TM-score score of the AlphaFold2 three-dimensional structure between the cytosine deaminase and DddA having a sequence shown in SEQ ID No: 65 is not less than 0.6, not less than 0.7, not less than 0.75, not less than 0.8, not less than 0.85, and the cytosine deaminase comprises an amino acid sequence having 20-70%, 20-60%, 20-50%, 20-45%, 20-40%, 20-35% sequence identity or at least 20%, at least 30%, at least 40%, at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99% sequence identity with SEQ ID No: 65; the cytosine deaminase has the function of deaminating the cytosine base of deoxycytidine in DNA.

In some embodiments, the TM-score score of the AlphaFold2 three-dimensional structure between the cytosine deaminase and Sdd7 having a sequence shown in SEQ ID No: 4 is not less than 0.6, not less than 0.7, not less than 0.75, not less than 0.8, not less than 0.85, and the cytosine deaminase comprises an amino acid sequence having 20-70%, 20-60%, 20-50%, 20-45%, 20-40%, 20-35% sequence identity or at least 20%, at least 30%, at least 40%, at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99% sequence identity with SEQ ID No: 4; the cytosine deaminase has the function of deaminating the cytosine base of deoxycytidine in DNA.

In some embodiments, the cytosine deaminase is from the AID/APOBEC clade, SCP1.201 clade, MafB19 clade, Novel AID/APOBEC-like clade, TM1506 clade, or XOO2897 clade.

Herein, the cytosine deaminase clade is determined according to the contents described in Iyer, L. M., Zhang, D., Rogozin, I. B., & Aravind, L. (2011). Evolution of the deaminase fold and multiple origins of eukaryotic editing and mutagenic nucleic acid deaminases from bacterial toxin systems. Nucleic acids research, 39(22), 9473-9497.

In some embodiments, the cytosine deaminase is from the AID/APOBEC clade and comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity to SEQ ID NO: 1.

In some embodiments, the cytosine deaminase is from the SCP1.201 clade, and comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity with any one of SEQ ID No: 28-40. In some embodiments, the cytosine deaminase is capable of deaminating the cytosine base of double-stranded DNA. In some embodiments, the amino acid sequence of the cytosine deaminase consists of the amino acid sequence of any one of SEQ ID No: 28-40. In some embodiments, the amino acid sequence of the cytosine deaminase consists of the amino acid sequence of any one of SEQ ID No: 28, 33, 34, 35.

In some embodiments, the cytosine deaminase is from the SCP1.201 clade and comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity with any one of SEQ ID No: 2-18, 41-49. In some embodiments, the cytosine deaminase is capable of deaminating the cytosine base of single-stranded DNA. In some embodiments, the amino acid sequence of the cytosine deaminase consists of the amino acid sequence of any one of SEQ ID No: 2-18, 41-49. In some embodiments, the amino acid sequence of the cytosine deaminase consists of the amino acid sequence of any one of SEQ ID No: 2-7, 12, 17.

In some embodiments, the cytosine deaminase is a truncated cytosine deaminase, which is capable of deaminating the cytosine base of deoxycytidine of DNA. In some embodiments, the length of the truncated cytosine deaminase ranges from 130 to 160 amino acids. In some embodiments, the truncated cytosine deaminase can be packaged entirely in one AAV particle.

In some embodiments, the truncated cytosine deaminase comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity to any one of SEQ ID Nos: 50-55. In some embodiments, the truncated cytosine deaminase is capable of deaminating cytosine bases of single-stranded DNA. In some embodiments, the truncated cytosine deaminase consists of the amino acid sequence of any one of SEQ ID Nos: 50-55.

In some embodiments, the cytosine deaminase is from the MafB19 clade and comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity to any one of SEQ ID Nos: 19, 56, 57, and 58.

In some embodiments, the cytosine deaminase is from the Novel AID/APOBEC-like clade and comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity to any one of SEQ ID No: 20, 21.

In some embodiments, the cytosine deaminase is from the TM1506 clade and comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity to SEQ ID No: 22.

In some embodiments, the cytosine deaminase is from the XOO2897 clade and comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity to SEQ ID Nos: 23, 24, 59-62.

In some embodiments, the cytosine deaminase is from the Toxin deam clade and comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity to SEQ ID No: 74 or 75.

In one aspect, the present application relates to use of the cytosine deaminase of the present invention for gene editing, such as base editing, in an organism or a cell of an organism.

In some embodiments, the cytosine deaminase is used for preparing a base editing fusion protein or a base editing system, which is used to perform base editing in an organism or a cell of an organism.

In another aspect, the present invention provides a base editing fusion protein comprising a nucleic acid targeting domain and a cytosine deamination domain, wherein the cytosine deamination domain comprises at least one (e.g., one or two) cytosine deaminase polypeptide of the present invention.

In the embodiments herein, "fusion protein", "base editing fusion protein" and "base editor" are used interchangeably to refer to a protein that can mediate one or more nucleotide substitutions of a target sequence in a genome in a sequence-specific manner. The one or more nucleotide substitutions are, for example, C to T substitutions.

As used herein, a "nucleic acid targeting domain" refers to a domain that can mediate the attachment of the base editing fusion protein to a specific target sequence in the genome in a sequence-specific manner (e.g., through a guide RNA). In some embodiments, the nucleic acid targeting domain may include one or more zinc finger protein domains (ZFP) or transcription factor effector domains (TALE) against a specific target sequence. In some embodiments, the nucleic acid targeting domain comprises at least one (e.g., one) CRISPR effector protein (CRISPR effector) polypeptide.

The "Zinc finger protein domain (ZFP)" usually contains 3-6 individual zinc finger repeat sequences, each of which can recognize a unique sequence of, for example, 3 bp. By combining different zinc finger repeat sequences, different genomic sequences can be targeted.

The "transcription activator-like effector domain" is the DNA binding domain of a transcription activator-like effector (TALE). TALEs can be engineered to bind to almost any desired DNA sequence.

As used herein, the term "CRISPR effector protein" generally refers to a nuclease (CRISPR nuclease) or a functional variant thereof present in a naturally occurring CRISPR system. The term encompasses any effector protein based on the CRISPR system that is capable of achieving sequence-specific targeting within a cell.

As used herein, a "functional variant" with respect to a CRISPR nuclease means that it at least retains the sequence-specific targeting ability mediated by a guide RNA. Preferably, the functional variant is a nuclease-inactivated variant, i.e., it lacks double-stranded nucleic acid cleavage activity. However, CRISPR nucleases lacking double-stranded nucleic acid cleavage activity also encompass nickases, which form a nick in a double-stranded nucleic acid molecule but do not completely cut off the double-stranded nucleic acid. In some preferred embodiments of the present invention, the CRISPR effector protein of the present invention has nickase activity. In some embodiments, the functional variant recognizes a different PAM (protospacer adjacent motif) sequence relative to the wild-type nuclease.

"CRISPR effector protein" can be derived from Cas9 nuclease, including Cas9 nuclease or its functional variant. The Cas9 nuclease can be a Cas9 nuclease from different species, such as spCas9 from *Streptococcus pyogenes* (*S. pyogenes)* or SaCas9 derived from *Staphylococcus aureus (S. aureus).* "Cas9 nuclease" and "Cas9" are used interchangeably herein and refer to RNA-guided nucleases including Cas9 proteins or fragments thereof (e.g., proteins comprising active DNA cleavage domains of Cas9 and/or gRNA binding domains of Cas9). Cas9 is a component of the CRISPR/Cas (clustered regularly interspaced short palindromic repeats and CRISPR associated) genome editing system, which can target and cut DNA target sequences under the guidance of guide RNA to form DNA double-strand breaks (DSBs). An exemplary amino acid sequence of wild-type SpCas9 is shown in SEQ ID NO:25.

The "CRISPR effector protein" can also be derived from a Cpf1 nuclease, including a Cpf1 nuclease or a functional variant thereof. The Cpf1 nuclease can be a Cpf1 nuclease from different species, such as a Cpf1 nuclease from *Francisella novicida* U112, *Acidaminococcus* sp. BV3L6, and *Lachnospiraceae bacterium* ND2006.

Useful "CRISPR effector proteins" can also be derived from nucleases such as Cas3, Cas8a, Cas5, Cas8b, Cas8c, Cas10d, Cse1, Cse2, Csy1, Csy2, Csy3, GSU0054, Cas10, Csm2, Cmr5, Cas10, Csx11, Csx10, Csf1, Csn2, Cas4, C2c1 (Cas12b), C2c3, C2c2, Cas12c, Cas12d (i.e., CasY), Cas12e (i.e., CasX), Cas12f (i.e., Cas14), Cas12g, Cas12h, Cas12i, Cas12j (i.e., CasΦ), Cas12k, Cas12l, Cas12m, etc., for example, including these nucleases or functional variants thereof.

In some embodiments, the CRISPR effector protein is a nuclease-inactivated Cas9. The DNA cleavage domain of the Cas9 nuclease is known to contain two subdomains: the HNH nuclease subdomain and the RuvC subdomain. The HNH subdomain cuts the strand complementary to the gRNA, while the RuvC subdomain cuts the non-complementary strand. Mutations in these subdomains can inactivate the nuclease activity of Cas9, forming a "nuclease-inactivated Cas9". The nuclease-inactivated Cas9 still retains the DNA binding ability guided by the gRNA.

The nuclease-inactivated Cas9 of the present invention can be derived from Cas9 of different species, for example, derived from *Streptococcus pyogenes (S. pyogenes)* Cas9 (SpCas9), or derived from *Staphylococcus aureus*

*(S. aureus)* Cas9 (SaCas9). Mutating the HNH nuclease subdomain and the RuvC subdomain of Cas9 (for example, comprising mutations D10A and H840A) simultaneously inactivates the nuclease of Cas9 and becomes nuclease-dead Cas9 (dCas9). Mutation-inactivation of one of the subdomains can enable Cas9 to have nickase activity, i.e., Cas9 nickase (nCas9), for example, nCas9 with only the mutation D10A.

Therefore, in some embodiments of various aspects of the present invention, the nuclease-inactivated Cas9 variant of the present invention comprises an amino acid substitution D10A and/or H840A relative to wild-type Cas9, wherein the amino acid numbering refers to SEQ ID NO: 25. In some preferred embodiments, the nuclease-inactivated Cas9 comprises an amino acid substitution D10A relative to wild-type Cas9, wherein the amino acid numbering refers to SEQ ID NO: 25. In some embodiments, the nuclease-inactivated Cas9 comprises the amino acid sequence shown in SEQ ID NO: 26 (nCas9 (D10A)).

When Cas9 nuclease is used for gene editing, it is usually required that the target sequence has a PAM (protospacer adjacent motif) sequence of 5'-NGG-3' at its 3' end. However, the inventors surprisingly found that this PAM sequence has a very low frequency of occurrence in certain species such as rice, which greatly limits gene editing in these species such as rice. For this reason, CRISPR effector proteins that recognize different PAM sequences are preferably used in the present invention, such as functional variants of Cas9 nucleases with different PAM sequences.

In some embodiments of the present invention, the cytidine deamination domain in the fusion protein is capable of converting the cytidine of the single-stranded DNA generated during the formation of the fusion protein-guide RNA-DNA complex into U by deamination, and then achieving base substitution from C to T through base mismatch repair.

In some embodiments of the present invention, the nucleic acid targeting domain and the cytosine deamination domain are fused via a linker.

As used herein, a "linker" can be a non-functional amino acid sequence having a length of 1-50 (e.g., 1, 2, 3, 4, 5, 6, 7, 8, 9, 10, 11, 12, 13, 14, 15, 16, 17, 18, 19, 20 or 20-25, 25-50) or more amino acids and having no secondary or higher structure. For example, the linker can be a flexible linker.

In some embodiments, the base editing fusion protein comprises, in the following order from N-terminus to C-terminus: a cytosine deamination domain and a nucleic acid targeting domain.

In addition, in cells, uracil DNA glycosylase catalyzes the removal of U from DNA and initiates base excision repair (BER), resulting in the repair of U:G to C:G. Therefore, without being limited by any theory, combining a uracil DNA glycosylase inhibitor (UGI) with the base editing fusion protein of the present invention will be able to increase the efficiency of C to T base editing.

In some embodiments, the base editor fusion protein is co-expressed with a uracil DNA glycosylase inhibitor (UGI).

In some embodiments, the base editor fusion protein further comprises a uracil DNA glycosylase inhibitor (UGI).

In some embodiments, UGI is connected to other parts of the base editing fusion protein through a linker.

In some embodiments, UGI is connected to other parts of the base editing fusion protein through a "self-cleaving peptide".

As used herein, "self-cleaving peptide" means a peptide that can achieve self-cleavage in a cell. For example, the self-cleaving peptide may include a protease recognition site, thereby being recognized and specifically cleaved by a protease in the cell. Alternatively, the self-cleaving peptide may be a 2A polypeptide. 2A polypeptides are a class of short peptides from viruses, and their self-cleavage occurs during translation. When two different target polypeptides are expressed in the same reading frame by connecting them with a 2A polypeptide, two target polypeptides are generated at a ratio of almost 1:1. Commonly used 2A polypeptides may be P2A from porcine techovirus-1, T2A from Thosea asigna virus, E2A from equine rhinitis A virus, and F2A from foot-and-mouth disease virus. A variety of functional variants of these 2A polypeptides are also known in the art, and these variants may also be used in the present invention.

Preferably, the self-cleaving peptide does not exist between or within the nucleic acid targeting domain and the cytosine deamination domain. In some embodiments, the UGI is located at the N-terminus or C-terminus of the base editing fusion protein, preferably at the C-terminus.

In some specific embodiments, the uracil DNA glycosylase inhibitor (UGI) comprises the amino acid sequence shown in SEQ ID NO:27.

In some embodiments of the present invention, the fusion protein of the present invention may further include a nuclear localization sequence (NLS). In general, one or more NLSs in the fusion protein should have sufficient strength to drive the fusion protein in the nucleus of the cell to accumulate in an amount that can realize its base editing function. In general, the intensity of nuclear localization activity is determined by the number, position, one or more specific NLSs used in the fusion protein, or a combination of these factors.

In some embodiments of the present invention, the NLSs of the fusion protein of the present invention may be located at the N-terminus and/or the C-terminus. In some embodiments of the present invention, the NLSs of the fusion protein of the present invention may be located between the cytosine deamination domain, the nucleic acid targeting domain and/or the UGI. In some embodiments, the fusion protein comprises about 1, 2, 3, 4, 5, 6, 7, 8, 9, 10 or more NLSs. In some embodiments, the fusion protein comprises about 1, 2, 3, 4, 5, 6, 7, 8, 9, 10 or more NLS at or close to the N-terminus. In some embodiments, the fusion protein comprises about 1, 2, 3, 4, 5, 6, 7, 8, 9, 10 or more NLS at or close to the C-terminus. In some embodiments, the polypeptide comprises a combination of these, such as one or more NLSs at the N-terminus and one or more NLSs at the C-terminus. When there are more than one NLS, each can be selected to be independent of other NLSs.

Generally, NLS consists of one or more short sequences of positively charged lysine or arginine exposed on the surface of the protein, but other types of NLSs are also known. Nonlimiting examples of NLSs include: KKRKV, PKKKRKV or KRPAATKKAGQAKKKK.

In addition, depending on the DNA location to be edited, the fusion protein of the present invention may also include other localization sequences, such as cytoplasmic localization sequences, chloroplast localization sequences, mitochondrial localization sequences, etc.

## IV. Base Editing System

In another aspect, the present invention provides a base editing system comprising: i) a cytosine deaminase or a base editing fusion protein of the present invention, and/or an expression construct containing a nucleotide sequence encoding the cytosine deaminase or the base editing fusion protein.

In some embodiments, the base editing system is used to modify a target nucleic acid region.

In some embodiments, the base editing system further comprises ii) at least one guide RNA and/or at least one expression construct comprising a nucleotide sequence encoding the at least one guide RNA. However, one skilled in the art will appreciate that if the base editing fusion protein is not based on a CRISPR effector protein, the system may not require a guide RNA or an expression construct encoding the same.

In some embodiments, the at least one guide RNA can bind to the nucleic acid targeting domain of the fusion protein. In some embodiments, the guide RNA is directed to at least one target sequence within the target nucleic acid region.

As used herein, a "base editing system" refers to a combination of components required for base editing a nucleic acid sequence such as a genomic sequence in a cell or an organism. The individual components of the system, such as cytosine deaminase, base editing fusion protein, and one or more guide RNAs, may exist independently of each other, or may exist in any combination as a composition.

In some embodiments, the system comprises a cytosine deaminase of the present invention or a fusion protein of the present invention and a guide RNA that can targeted bind to a nucleic acid.

As used herein, "guide RNA" and "gRNA" are used interchangeably and refer to an RNA molecule that is capable of forming a complex with a CRISPR effector protein and is capable of targeting the complex to a target sequence due to certain identity with the target sequence. For example, the gRNA used by Cas9 nuclease or its variants is usually composed of crRNA and tracrRNA molecules that are partially complementary to form a complex, where the crRNA contains sufficient identity to the target sequence to hybridize to the complementary strand of the target sequence and guide the CRISPR complex (Cas9+crRNA+tracrRNA) to specifically bind to the target sequence. However, it is known in the art that single guide RNAs (sgRNAs) can be designed that contain characteristics of both crRNA and tracrRNA. The gRNA used by Cpf1 nuclease or its variants is usually composed only of a mature crRNA molecule, which can also be called sgRNA. It is within the ability of those skilled in the art to design a suitable gRNA based on the CRISPR nuclease or variant thereof as used and the target sequence to be edited.

In some embodiments, the guide RNA is 15-100 nucleotides in length and comprises a sequence having at least 10, at least 15, or at least 20 consecutive nucleotides complementary to the target sequence.

In some embodiments, the guide RNA comprises a sequence of 15 to 40 consecutive nucleotides complementary to the target sequence.

In some embodiments, the guide RNA is 15-50 nucleotides in length.

In some embodiments, the target sequence is a DNA sequence.

In some embodiments, the target sequence is within the genome of an organism. In some embodiments, the organism is a prokaryote. In some embodiments, the prokaryote is a bacterium. In some embodiments, the organism is a eukaryote. In some embodiments, the organism is a plant or a fungus. In some embodiments, the organism is a vertebrate. In some embodiments, the vertebrate is a mammal. In some embodiments, the mammal is a mouse, a rat, or a human. In some embodiments, the organism is a cell. In some embodiments, wherein the cell is a mouse cell, a rat cell, or a human cell. In some embodiments, wherein the cell is a HEK-293 cell.

In some embodiments, after the base editing system of the present invention is introduced into the cell, the base editing fusion protein and the guide RNA are capable of forming a complex, and the complex specifically targets the target sequence under the mediation of the guide RNA, and results in one or more C in the target sequence being substituted by T and/or one or more A being substituted by G.

In some embodiments, the at least one guide RNA may be directed to a target sequence on a sense strand (e.g., a protein coding strand) and/or an antisense strand located within a genomic target nucleic acid region. When the guide RNA targets the sense strand (e.g., a protein coding strand), the base editing composition of the present invention may result in one or more C in the target sequence on the sense strand (e.g., a protein coding strand) being substituted by T and/or one or more A being substituted by G. When the guide RNA targets the antisense strand, the base editing composition of the present invention may result in one or more Gs in the target sequence on the sense strand (e.g., a protein coding strand) being substituted by A and/or one or more T being substituted by C.

In order to obtain efficient expression in the cell, in some embodiments of the present invention, the nucleotide sequence encoding the cytosine deaminase or base editing fusion protein is codon-optimized against the organism whose genome is to be modified.

The codon optimization refers to a method for replacing at least one codon in the natural sequence (for example, about or more than about 1, 2, 3, 4, 5, 10, 15, 20, 25, 50 or more codons) with a codon used more frequently or most frequently in the gene of the host cell, and maintaining the natural amino acid sequence while modifying the nucleic acid sequence to enhance expression in the host cell of interest. Different species exhibit specific preferences for certain codons of specific amino acids. Codon preference (difference in codon usage between organisms) is often related to the translation efficiency of messenger RNA (mRNA), which is considered as depending on the nature of the codon being translated and the availability of the specific transfer RNA (tRNA) molecule. The advantages of the selected tRNA in the cell generally reflect the codons most frequently used for peptide synthesis. Therefore, genes may be tailored to the optimal gene expression in a given organism based on codons optimization. The codon usage tables may be easily obtained, for example, in the codon usage database ("Codon Usage Database") available at www.kazusa.orjp/codon/, and these tables may be adjusted and applied in different ways. See Nakamura Y. et al., "Codon usage tabulated from the international DNA sequence databases: status for the year 2000". Nucl. Acids Res., 28: 292 (2000).

Organisms that can be subjected to genome modification by the base editing system of the present invention include any organisms suitable for base editing, preferably eukaryotic organisms. Examples of organisms include, but are not limited to, mammals such as human, mouse, rat, monkey, dog, pig, sheep, cattle, cat; poultry such as chicken, duck, goose; plants, including monocots and dicots, for example, the plants are crop plants, including but not limited to wheat, rice, corn, soybean, sunflowers, sorghum, rapeseed, alfalfa, cotton, barley, millet, sugarcane, tomato, tobacco, cassava and potato.

## V. Base Editing Method

In another aspect, the present invention provides a base editing method, which comprises contacting the base editing system of the present invention with a target sequence of a nucleic acid molecule.

In some embodiments, the nucleic acid molecule is a DNA molecule. In some preferred embodiments, the nucleic acid molecule is a double-stranded DNA molecule or a single-stranded DNA molecule.

In some embodiments, the target sequence of the nucleic acid molecule comprises a sequence associated with a plant trait or expression.

In some embodiments, the target sequence of the nucleic acid molecule comprises a sequence or a point mutation associated with a disease or condition.

In some embodiments, the base editing system exert the deamination by contacting with the target sequence of the nucleic acid molecule to, and the deamination results in one or more nucleotides of the target sequence being substituted.

In some embodiments, the target sequence comprises a DNA sequence 5'-MCN-3', wherein M is A, T, C or G; N is A, T, C or G; wherein the C in the middle of the 5'-MCN-3' sequence is deaminated.

In some embodiments, the deamination results in the introduction or removal of a splicing site.

In some embodiments, the deamination results in the introduction of a mutation in a gene promoter, the mutation resulting in an increase or decrease in transcription of a gene operably linked to the gene promoter.

In some embodiments, the deamination results in the introduction of a mutation in a gene repressor, the mutation resulting in an increase or decrease in transcription of a gene operably linked to the gene repressor.

In some embodiments, the contacting is performed in vivo.

In some embodiments, the contacting is performed in vitro.

## VI. Method for Producing a Genetically Modified Cell

In another aspect, the present invention also provides a method for producing at least one genetically modified cell, comprising introducing the base editing system of the present invention into at least one said cell, thereby causing one or more nucleotide substitutions in the target nucleic acid region of the at least one cell. In some embodiments, the one or more nucleotide substitutions are C to T substitution.

In some embodiments, the method further comprises a step of screening for a cell having the desired one or more nucleotide substitutions from the at least one cell.

In some embodiments, the method of the present invention is performed in vitro. For example, the cell is an isolated cell, or a cell within an isolated tissue or organ.

In another aspect, the present invention also provides a genetically modified organism comprising a genetically modified cell produced by the method of the present invention or a progeny cell thereof. Preferably, the genetically modified cell or a progeny cell thereof has one or more desired nucleotide substitutions.

In the present invention, the target nucleic acid region to be modified can be located at any position of the genome, for example, in a functional gene such as a protein coding gene, or, for example, in a gene expression regulatory region such as a promoter region or an enhancer region, thereby achieving modification of the gene function or modification of gene expression. In some embodiments, the desired nucleotide substitution results in a desired gene function modification or gene expression modification.

In some embodiments, the target nucleic acid region is related to the proterties of the cell or organism. In some embodiments, the mutation in the target nucleic acid region results in a change in the proterties of the cell or organism. In some embodiments, the target nucleic acid region is located in the coding region of protein. In some embodiments, the target nucleic acid region encods the function-related motif or domain of a protein. In some preferred embodiments, one or more nucleotide substitutions in the target nucleic acid region results in amino acid substitution in the amino acid sequence of the protein. In some embodiments, the one or more nucleotide substitutions results in change of the protein function.

In the method of the present invention, the base editing system can be introduced into the cell by various methods well known to those skilled in the art.

Methods that can be used to introduce the base editing system of the present invention into a cell include, but are not limited to, calcium phosphate transfection, protoplast fusion, electroporation, liposome transfection, microinjection, viral infection (such as baculovirus, vaccinia virus, adenovirus, adeno-associated virus, lentivirus and other viruses), gene gun technique, PEG-mediated protoplast transformation, and Agrobacterium-mediated transformation.

The cell that can be base edited by the method of the present invention can be from, for example, mammals such as human, mouse, rat, monkey, dog, pig, sheep, cattle, cat; poultry such as chicken, duck, goose; plants, including monocots and dicots, preferably crop plants, including but not limited to wheat, rice, corn, soybean, sunflower, sorghum, rapeseed, alfalfa, cotton, barley, millet, sugarcane, tomato, tobacco, cassava and potato.

## VII. Application in plants

The base editing fusion protein, base editing system and method for producing a genetically modified cell of the present invention are particularly suitable for genetically modifying plants. Preferably, the plant is a crop plant, including but not limited to wheat, rice, corn, soybean, sunflower, sorghum, rapeseed, alfalfa, cotton, barley, millet, sugarcane, tomato, tobacco, cassava and potato. More preferably, the plant is rice.

In another aspect, the present invention provides a method for producing a genetically modified plant, comprising introducing the base editing system of the present invention into at least one plant, thereby causing one or more nucleotide substitutions within a target nucleic acid region in the genome of the at least one plant.

In some embodiments, the method further comprises screening for a plant having one or more desired nucleotide substitutions from the at least one plant.

In the method of the present invention, the base editing composition can be introduced into plants by various methods well known to those skilled in the art. Methods that can be used to introduce the base editing system of the present invention into plants include, but are not limited to, gene gun method, PEG-mediated protoplast transformation, Agrobacterium-mediated transformation, plant virus-mediated transformation, pollen tube channel method, and ovary injection method. Preferably, the base editing composition is introduced into the plant by transient transformation.

In the method of the present invention, the modification of the target sequence can be achieved by simply introducing or producing the base editing fusion protein and guide RNA in the plant cell, and the modification can be stably inherited without the need to stably transform the exogenous polynucleotides encoding the components of the base editing system into the plant. This avoids the potential off-target effects of the stably existing (continuously produced) base editing composition and also avoids the integration of exogenous nucleotide sequences into the plant genome, thereby having higher biosafety.

In some preferred embodiments, the introduction is carried out in the absence of selection pressure to avoid integration of the exogenous nucleotide sequence into the plant genome.

In some embodiments, the introduction comprises transforming the base editing system of the present invention into an isolated plant cell or tissue and then regenerating the transformed plant cell or tissue into an intact plant. Preferably, the regeneration is carried out in the absence of selection pressure, i.e., no selection agent for the selectable gene on the expression vector is used during tissue culture. Avoiding the use of a selection agent can increase the plant regeneration efficiency, obtaining a modified plant free of exogenous nucleotide sequences.

In some other embodiments, the base editing system of the present invention can be transformed into a specific part of an intact plant, such as leave, shoot tip, pollen tube, young ear or hypocotyl. This is particularly suitable for the transformation of plants that are difficult to regenerate in tissue culture.

In some embodiments of the invention, in vitro expressed protein and/or in vitro transcribed RNA molecule (e.g., the expression construct is in vitro transcribed RNA molecule) are directly transformed into the plant. The protein and/or RNA molecule is capable of performing base editing in the plant cell and is subsequently degraded by the cell, avoiding integration of the exogenous nucleotide sequence in the plant genome.

Thus, in some embodiments, using the method of the present invention for plant genetic modification and breeding can obtain a plant with the genome free of exogenous polynucleotide integration, namely a transgene-free modified plant.

In some embodiments of the present invention, the modified target nucleic acid region is associated with a plant trait, such as a agronomic trait, so that modification results in a plant having an altered (preferably improved) trait, such as an agronomic trait, relative to a wild type plant.

In some embodiments, the method further comprises the step of screening for a plant having the desired modification and/or desired trait, such as agronomic trait.

In some embodiments of the present invention, the method further comprises the step of obtaining progenies of the genetically modified plant. Preferably, the genetically modified plant or progenies thereof have the desired modification and/or the desired trait, such as agronomic trait.

In another aspect, the invention also provides a genetically modified plant or progenies or a part thereof, wherein the plant is obtained by the method according to the present invention as described above. In some embodiments, the genetically modified plant or the progenies or a part thereof is transgene-free. Preferably, the genetically modified plant or progenies thereof have the desired genetic modification and/or the desired trait, such as agronomic trait.

In another aspect, the present invention provides a method of plant breeding comprising crossing a first genetically modified plant containing one or more nucleotide substitutions in the target nucleic acid region obtained by the above method of the present invention with a second plant not containing one or more nucleotide substitutions, thereby the one or more nucleotide substitutions are introduced into the second plant. Preferably, the first genetically modified plant has a desired trait, such as an agronomic trait.

**VIII. Therapeutic applications**

The present invention also emcompases the use of the base editing system of the present invention in disease treatment.

By modifying disease-related genes through the base editing system of the present invention, up-regulation, down-regulation, inactivation, activation or mutation correction of disease-related genes can be achieved, thereby achieving prevention and/or treatment of diseases. For example, the target nucleic acid region described in the present invention can be located in the protein coding region of a disease-related gene, or can be located in a gene expression regulatory region such as a promoter region or enhancer region, thereby achieving functional modification or expression modification of the disease-related gene. Therefore, modification of disease-related genes described herein includes modification to the disease-related genes themselves (such as protein coding regions), as well as modification to their expression regulatory regions (such as promoters, enhancers, introns, etc.).

A "disease- related" gene refers to any gene that produces a transcription or translation product at abnormal levels or in an abnormal form in cells derived from disease-affected tissue as compared to non-disease control tissues or cells. Where altered expression is associated with the emergence and/or progression of a disease, it may be a gene that is expressed at an abnormally high level; or it may be a gene that is expressed at an abnormally low level. Disease- related genes also refer to genes that have one or more mutations or genetic variants that are directly responsible for or in linkage disequilibrium with one or more genes responsible for the etiology of the disease. The mutation or genetic variation is, for example, a single nucleotide variation (SNV). The product of transcription or translation may be known or unknown, and may be at normal or abnormal level.

Accordingly, the invention also provides a method of treating a disease in a subject in need thereof, comprising delivering to said subject an effective amount of the base editing system of the invention to modify a gene related to said disease (e.g., deamination of mitochondrial DNA by a fusion protein or multiple fusion proteins). The invention also provides the use of the base editing system for the preparation of a pharmaceutical composition for treating a disease in a subject in need thereof, wherein the base editing system is used to modify a gene related to said disease. The present invention also provides a pharmaceutical composition for treating a disease in a subject in need thereof, comprising the base editing system of the present invention, and optionally a pharmaceutically acceptable carrier, wherein the base editing system is used to modify a gene related to said disease.

In some embodiments, the fusion protein or base editing system described herein are used to introduce point mutations into nucleic acids by deaminating target nucleobases (e.g., C residues). In some embodiments, deamination of target nucleobases results in correction of genetic defects, such as in correction of point mutations that result in loss of function in gene products. In some embodiments, the genetic defect is associated with a disease or condition (e.g., a lysosomal storage disease or a metabolic disease, such as, for example, Type I diabete). In some embodiments, the method provided herein can be used to introduce an inactivating point mutation into a gene or allele encoding a gene product associated with a disease or disorder.

In some embodiments, the purpose of the embodiment described in the present invention is to restore the function of dysfunctional genes via genome editing. The nucleobase editing protein provided herein is used for in vitro gene editing of human cells, such as correcting disease-related mutations in human cell cultures. The nucleobase editing protein provided herein, such as fusion protein containing nucleic acid editable DNA protein (e.g., CRISPR effector protein Cas9) and cytosine deaminase domain, can be used to correct any single-point T to C or A to G mutations. In the first case, the C of the mutant is corrected by deamination, while in the latter case, the C paired with the mutant A is corrected by deamination and a subsequent round of replication.

In some embodiments, the embodiments described herein are intended for the treatment of patients with diseases associated with or caused by point mutations that can be corrected by the DNA base editing fusion protein provided herein. In some embodiments, the disease is a proliferative disease. In some embodiments, the disease is a genetic disease. In some embodiments, the disease is a neoplastic disease. In some embodiments, the disease is a metabolic disease. In some embodiments, the disease is a lysosomal storage disease.

In some embodiments, the embodiments described herein are intended to be useful in the treatment of mitochondrial diseases or disorders. As used herein, "mitochondrial disease" refers to diseases caused by abnormal mitochondria, such as mitochondrial gene mutations, enzymatic pathways, etc. Examples of disorders include, but are not limited to: neurological disorders, loss of motor control, muscle weakness and pain, gastrointestinal disorders and difficulty swallowing, poor growth, heart disease, liver disease, diabetes, respiratory complications, epilepsy, vision/hearing problems, lactic acidosis, developmental delay, and susceptibility to infection.

Examples of diseases described in the present invention include, but are not limited to, genetic diseases, circulatory system diseases, muscle diseases, brain, central nervous system and immune system diseases, Alzheimer's disease, secretase disorders, amyotrophic lateral sclerosis (ALS) ), autism, trinucleotide repeat expansion disorders, hearing disorders, gene-targeted therapy of non-dividing cells (neurons, muscles), liver and kidney diseases, epithelial cell and lung diseases, cancer, Usher syndrome or retinitis pigmentosa-39, cystic fibrosis, HIV and AIDS, beta thalassemia, sickle cell disease, herpes simplex virus, autism, drug addiction, age-related macular degeneration, schizophrenia. Other diseases treated by correcting point mutations or introducing inactivating mutations into disease-related genes are known to those skilled in the art, and thus the present disclosure is not limited in this regard. In addition to the diseases illustratively described in the present invention, the strategies and base editing system provided by the present invention can also be used to treat other related diseases, and these uses will be obvious to those skilled in the art. For diseases or targets applicable to the present invention, reference can be made to WO2015089465A1 (PCT/US2014/070135), WO2016205711A1 (PCT/US2016/038181), WO2018141835A1 (PCT/EP2018/052491), WO2020191234A1 (PCT/US2020/023713), WO2020191233A1(PCT/ US2020/023712), WO2019079347AI (PCT/US2018/056146), and WO2021155065A1 (PCT/US2021/015580).

Administration of the base editing system or pharmaceutical composition of the invention can be tailored to the weight and species of the patient or subject. The frequency of administration is within the limits permitted by medical or veterinary medicine. It depends on general factors including the patient or subject's age, gender, general health, other conditions, and the specific condition or symptom to be addressed.

## IX. Adeno-associated virus (AAV)

The base editing fusion protein provided by the present invention and/or the expression construct containing the nucleotide sequence encoding the base editing fusion protein, or one or more gRNAs contained in the base editing system of the present invention can be delivered using adeno-associated virus (AAV), lentivirus, adenovirus, or other plasmid or viral vector types. AAV has a packaging limit of 4.5-4.75Kb which indicates that both the promoter and the transcription terminator must be integrated into a same viral vector. Constructs larger than 4.5-4.75Kb will result in a significant reduction in viral delivery efficiency. The large size of cytosine deaminase makes it difficult to be packaged into AAV. Therefore, embodiments of the present invention provide the use of truncated cytosine deaminases for packaging into AAV to achieve base editing.

## X. Nucleic Acid, Cell and Composition

In another aspect, the present invention provides a nucleic acid molecule, which encodes the cytosine deaminase of the present invention, or the fusion protein of the present invention.

In another aspect, the present invention provides a cell comprising the cytosine deaminase of the present invention, or the fusion protein of the present invention, or the base editing system of the present invention, or the nucleic acid molecule of the present invention.

In another aspect, the present invention provides a composition comprising the cytosine deaminase of the present invention, or the fusion protein of the present invention, or the base editing system of the present invention, or the nucleic acid molecule of the present invention.

In some embodiments, the cytosine deaminase, fusion protein, base editing system or nucleic acid molecule is packaged into a virus, a virus-like particle, a virosome, a liposome, a vesicle, an exosome, a liposomal nanoparticle (LNP).

In some embodiments, the virus is an adeno-associated virus (AAV) or a recombinant adeno-associated virus (rAAV).

**XI. Kit**

The present invention also includes a kit for the method of the present invention, the kit comprising the base editing fusion protein of the present invention and/or an expression construct containing a nucleotide sequence encoding the base editing fusion protein, or comprising the base editing system of the present invention. The kit generally includes a label indicating the intended use and/or method for use of the contents of the kit. The term label includes any written or recorded material provided on or with the kit or otherwise provided with the kit. The kit of the present invention may also include suitable materials for constructing an expression vector in the base editing system of the present invention. The kit of the present invention may also include reagents suitable for transforming the base editing fusion protein or base editing composition of the present invention into a cell.

In one aspect, the present invention provides a kit containing a nucleic acid construct, wherein the nucleic acid construct comprises:

(a) a nucleic acid sequence encoding the cytosine deaminase of the present invention; and
(b) a heterologous promoter driving the expression of the sequence of (a).

In one aspect, the present invention provides a kit containing a nucleic acid construct, wherein the nucleic acid construct comprises:

(a) a nucleic acid sequence encoding a fusion protein of the present invention; and
(b) a heterologous promoter driving the expression of the sequence of (a).

In some embodiments, the expression construct further comprises an expression construct encoding a guide RNA backbone, wherein the construct comprises a cloning site that allows a nucleic acid sequence identical or complementary to a target sequence to be cloned into the guide RNA backbone.

**Examples**

In order to facilitate the understanding of the present invention, the present invention will be described more fully below with reference to relevant specific examples and accompanying drawings. Preferred examples of the present invention are provided in the accompanying drawings. However, the present invention can be implemented in many different forms and is not limited to the examples described herein. On the contrary, the purpose of providing these examples is to allow more thorough and comprehensive understanding of the disclosure of the present invention.

**Materials and methods**

1. Vector construction

The sequences of the new deaminases identified were codon-optimized by Nanjing GenScript against both rice and wheat and constructed into the pJIT63-nCas9-PBE backbone (Addgene number #98164). The plasmids of the reporter system used in the examples were constructed by our laboratory previously. pOsU3 vector (Addgene number #170132) was used for sgRNA expression.

2. Protoplast isolation and transformation

The protoplasts used in the present invention were derived from rice variety Zhonghua 11.

2.1 Rice seedling culture

Rice seeds were first rinsed with 75% ethanol for 1 minute, then treated with 4% sodium hypochlorite for 30 minutes, and washed with sterile water for more than 5 times; cultured on M6 medium for 3-4 weeks at 26°C in the dark.

2.2 Protoplast isolation

(1) the rice stems were cut off, the middle parts were cut into 0.5-1 mm small stripes with a blade, placed into 0.6 M Mannitol solution in the dark for 10 min, filtered with a filter, palced into 50 mL enzyme solution (0.45 $\mu$m filter), vacuumed (with a pressure of about 15 Kpa) for 30 min, took out and placed on a shaker (10 rpm) for room temperature enzyme hydrolysis for 5 h;
(2) 30-50 mL W5 was added to dilute the enzymatic hydrolysis product, and the enzymatic hydrolysis solution was filtered with a 75 $\mu$m nylon filter into a round-bottom centrifuge tube (50 mL);
(3) 23°C, 250 g (rcf), up 3 down 3, centrifuged for 3 min, and the supernatant was discarded;
(4) the cells were gently suspended with 20 mL W5 and step (3) was repeated;
(5) appropriate amount of MMG was added to suspend and stored for transformation.

2.3 Rice protoplast transformation

(1) 10 $\mu$g of each vector to be transformed was added to a 2 mL centrifuge tube, mixed well, then a sharpened pipette was used to draw 200 $\mu$L of protoplasts, flicked to mix, 220 $\mu$L of PEG4000 solution was added, flicked to mix, and transformation was induced at room temperature in the dark for 20-30 min;
(2) 880 $\mu$L of W5 was added and gently inverted to mix, centrifuged at 250 g (rcf), up 3 and down 3, and the supernatant was discarded;
(3) 1 mL of WI solution was added, gently inverted to mix, gently transferred to a flow cytometry tube, and cultured in the dark at room temperature for 48 hours.

3. Observation of cell fluorescence by flow cytometry

The FACSAria III (BD Biosciences) instrument was used to analyze the GFP-negative and -positive populations of protoplasts.

4. Protoplast and plant DNA extraction and amplicon sequencing analysis

Protoplasts were collected in 2 mL centrifuge tubes, protoplast DNA (~30 $\mu$L) was extracted using the CTAB method, and its concentration (30-60 ng/$\mu$L) was measured using a NanoDrop ultra-micro spectrophotometer, and DNA was stored at -20°C.
PCR amplification of protoplast DNA template was performed using genomic primers specific for the target sites. The 20 $\mu$L amplification system contained 4 $\mu$L 5$\times$ Fastpfu buffer, 1.6 $\mu$L dNTPs (2.5 mM), 0.4 $\mu$L Forward primer (10 $\mu$M), 0.4 $\mu$L Reverse primer (10 $\mu$M), 0.4 $\mu$L FastPfu polymerase (2.5U/$\mu$L), and 2 $\mu$L DNA template (~60 ng). Amplification conditions: 95°C pre-denaturation for 5 min; 95°C denaturation for 30 s, 50-64°C annealing for 30 s, 72°C extension for 30 s, 35 cycles; 72°C full extension for 5 min, and storage at 12°C.
The above amplification product was diluted 10 times, and 1 $\mu$L was taken as the second round of PCR amplification template. The amplification primer was a sequencing primer containing a barcode. The 50 $\mu$L amplification system contained 10 $\mu$L 5$\times$ Fastpfu buffer, 4 $\mu$L dNTPs (2.5 mM), 1 $\mu$L Forward primer (10 $\mu$M), 1 $\mu$L Reverse primer (10 $\mu$M), 1 $\mu$L FastPfu polymerase (2.5U/$\mu$L), and 1 $\mu$L DNA template. The amplification conditions were the same as above, and the number of amplification cycles was 35.
PCR products were separated by 2% agarose gel electrophoresis, and the target fragments were recovered by gel extraction using AxyPrep DNA Gel Extraction kit. The recovered products were quantitatively analyzed using NanoDrop ultra-micro spectrophotometer. 100 ng of the recovered products were mixed and sent to Novogene for amplicon sequencing library construction and amplicon sequencing analysis.

5. Transfection of Human and animal cells

Human HEK293T cells (ATCC, CRL-3216) and mouse N2a cells (ATCC, CCL131) were cultured in Dulbecco's Modified Eagle's Medium (DMEM, Gibco) supplemented with 10% (vol/vol) fetal bovine serum (FBS, Gibco) and 1% (vol/vol) penicillin-streptomycin (Gibco) at 37°C in a humidified incubator with 5% $CO_2$. All cells were routinely tested for mycoplasma contamination using a mycoplasma detection kit (Transgen Biotech). Cells were seeded into 48-well Poly-D-Lysinecoated plates (Corning) in the absence of antibiotics. After 16-24 hours, cells were incubated with 1 uL Lipofectamine 2000 (ThermoFisher Scientific), 300 ng deaminase vector, and 100 ng sgRNA expression vector. When transfecting the cytosine base editing system, cells were incubated with 1 uL Lipofectamine2000, 300ng TALE-L and 300ng TALE-R. After 72 hours, the cells were washed with PBS and DNA was extracted. To detect off-target effects using the R-loop method, cells were co-transfected with a vector called BE4max, a SaCas9BE4max vector and a corresponding sgRNA vector (Koblan, L. W., Doman, J. L., Wilson, C., Levy, J. M., Tay, T., Newby, G. A., Maianti, J. P., Raguram, A., & Liu, D. R. (2018). Improving cytidine and adenine base editors by expression optimization and ancestral reconstruction. Nat. Biotechnol., 36, 843-846.).

6. TRAPseq library

We used the sgRNA 12K-TRAPseq library for evaluation of properties of the cytosine deaminase base editing system. We seeded $2\times10^6$ cells into 100 mm cell-culture dish (Corning) 20-hours before viral transduction. We transduced 500 μL of sgRNA lentivirus. For stably integrated cells, we used 1 μg/mL of puromycin (Gibco) to select. For each base editor, we seeded $2\times10^6$ cells into 6-plates 24-hours before transfection. We transfected 15 μg of each CBE member plasmid DNA and 15 μg of Tol2 DNA with 60 μL of Lipofectamine 2000. Following 24 hours after transfection, we changed new culturing media to contain 10 μg/mL blasticidin (Gibco). After another 3 days, we washed the cells, suspended and reseeded all cells in 10 μg/mL blasticidin-containing media. After 6 days, we harvested all cells by washing with PBS then centrifuged and extracted DNA using Cell/Tissue DNA Isolation Mini Kit (Vazyme). For each deaminase base editor, we performed next-generating sequencing fot sequence analysis.

7. DNA extraction

For HEK293T cells and N2a cells, genomic DNA was extracted with Lysis Buffer and Proteinase K with a Triumfi Mouse Tissue Direct PCR Kit (Beijing Genesand Biotech).
For plant protoplasts, genomic DNA was extracted with a Plant Genomic DNA Kit (Tiangen Biotech) after 72 hours' incubation. All DNA samples were quantified with a NanoDrop 2000 spectrophotometer (Thermo Scientific).

8. Protein structure analysis and clustering

AlphaFold v2.2.0 was used to analyze protein structure (John Jumper and others, 'Highly Accurate Protein Structure Prediction with AlphaFold', Nature, 596.7873 (2021), 583-89).
The TM-align software was used to calculate the TM-score for the analysis results. The specific calculation formula of TM-score is as follows (reference: Zhang, Yang, and Jeffrey Skolnick. (2004). Scoring function for automated assessment of protein structure template quality. Proteins 57(4), 702-710.)

$$\text{TM-score} = \text{Max}\left[\frac{1}{L_N}\sum_{i=1}^{L_T}\frac{1}{1+\left(\frac{d_i}{d_0}\right)^2}\right]$$

where $L_N$ is the length of the amino acid sequence of the target protein, LT is the length of the amino acid sequence that appears in both the template and the target structure, di is the distance between the i-th pair of residues in the template and the target structure, and d0 is the scale of the normalized matching difference. "Max" indicates the maximum value after optimal spatial superposition.
After convertion of the TM-Score, the APE and phangorn packages in R language were used to perform clustering calculations using the UPGMA method (C. P. Kurtzman, Jack W. Fell, and T. Boekhout, The Yeasts: A Taxonomic Study, 5th ed (Amsterdam: Elsevier, 2011); 'A Statistical Method for Evaluating Systematic Relationships - Robert Reuven Sokal, Charles Duncan Michener - Google Books'); First, the following formula was used to obtain the distance between any two points.

$$d_{(ABX)} = \frac{1}{2}(d_{ax} + d_{bx})$$

where $d_{(ABX)}$ is the distance between two points.

Then, the formula for calculating the average distance used in the clustering process is as follows. If C1, C2 are the terminal taxa containing sets n1 and n2 to be merged into the new set C, then the average distance to any other cluster D is calculated by the following formula:

$$d_{(c_1c_2)D} = \frac{n_1}{n_1 + n_2}d_{C_1D} + \frac{n_2}{n_1 + n_2}d_{C_2D}$$

**Example 1: Identification of novel deaminases in the APOBEC/AID clade that can be used for base editing**

In order to find a new deaminase different from the deaminases used in the existing base editing systems, we first tested the deaminase in the APOBEC/AID clade with low sequence similarity to the existing deaminases in the list of representative deaminases listed in the work of Iyer et al. (Iyer, L. M., Zhang, D., Rogozin, I. B., & Aravind, L. (2011). Evolution of the deaminase fold and multiple origins of eukaryotic editing and mutagenic nucleic acid deaminases from bacterial toxin systems. Nucleic acids research, 39(22), 9473-9497.). Among them, No.182 deaminase (SEQ ID NO: 1) has very low similarity with the existing deaminases, and its amino acid sequence has only 34% sequence identity with the most similar mouse rAPOBEC1. Deaminase 182 was constructed onto the pJIT163-nCas9-PBE backbone, that is, deaminase 182 was used to replace rAPOBEC1 and fused to nCas9. Through evaluation by the reporter system, it was found that 182-PBE could undergo base editing in cells (Figures 1, 6, and 7).

To further confirm its editing ability, the 182-PBE construct and the endogenous-targeting sgRNA construct were co-transformed into rice protoplasts. Analysis of the editing results of six endogenous sites found that 182-PBE can effectively achieve base editing, and its editing window is significantly larger than the commonly used rAPOBEC1-based cytosine base editing system (Figure 2, Figure 6 and Figure 7). Therefore, protein 182 has the function of deaminating cytosine on single-stranded DNA, and a new cytosine base editing system can be established based on this protein.

**Example 2: Detection of cytosine deamination activity of deaminases in different clades**

Iyer et al. searched for proteins with similar folding patterns to known deaminases in the database and divided the above proteins into at least 21 clades according to different structural domains (Table 1). The cytosine deaminases APOBEC1, APOBEC3, AID, and CDA1, which are currently widely used in base editing, are all classified into the APOBEC/AID-like clade. In addition to the above clades, there are also clades with proven functions, such as the "dCMP deaminase and ComE" clade that can convert dCMP into dUMP, the "Guanine deaminase" clade that can convert guanine (G) into xanthine (I), the "RibD-like" clade with diaminohydroxyphosphoribosylamidopyrimidine deaminase function, the "Tad1/ADAR" clade with RNA editing enzyme function that converts RNA adenine (A) to xanthine (I), and the "PurH/AICAR transformylase" clade with formyl transferase activity. In addition, the deamination functions of some clades have not yet been clarified, such as the SCP1.201 clade, XOO2897 clade, MafB19 clade, Pput_2613 clade, etc., which are named based on protein domains and originate from bacteria.

Table 1. Deaminase classification families (Iyer et al., 2011)

| Family name of deaminases | | |
|---|---|---|
| Wolbachia_B3gp45 (AID/APOBEC deaminases) | DYW-like clade | RibD-like deaminases |
| Novel AID/APOBEC-like clade | FdhD clade | SCP1.201 clade |
| CDD/CDA-like cytidine deaminases | MafB19 clade | TM1506 clade |
| Guanine deaminases clade | OTT1508 clade | XOO2897 clade |
| Tad1/ADAR clade | Pput_2613 clade | YwqJ clade |
| Bd3614 clade | PurH/AICAR transformylase clade | JAB clade |
| Toxin deam (BURPS668_1122) clade | TadA-Tad2(ADAT2), Tad3(ADAT3) clade | Imm1 immunity protein associated with SCP1.201 deaminases clade |

In order to detect whether the above clades have cytosine deaminase activity, a total of 48 deaminase proteins were selected from the representative deaminase list listed by Iyer et al., except for the APOBEC/AID clade, distributed in 14 clades: Bd3614, CDD/CDA-like, DYW-like, FdhD, MafB19, Novel AID/APOBEC-like, OTT1508, PurH/AICAR transformylase, RibD-like, TM1506, SCP1.201, Imm1 immunity protein associated with SCP1.201 deaminases, YwqJ and XOO2897. All proteins were constructed on the pJIT163-nCas9-PBE backbone, and their deamination activity upon binding to single-stranded DNA was evaluated by the BFP-to-GFP reporter system (Zong, Y. et al. Nat. Biotechnol. 35, 438-440 (2017)). It was found that 23 proteins from five clades had cytosine deaminase activity, which were from the Novel AID/APOBEC-like clade (No. 2-1479 and No.2-1478), the bacterial SCP1.201 clade (No.69, No.55, No.57, No.64, No.76, No.2-1146, No.2-1160, No.54, No.56, No.59, No.60, No.61, No.72, No.74, No.75, No.63, No.2-1158), the XOO2897 clade (No.2-1429, No.2-1442), the TM1506 clade (No.2-39) and the MafB19 clade (No.101m). In particular, for the SCP1.201 clade, 18 of the 19 proteins tested had cytosine deaminase activity. Through the test of two endogenous sites in rice, the above 23 proteins with cytosine deaminase activity can be divided into 8 high-editing-efficiency (Figures 4 and 5), 8 medium-editing-efficiency (Figures 6 and 7), and 7 low-editing-efficiency deaminases.

In order to further confirm the editing ability of the newly discovered deaminases, candidate deaminase No. 69 was selected for testing from the group that enables the reporter system to emit fluorescence. This protein belongs to the SCP1.201 clade. To further confirm its editing ability, the 69-PBE construct and the endogenous-targeting sgRNA construct were co-transformed into rice protoplasts. Analysis of the editing results of six endogenous sites found that 69-PBE can effectively achieve base editing, and its editing efficiency is significantly greater than the commonly used rAPOBEC1-based cytosine base editing system (Figure 3). Therefore, the newly identified proteins can have the function of deaminating cytosine on single-stranded DNA, and new cytosine base editing systems can be established based on these proteins.

**Example 3: Clustering and discovery of new cytidine deaminases via protein structures**

According to the above examples, an effective method for protein enzyme function identification and screening needs to be proposed in order to efficiently analyze protein functions. Based on the decisive role of three-dimensional structure to a protein's function, comparative analysis and classification clustering of known or predicted protein structures may be an effective method for classifying deaminases into functional clades. Therefore, we combined AI-assisted protein structure prediction, structure calibration and clustering to generate new protein classification relationships between deaminases (Figure 8).

We selected 238 protein sequences annotated as containing deaminase domains and 4 candidate protein sequences from a distant outgroup of the JAB-domain family from the InterPro database (Figure 9). Specifically, we selected 15 candidate genes with a length of at least 100 amino acids from each of the 16 deaminase families and used AlphaFold2 to predict their protein structures. We used the standardized scoring model TM-score to perform multiple structural alignments (MSA) on all candidate proteins. The specific calculation formula of TM-score is as follows (Reference: Zhang, Yang, and Jeffrey Skolnick. (2004). Scoring function for automated assessment of protein structure template quality. Proteins 57(4), 702-710.)

$$\text{TM-score} = \text{Max}\left[\frac{1}{L_N}\sum_{i=1}^{L_T}\frac{1}{1+\left(\frac{d_i}{d_0}\right)^2}\right]$$

where $L_N$ is the length of the amino acid sequence of the target protein, $L_T$ is the length of the amino acid sequence that appears in both the template and the target structure, $d_i$ is the distance between the i-th pair of residues in the template and the target structure, and $d_0$ is the scale of the normalized matching difference. "Max" indicates the maximum value after optimal spatial superposition.

Based on the MSA results, we generated a structural similarity matrix that reflects the overall structural relatedness between proteins. We then organized these similarity matrices into a structure-based dendrogram using the Unweighted Pairwise Grouping Algorithm (UPGMA) (Figure 10). The dendrogram clustered the 238 proteins into 20 unique structural clades, and the deaminases in each clade had different conserved protein domains (Figures 11A and 11B). We found that even without using contextual information such as conserved gene neighborhoods and domain architectures, accurate protein clustering classification can be generated based on protein structures. When using structure-based hierarchical clustering, different clades reflect unique structures, implying different catalytic functions and properties (Figures 11A and 11B). Interestingly, we also found that this structure-based clustering method is more effective than traditional one-dimensional amino acid sequence clustering methods in ranking functional similarity. For example, in the amino acid sequence-based clustering method, adenine deaminases (A_deamin, PFO2137 in the InterPro database) involved in purine metabolism were divided into different clades, while in the structure-based clustering method, they were classified into one deaminase clade.

In addition, we used a structure-based clustering method to divide each of the four deaminase families (dCMP, MafB19, LmjF365940 and APOBEC, as annotated by InterPro) into two independent clades (Figures 11A and 11B). Comparison of protein structures showed that the two clades of these four deaminase families have completely different structures, which may be contrary to their InterPro naming and sequence-based classification (Figures 11B and 12). In summary, protein clustering classification based on artificial intelligence-assisted protein three-dimensional structure provides reliable clustering results and only requires an amino acid sequence without any other genomic reasoning. It is a protein relationship generation strategy more convenient and effective than other methods.

**Example 4: Using a three-dimensional structure tree to identify the function of deaminases that can be used for base editing in the SCP1.201 clade**

By evaluating the functions of the deaminases in each clade in Example 2, we surprisingly found that some deaminases in the SCP1.201 clade have the ability to catalyze the deamination of single-stranded DNA substrates. Previously, these deaminases were annotated as double-stranded DNA deaminase toxin A-like (DddA-like) deaminases in the InterPro database (PF14428). Among them, DddA enzyme is a deaminase that has recently been used in a non-CRISPR double-stranded DNA cytosine base editor (CRISPR-free double-stranded DNA cytosine base editor, DdCBE), which can be used to deaminate double-stranded DNA cytosine bases (NCBI Reference Sequence: WP_006498588.1) (B. Y. Mok, M. H. de Moraes, J. Zeng, D. E. Bosch, A. V. Kotrys, A. Raguram, F. Hsu, M. C. Radey, S. B. Peterson, V. K. Mootha, J. D. Mougous, D. R. Liu, A bacterial cytidine deaminase toxin enables CRISPR-free mitochondrial base editing. Nature 583, 631-637 (2020).).Because of the existence of DddA, all proteins in the SCP1.201 clade where it is located are annotated as double-stranded DNA deaminases (Ddd).

Based on this problem, we used the three-dimensional structure protein function prediction in Example 3 and performed the following work. In order to re-analyze this SCP1.201 clade, we selected all 489 SCP1.201 deaminases from the InterPro database. We also included 7 other proteins that were found to have 35% to 50% similarity with DddA by BLAST alignment but were described separately in InterPro. After identification and coverage screening, we performed a new artificial intelligence-assisted protein structure classification on 332 SCP1.201 deaminases. The results of structural clustering analysis showed that SCP1.201 deaminases were clustered into different subclades with their own unique core domain motifs (Figures 13A-E).

Importantly, we found that DddA and 10 other proteins were clustered in a subclade of SCP1.201. By analyzing the 3D predicted structures of all 11 proteins in this subclade, we found that they have similar core structures to DddA. Given their structural similarities to DddA, we predicted that other proteins in this subclade also have double-stranded DNA cytosine deamination function.

**Example 5. Verification of base editing of deaminases in DddA subclade in animal cells**

In order to evaluate whether the SCP1.201 candidate proteins of the subclade where DddA is located obtained by the prediction method of the present invention in Example 4 have functional similarity with DddA, that is, deamination effect on dsDNA. We designed DdCBEs comprising each deaminase alone or split in half at a residue similar to the site where DddA was split by protein structure alignment and joined together using a dual transcription activator-like effector (TALE) system (the method can be seen in: B. Y. Mok, M. H. de Moraes, J. Zeng, D. E. Bosch, A. V. Kotrys, A. Raguram, F. Hsu, M. C. Radey, S. B. Peterson, V. K. Mootha, J. D. Mougous, D. R. Liu, A bacterial cytidine deaminase toxin enables CRISPR-free mitochondrial base editing. Nature 583, 631-637 (2020).) (Figure 14, Table 2). We evaluated proteins from this Ddd subclade at the JAK2 and SIRT6 loci in HEK293T cells and observed that 13 proteins could perform dsDNA base editing (Table 2). We hereafter referred to these deaminases as Ddds and assigned them to this newly identified Ddd subclade.

Table 2. Catalytic activity of proteins in the subclade where DddA is located

| Name | Gene ID | SEQ ID No. | dsDNA catalytic activity |
|---|---|---|---|
| Ddd1 | SCP177 | 28 | ++ |
| Ddd2 | / | 29 | + |
| Ddd3 | SCP001 | 30 | ++ |
| Ddd4 | / | 31 | + |
| Ddd6 | SCP009 | 32 | + |
| Ddd7 | SCP103 | 33 | ++ |
| Ddd8 | SCP234 | 34 | ++ |

(continued)

| Name | Gene ID | SEQ ID No. | dsDNA catalytic activity |
|------|---------|------------|--------------------------|
| Ddd9 | SCP003 | 35 | ++ |
| Ddd10 | SCP004 | 36 | + |
| Ddd11 | SCP271 | 37 | + |
| Ddd12 | SCP005 | 38 | + |
| Ddd13 | SCP006 | 39 | ++ |
| Ddd14 | SCP007 | 40 | + |

**Example 6. Verification of deaminases of non-DddA subclades in base editing in plant and animal cells**

For comparison, this experiment further evaluated the deamination of other SCP1.201 candidate proteins that were not from the DddA subclade. We randomly selected 24 proteins from them and placed them in our CBE fluorescent reporter system. We found that 22 of them showed detectable fluorescence, and 13 of them were selected to evaluate base editing of endogenous sites under CBE conditions in mammalian cells (Figure 16A, Table 3). Although these proteins were previously annotated as DddA-like proteins, the experimental results showed that these proteins only showed cytosine base editing activity on ssDNA (Figure 13A, Figure 16A and Table 3), but did not show activity on dsDNA (Figure 16B). Based on their functions and effects, we hereafter referred to these ssDNA-targeting proteins from the SCP1.201 clade as single-stranded DNA deaminases (Sdd).

According to the above experimental results, we were surprised to find that most of the protein members from the SCP1.201 clade are Sdd proteins, rather than the DddA-like proteins annotated in the InterPro database (PF14428). We also observed that these Sdd proteins were similar to each other and clearly distinguished from the structures of Ddd proteins, for example, as shown by the structure of Sdd7 (Figures 13D, 13E). Sdd7 is one of the most efficient cytosine base editors for ssDNA editing. Therefore, through the method of the present invention, it is shown that the DddA-like deaminases annotated in the InterPro database (PF14428) should be further subdivided and re-annotated accordingly.

As a control, we also clustered proteins from the SCP1.201 clade based on their one-dimensional amino acid sequences and validated the structure tree with the JAB outgroup, and found that the JAB outgroup members were scattered throughout the tree. These results demonstrate the validity and importance of using protein structure-based classification to compare and evaluate protein relationships.

Table 3. Catalytic activity of proteins in the subclade where DddA is not located

| Name | Gene ID | SEQ ID No. | dsDNA catalytic activity (endogenous sites) | ssDNA catalytic activity (endogenous sites) | ssDNA catalytic activity (reporter sysntem) | sequence preference | | | |
|------|---------|------------|---------------------|---------------------|---------------------|----|----|----|----|
| | | | | | | OC | AC | GC | TC |
| Sdd9 | SCP044 | 2 | × | √ | √ | ++ | ++ | ++ | ++ |
| Sdd5 | SCP017 | 3 | | | √ | ++ | ++ | ++ | + |
| Sdd7 | SCP016 | 4 | × | √ | √ | ++ | ++ | ++ | ++ |
| Sdd4 | SCP014 | 5 | | | √ | ++ | ++ | ++ | ++ |
| Sdd76 | SCP012 | 6 | | | √ | ++ | ++ | ++ | ++ |
| Sdd6 | SCP273 | 7 | | | √ | ++ | ++ | + | + |
| / | SCP021 | 8 | | | √ | ++ | + | + | - |
| / | SCP038 | 9 | | | √ | + | + | - | - |
| / | SCP051 | 10 | | | √ | - | - | - | + |
| Sdd59 | SCP183 | 11 | × | √ | √ | + | - | - | |
| Sdd10 | SCP018 | 12 | | | √ | + | + | - | + |
| / | SCP157 | 14 | | | √ | - | + | - | + |
| Sdd3 | SCP170 | 17 | | | √ | - | + | + | - |

(continued)

| Name | Gene ID | SEQ ID No. | dsDNA catalytic activity (endogenous sites) | ssDNA catalytic activity (endogenous sites) | ssDNA catalytic activity (reporter sysntem) | sequence preference | | | |
|---|---|---|---|---|---|---|---|---|---|
| | | | | | | OC | AC | GC | TC |
| / | 2-1158 | 18 | | | √ | + | - | - | - |
| / | SCP158 | 42 | | | √ | + | + | - | + |
| / | SCP315 | 43 | × | √ | √ | ++ | + | + | ++ |
| / | SCP020 | 44 | × | 4 | √ | ++ | + | ++ | + |
| / | 2-1156 | 45 | | | √ | + | + | + | + |
| Sdd2 | Sdd2 | 46 | | | √ | + | + | - | + |
| / | SCP008 | 47 | | | √ | + | + | - | - |
| / | SCP011 | 48 | | | √ | + | - | - | - |
| / | SCP013 | 49 | | | √ | + | - | - | - |
| / | SCP090 | 66 | × | × | × | - | - | - | - |
| / | SCP015 | 67 | × | × | × | - | - | - | - |
| / | SCP278 | 68 | × | × | × | - | - | - | - |
| / | SCP287 | 69 | × | × | × | - | - | - | - |
| / | SCP341 | 70 | × | × | × | - | - | - | - |
| / | SCP353 | 71 | × | × | × | - | - | - | - |
| / | SCP357 | 72 | × | × | × | - | - | - | - |
| / | SCP372 | 73 | × | × | × | - | - | - | - |
| The symbol ++ indicates strong editing preference, + indicates weak editing preference, - indicates no editing preference | | | | | | | | | |

In addition, the verification results of protein functions in Examples 5 and 6 were comprehensively analyzed. The results show that protein clustering classification based on artificial intelligence-assisted protein three-dimensional structure provides reliable clustering results, and the three-dimensional structure tree constructed using the method of the present invention can accurately identify and predict the detailed functions of proteins. And it only requires an amino acid sequence and does not require any other genome reasoning, making it a protein relationship generation strategy more convenient and effective than other methods. In the three-dimensional structural tree, when the TM-score of not less than 0.7 is used as the clustering criterion, the prediction of protein catalytic function by the clustering results is consistent with the experimental verification conclusion (Table 4). That is, when clustering is performed based on a TM-score of not less than 0.7 as compared to the reference protein, the obtained subclade has the same or similar catalytic function as the reference protein. The method of the present invention significantly improves the identification and prediction efficiency.

Table 3. Catalytic activity of proteins in the subclade where DddA is not located

| Name | Gene ID | SEQ ID No. | dsDNA catalytic activity (endogenous sites) | ssDNA catalytic activity (endogenous sites) | ssDNA catalytic activity (reporter sysntem) |
|---|---|---|---|---|---|
| Sdd9 | SCP044 | 2 | × | √ | √ |
| Sdd5 | SCP017 | 3 | | | √ |
| Sdd7 | SCP016 | 4 | × | √ | √ |
| Sdd4 | SCP014 | 5 | | | √ |
| Sdd76 | SCP012 | 6 | | | √ |
| Sdd6 | SCP273 | 7 | | | √ |

(continued)

| Name | Gene ID | SEQ ID No. | dsDNA catalytic activity (endogenous sites) | ssDNA catalytic activity (endogenous sites) | ssDNA catalytic activity (reporter sysntem) |
|---|---|---|---|---|---|
| / | SCP021 | 8 | | | √ |
| / | SCP038 | 9 | | | √ |
| / | SCP051 | 10 | | | √ |
| Sdd59 | SCP183 | 11 | × | √ | √ |
| Sdd10 | SCP018 | 12 | | | √ |
| / | SCP157 | 14 | | | √ |
| Sdd3 | SCP170 | 17 | | | √ |
| / | 2-1158 | 18 | | | √ |
| / | SCP158 | 42 | | | √ |
| / | SCP315 | 43 | × | √ | √ |
| / | SCP020 | 44 | × | √ | √ |
| / | 2-1156 | 45 | | | √ |

**Example 7. New Ddd proteins have distinct editing preferences to DddA**

Due to the strict preference of DddA for the 5'-TC motif, the application of DddA-based dsDNA base editors is mainly limited to TC targets (B. Y. Mok, M. H. de Moraes, J. Zeng, D. E. Bosch, A. V. Kotrys, A. Raguram, F. Hsu, M. C. Radey, S. B. Peterson, V. K. Mootha, J. D. Mougous, D. R. Liu, A bacterial cytidine deaminase toxin enables CRISPR-free mitochondrial base editing. Nature 583, 631-637 (2020)). Although the recently evolved DddA11 shows more general applicability and can be used for deamination of 5'-HC (H = A, C or T) motifs to achieve cytosine base editing, the editing efficiency of AC, CC and GC targets still needs to be improved (B. Y. Mok, A. V. Kotrys, A. Raguram, T. P. Huang, V. K. Mootha, D. R. Liu, CRISPR-free base editors with enhanced activity and expanded targeting scope in mitochondrial and nuclear DNA. Nat. Biotechnol. 40, 1378-1387). We evaluated the newly discovered Ddd proteins of the present invention to determine whether they could expand the utility and targeting scope of DdCBEs. We constructed 13 deaminases belonging to the Ddd subclade into DdCBE and evaluated the dsDNA base editing of endogenous JAK2 and SIRT6 sites in HEK293T cells (Figure 15, Figure 18 and Table 2). Interestingly, we found that Ddd1, Ddd7, Ddd8, and Ddd9 had similar or higher editing efficiencies than DddA (Figures 17A and 18). Importantly, we found that Ddd1 and Ddd9 had much higher editing activity than DddA at the 5'-GC motif (Figures 17A and 18). Strikingly, at the C10 (5'-GC) residue in JAK2 and the C11 (5'-GC) residue in SIRT6, we found that the editing rates of DddA were only 21.1% and 0.6%, respectively, while the editing rates of Ddd9 were 65.7% and 45.7%, respectively (Figure 17A).

Because certain Ddd proteins seemed to exhibit distinct editing patterns, compared with DddA, we sought to evaluate any sequence motif preference for these Ddd proteins. We first constructed plasmids encoding the JAK2 target sequence (B. Y. Mok, A. V. Kotrys, A. Raguram, T. P. Huang, V. K. Mootha, D. R. Liu, CRISPR-free base editors with enhanced activity and expanded targeting scope in mitochondrial and nuclear DNA. Nat. Biotechnol. 40, 1378-1387) and modified the GCC at positions 9-11 to MCN (M/N = A, T, C, and G) at positions 9-11, thereby obtaining 16 different plasmids, and co-transfected each plasmid with a DdCBE variant (Figure 17B). After comparative analysis of the C•G-to-T•A base conversion frequencies for each MCN, we generated corresponding motif logos to reflect the sequence context preference of each dsDNA deaminase (Figure 17C). As mentioned above, we found that DddA and its structural homolog Ddd7 strongly preferred the 5'-TC motif (Figure 17C, Figure 19). In contrast, we found that Ddd1 and Ddd9 preferred to edit substrates with 5'-GC motifs, while Ddd8 preferred to edit substrates with 5'-WC (W=A or T) motifs. Therefore, through a deeper analysis of the Ddd subclade, we discovered a whole set of new Ddd proteins that can be used for different motif editing, which greatly expanded the targeting range and practicality of DdCBE and showed great application potential (Figure 17C, Figure 19).

**Example 8. Sdds enable base editing in human cells and plants**

We next wondered whether the newly characterized Sdd proteins could be used for more precise or efficient base editing. We chose to evaluate the six most active Sdds as well as four weaker Sdds and compared their activities using a fluorescent reporter system. We designed plant CBEs for each of the 10 Sdds and evaluated their endogenous base editing at six sites in rice protoplasts (Figures 20 and 21). We found that seven of the deaminases (Sdd7, Sdd9, Sdd5, Sdd6, Sdd4, Sdd76, and Sdd10) had higher activity, compared with the rat APOBEC1 (rAPOBEC1)-based CBE. The most active Sdd7 base editor reached as high as 55.6% cytosine base editing, which was more than 3.5-fold higher than that of rAPOBEC1.

To examine the versatility of these deaminases, we also constructed the corresponding human-cell-targeting BE4max vectors (L. W. Koblan, J. L. Doman, C. Wilson, J. M. Levy, T. Tay, G. A. Newby, J. P. Maianti, A. Raguram, D. R. Liu, Improving cytidine and adenine base editors by expression optimization and ancestral reconstruction. Nat. Biotechnol. 36, 843-846 (2018)) and evaluated their editing efficiencies across three endogenous target sites in HEK293T cells. In agreement with the results in rice, we found that Sdd7 had the highest editing activity in HEK293T cells (Figure 22).

We previously showed that human APOBEC3A (hA3A) performed robust base editing with a large editing window in plants (Y. Zong, Q. Song, C. Li, S. Jin, D. Zhang, Y. Wang, J.-L. Qiu, C. Gao, Efficient C-to-T base editing in plants using a fusion of nCas9 and human APOBEC3A. Nat. Biotechnol. 36, 950- 953 (2018), Q. Lin, Z. Zhu, G. Liu, C. Sun, D. Lin, C. Xue, S. Li, D. Zhang, C. Gao, Y. Wang, J.-L. Qiu, Genome editing in plants with MAD7 nuclease. J. Genet. Genomics 48, 444-451 (2021)). We therefore compared the editing activities of hA3A and Sdd7 in human cells (Figure 22) and plants (Figure 23). Interestingly, Sdd7 had comparable editing activities to hA3A across all three target sites in HEK293T cells (Figure 22) and five endogenous sites in rice protoplasts (Figure 23). These results confirmed that Sdd7 is a robust CBE for use in both plants and human cells.

### Example 9. Sdd proteins have unique base editing characteristics

When evaluating endogenous base editing, we observed different editing patterns by the different Sdd-CBEs across all tested genomic target sites in both human and rice cells. For instance, while Sdd7, Sdd9, and Sdd6 showed no particular motif editing preference, Sdd3 seemed to prefer editing 5'-GC and 5'-AC motifs and strongly disfavor editing 5'-TC and 5'-CC motifs (Figure 24). To better profile the editing patterns of each deaminase, we used Targeted Reporter Anchored Positional Sequencing (TRAP-seq), a high-throughput approach for parallel quantification of base editing outcomes (Xi Xiang, Kunli Qu, Xue Liang, Xiaoguang Pan, Jun Wang, Peng Han, Zhanying Dong, Lijun Liu, Jiayan Zhong, Tao Ma, Yiqing Wang, Jiaying Yu, Xiaoying Zhao, Siyuan Li, Zhe Xu, Jinbao Wang, Xiuqing Zhang, Hui Jiang, Fengping Xu, Lijin Zou, Huajing Teng, Xin Liu, Xun Xu, Jian Wang, Huanming Yang, Lars Bolund, George M. Church, Lin Lin, Yonglun Luo. (2020). Massively parallel quantification of CRISPR editing in cells by TRAP-seq enables better design of Cas9, ABE, CBE gRNAs of high efficiency and accuracy. bioRxiv 2020.05.20.103614). A 12K TRAP-seq library comprised of 12,000 TRAP constructs, each containing a unique gRNA expression cassette and the corresponding surrogate target site, was stably integrated into HEK293T cells by lentiviral transduction. Following cell culture and antibody selection, base editors were stably transfected into this 12K-TRAP cell line followed by 10 days of blasticidin selection (Figure 25A). On day 11 post transfection, we extracted the genomic DNA and performed deep amplicon sequencing to evaluate the editing products of each deaminase (Figure 25A). We found that Sdd7 and Sdd6 showed no strong sequence context preference, but rAPOBEC1 had a strong preference for 5'-TC and 5'-CC bases while disfavoring 5'-GC and 5'-AC bases (Figure 25B). By contrast, Sdd3 showed an entirely complementary pattern preferring to edit 5'-GC and 5'-AC bases while showing nearly no activity toward 5'-TC and 5'-CC bases (Figure 25B). Interestingly, we found that Sdd6 and Sdd3 had different editing windows and preferred to edit positions +1 to +3 distal to the PAM, as compared with rAPOBEC1 and Sdd7 (Figure 25B). In conclusion, the newly identified Sdd base editors show unique base editing properties such as increased editing efficiencies, disparate deamination preferences, and altered editing windows, compared with conventional cytosine base editors.

### Example 10. High-fidelity editing properties of Sdd protein

It was previously described that CBEs could cause genome-wide Cas9-independent off-target editing outcomes, which raises concerns about the safety of these precise genome editing technologies for clinical applications. We thought that these off-target mutations may be a result of overexpression of the cytidine deaminase. We wondered whether the newly discovered Sdd proteins could offer a more favorable balance between off-target and on-target editing. We therefore evaluated the Cas9-independent off-target effects of 10 Sdds, using an orthogonal R-loop assay in rice protoplasts. We found that 6 (Sdd2, Sdd3, Sdd4, Sdd6, Sdd10, and Sdd59) of the 10 deaminases had lower off-target activities than rAPOBEC1. Interestingly, while Sdd6 showed nearly no off-target editing activity, it was still robust at on-target base editing when tested across six endogenous sites in rice protoplasts (Figures 26A and Figure 27). When we analyzed the on-target off-target ratios of these 10 deaminases, Sdd6 exhibited the highest on-target off-target editing ratio, which was 37.6-fold higher than that of rAPOBEC1 (Figure 26B). We further compared the on-target and off-target editing of Sdd6 to that of rAPOBEC1 and its two high-fidelity deaminase variants, YE1 and YEE, in HEK293T cells. Importantly, we found that Sdd6 had the highest on-target off-target editing ratios, which were calculated to be 2.8-, 2.1-, and 2.5-fold higher than that of rAPOBEC1, YE1, and YEE, respectively, and 10.4-fold higher than that of hA3A (Figures 26C and Figure 28). Notably, the on-target activity of Sdd6 was comparable to that of rAPOBEC1 and much higher than that of YE1 and YEE (Figure 28). Thus, we identified that the SCP1.201 clade contains unique and more precise Sdd proteins to be used as high-fidelity base editors.

**Example 11. Rational design of Sdd proteins assisted by AlphaFold2 structure prediction**

Although viral delivery of CBEs has great potential for disease treatment, the large size of APOBEC/AID-like deaminases restricts their ability to be packaged into single-AAV particles for in vivo editing applications (31). Others have developed dual-AAV strategy delivery approaches by splitting CBEs into an amino-terminal and carboxy-terminal fragment and packaging them into separate AAV particles. However, these delivery efforts would challenge large-scale manufacturing, require higher viral dosages, and would pose potential safety challenges for human use. Recently, a truncated sea lamprey cytidine deaminase-like 1 (PmCDA1)-based CBE was developed that could be packaged into a single AAV, but these vectors had almost no editing activity in HEK293T cells. As SCP1.201 deaminases are canonically compact and conserved, we thought that they might be the ideal protein for developing single-AAV packaged CBEs. The present invention attempts to use artificial intelligence-assisted three-dimensional structural protein modeling to further design and shorten the size of the newly discovered Sdd proteins.

We first compared the AlphaFold2 predicted structures of all active Sdd deaminases and found that they have a conserved core structure (Figures 13D, 13E and Figure 29). We then generated multiple truncated variants of Sdd7, Sdd6, Sdd3, Sdd9, Sdd10, and Sdd4, and tested these variants for endogenous base editing in rice protoplasts across two sites each. We identified mini-Sdd7, mini-Sdd6, mini-Sdd3, mini-Sdd9, mini-Sdd10, and mini-Sdd4 as newly minimized deaminases that are small (~130-160 aa) and have comparable or higher editing efficiencies, compared with their full-length proteins, both in rice protoplasts and human cells (Figures 30A). Strikingly, all six mini deaminases would permit the construction of single-AAV-packaged SaCas9-based CBEs (<4.7 kb) (Figures 30B). We used mini-Sdd6 to construct a single-AAV SaCas9 vector and found that it had editing efficiencies of around 60% in mouse neuroblastoma N2a cells at two sites in the Mus musculus 4-hydroxyphenylpyruvate dioxygenase (HPD) gene by transient transfection (Figure 30C). These results highlight that the Sdd proteins offer greater advantages over APOBEC/AID-like deaminases in terms of AAV-based CRISPR base editing delivery. The success in further shortening Sdd proteins for AAV packaging highlights the great potential of protein function prediction of the present invention based on three-dimensional structure.

**Example 12: Base editing capability of CBEs based on new Sdds**

We next explored the use of newly engineered Sdd proteins for base editing in plants. We first evaluated the use of mini-Sdd7 in Agrobacterium-mediated genome editing of rice, and observed more positive rice plants, greater proportion of edited plants and higher editing efficiency by CBEs based on mini-Sdd7 as compared with the most used human A3A (hA3A)-based CBE in agricultural application, which reflects a higher efficiency and lower toxicity compared with the hA3A-based CBE (Figure 31).

Soybean is one of the most important staple crops grown around the world, serving as an essential source of vegetable oil and protein. Although base editing has been proven in soybean, it remains challenging and poorly efficient across most sites tested in soybean crops. To understan whether our newly developed Sdd-based CBEs would result in superior cytosine base editing in soybeans, we constructed vectors using the AtU6 promoter driving sgRNA expression and the CaMV 2×35S promoter driving CBE expression and evaluation was conducted in transgenic soybean hairy roots after Agrobacterium-mediated transformation (Figure 32). We found that the APOBEC/AID-like deaminases had low editing activities across all five sites evaluated, including at the GmALS1-T2 and GmPPO2 sites that were particularly difficult to edit by other CBEs in soybean (Figure 30D). Remarkably, mini-Sdd7 displayed 26.3-, 28.2-, and 10.8-fold increased cytosine base editing levels, compared with other deaminases rAPOBEC1, hA3A, and human AID (hAID), respectively, across the 5 sites and reached editing efficiencies up to 67.4% (Figure 30D). Therefore, we can focus on utilizing these newly discovered Sdd proteins to overcome the limitations of efficient cytosine base editing in soybean crops.

We next sought to use mini-Sdd7 to base edit and obtain transgenic soybean plants following Agrobacterium tumefaciens-mediated transformation. We chose to edit the endogenous GmPPO2 gene to create an R98C mutation, which would result in carfentrazone-ethyl-resistant soybean plants. We obtained 77 transgenic soybean seedlings from three independent transformation experiments, of which 21 were heterozygous for base editing (Figure 30E, F). After treatment with carfentrazone-ethyl for 10 days, we could obviously observe that while the wild-type plant was sensitive to wilting and could not generate roots, the mutated plants grew well and normal (Figure 30G). The development of efficient cytosine base editors for use in soybean plants could enable diverse applications in the future.

**Example 13. Base editing properties of proteins from other families**

In addition to the detailed classification and verification of proteins in the SCP1.201 family, we also verified the deaminase function and preference of other families in the Iyer deaminase classification families (Table 1). According to our analysis and verification methods, we also found a series of deaminases with similar Sdd activity in other families such as MafB19, AID/APOBEC, Novel AID/APOBEC-like, TM1506, Toxin deam, XOO2897, etc. (see Table 5 for specific deaminases). Taking the MafB19 family as an example, in Example 2, we have found that some proteins in the MafB19 clade (No.101m) have the function of single-strand deaminases. And in Example 3, based on the clustering classification of artificial intelligence-assisted three-dimensional protein structures, we found that there are two clades with completely different structures in the MafB19 deaminase family (Figures 11B and 12). Using the deaminase screening and identification method of the present invention, we found that three proteins in the MafB19 family (No.2-1241, No.2-1231, and No.99) also have Sdd catalytic activity, and obtained the motif preference of their base editing sequences (Table 5). The various new cytosine base deaminases with different editing characteristics screened by the method of the present invention enrich the base editing tools, expand the base editing systems, and enhance the ability to accurately manipulate target DNA sequences.

Table 5. Catalytic and editing properties of proteins from different families

| Gene ID | SEQ ID No. | Family | ssDNA catalytic activity | sequence preference | | | |
|---|---|---|---|---|---|---|---|
| | | | | CC | AC | GC | TC |
| 2-1241 | 56 | MafB19 | √ | ++ | ++ | ++ | ++ |
| 2-1231 | 57 | MafB 19 | √ | ++ | + | ++ | + |
| 99 | 58 | MafB19 | √ | ++ | + | + | + |
| 101m | 19 | MafB19 | √ | - | - | - | + |
| 2-1223 | 76 | MatB19 | × | - | - | - | - |
| 2-1224 | 77 | MafB19 | × | - | - | - | - |
| 2-1228 | 78 | MafB19 | × | - | - | - | - |
| 2-1229 | 79 | MafB19 | × | - | - | - | - |
| 2-1230 | so | MafB19 | × | - | - | - | - |
| 2-1234 | 81 | MafB19 | × | - | - | - | - |
| 2-1235 | 82 | MafB19 | × | - | - | - | - |
| 3-2107A_cl21x07 | 83 | MafB19 | × | - | - | - | - |
| 4-2130A_cl21x30 | 84 | MafB19 | × | - | - | - | - |
| 181 | 63 | AIDIAFOBEC | √ | ++ | ++ | ++ | ++ |
| 182 | 1 | AIDIAFOBEC | √ | ++ | ++ | ++ | ++ |
| 2-1479 | 20 | Novel AID/APOBEC-like | √ | + | + | - | + |
| 2-1478 | 21 | Novel AID/APOBEC-like | √ | + | - | - | - |
| 2-39 | 22 | TM1506 | √ | + | - | - | - |
| 2-1128 | 74 | Toxin deam | √ | ++ | + | ++ | ++ |
| 2-1114 | 75 | Toxin deam | √ | ++ | + | + | + |
| 2-1430 | 59 | XOO2897 | √ | ++ | ++ | ++ | + |
| 2-1429 | 23 | XOO2897 | √ | ++ | ++ | ++ | ++ |
| 2-1440 | 60 | XOO2897 | √ | + | + | + | + |
| 2-1432 | 61 | XOO2897 | √ | + | + | + | + |
| 2-1437 | 62 | XOO2897 | √ | + | + | + | + |

(continued)

| Gene ID | SEQ ID No. | Family | ssDNA catalytic activity | sequence preference | | | |
|---|---|---|---|---|---|---|---|
| | | | | CC | AC | GC | TC |
| 2-1442 | 24 | XOO2897 | √ | + | + | - | + |
| The symbol ++ indicates strong editing preference, + indicates weak editing preference, - indicates no editing preference | | | | | | | |

**Discussion**

CBEs based on traditional deaminases have the disadvantages of low editing efficiency, small editing window, obvious preference, etc. A series of new cytosine deaminases were obtained by using the three-dimensional structure-based protein function prediction method of the present invention.

These cytosine deaminases have been shown to have good application potential and broad application scenarios. For example, in transgenic soybean hairy roots after Agrobacterium-mediated transformation, we found that the editing activity of APOBEC/AID deaminases was low across all five evaluated sites, including the GmALS1-T2 and GmPPO2 sites, which are particularly difficult to be edited by other CBEs in soybean. Notably, mini-Sdd7 showed 26.3-fold, 28.2-fold, and 10.8-fold higher cytosine base editing levels at the five sites compared with rAPOBEC1, hA3A, and hAID, respectively, with an editing efficiency of up to 67.4%. Therefore, we emphasize the use of these newly discovered Sdd proteins to overcome the limitations of efficient cytosine base editing in soybean crops. Next, we sought to use mini-Sdd7 for base editing to obtain Agrobacterium-mediated transgenic soybean plants. We chose to edit the endogenous GmPPO2 gene to generate R98C mutation, which would produce soybean plants resistant to carfentrazone-ethyl. We obtained two base-edited heterozygotes from 30 transgenic soybean seedlings. After 10 days of treatment with carfentrazone-ethyl, we could clearly observe that the wild-type plants were sensitive to wilting and could not generate roots, but the mutant plants grew well and normally. The development of efficient cytosine base editors for soybean plants can enable multiple applications in the future. We believe that future sequencing efforts in parallel with structure prediction will greatly advance the mining, tracking, classification, and design of functional proteins. Currently, only a few cytosine deaminases have been used in cytosine base editors. Only canonical efforts based on protein engineering and directed evolution have helped to diversify editing properties, however, these efforts are usually difficult to establish. Using our three-dimensional structure-based clustering prediction method, we discovered and analyzed a set of deaminases with different properties. For example, among the newly discovered deaminases, we found that Sdd7 and Sdd6 have great promise in both therapeutic and agricultural applications. Sdd7 was capable of robust base editing in all tested species and had much higher editing activity than the most commonly used APOBEC/AID-like deaminases. Surprisingly, we found that Sdd7 was able to perform efficient editing in soybean plants, where editing of cytosine bases has previously been difficult in soybean plants (plant genes generally have high GC sequence content). We speculate that Sdd7, derived from the bacterium Actinosynnema minim, may possess high activity at temperatures suitable for soybean growth, in contrast to the mammalian APOBEC/AID-like deaminases. While profiling Sdd6, we found that this deaminase was by default more specific than the other deaminases, while maintaining high on-target editing activity. Interestingly, we found that AlphaFold2-based modeling further enabled our protein engineering efforts to minimize protein size, which is critical for viral delivery for using these editing technologies in in vivo therapeutic applications.

The above are only preferred embodiments of the present invention. It should be pointed out that a skilled person in the art can make certain improvements and supplements without departing from the method of the present invention. These improvements and supplements should also be regarded as within the scope of protection of the present invention.

**Related sequences and brief description thereof**

>SEQ ID NO:1 No.182 (AID/APOBEC clade)

MICKLDSVLMTQKKFIFHYKNVRWARGRHETYLCFVVKRRIGPDSLSFDFGHLRNRSGCHVELLF
LRHLGALCPGLSASSVDGARLCYSVTWFCSWSPCSKCAQQLAHFLSQTPNLRLRIFVSRLYFCDEE
DSVEREGLRHLKRAGVQISVMTYKDFFYCWQTFVARRERSFKAWDGLHENSVRLVRKLNRILQP
CETEDLRDVFALLGL

>SEQ ID NO:2 Sdd9/SCP044/No.69 (SCP1.201 clade)

MLDVDDPHTFYVLAGKTPVLVHNSECPIWVKNALQELVGRKETSGKVFDVDGKPIGPDIQSGYKD
RELRRGVYETLRKSPFFQKHFPSTATWYVSLHVEAQYAVWMLRNRIKHATVVINNTYVCSDMNR
LHDNCMTAVPHILPEGYTMTVWKADRTEVTLRGKAPKE

>SEQ ID NO:3 Sdd5/SCP017/No.55 (SCP1.201 clade)


MGPLLDGIAARLEAVRAALLGEGGGAGDDEPPAVPWDRVERLRRELPPPVVPNTGQKTHGRWIG
PDGQARPIVSGRDDKSVLVNPLLRGKGAPGPTRRDSDVEMKLAAHMAARGIRHATVVINNTPCR
GPLRCDTLVPILLPEGSTLTVHGINENGTRTRIRYTGGARPWWS

>SEQ ID NO:4 Sdd7/SCP016/No.57 (SCP1.201 clade)


MLEAVRARLIGEGGGPGAVPEGGDGPPAVPAEEVERLRGELPPPVVPGTGQKTHGRWIGPDGRVR
AIVSGRDEDAALVHAQLAAKGIPDEPTRNSDVEQKLAAHMVANGIRHVTLVINHRPCRGFDDSCD
TLVPIILPEGCTLTVHGQTDKGMRVRVRYTGGARPWWS

>SEQ ID NO:5 Sdd4/SCP014/No.64 (SCP1.201 clade)


MLDAMDAYLSEIAGGNAPARAGPKAPEPKQPGGSSSPRARDGRIDFRALLERLKAQGVVGLEGRS
DDPIPDFDPKKQNPACYQGLAPRQKGKPVRGNLFFPDGRRWNDVALESSRGEPAFDLNIIKPEYRS
LSPARGHLEGNVAAWMRSTFHQEMVLYINESPCRKHGKGCLYTLEHFLPRGYVLHVWSRNDRGE
WRGNTFRGSGEAFTEGA

>SEQ ID NO:6 Sdd76/SCP012/No.76 (SCP1.201 clade)


MYVLAGNTPVLVHNTGPGCGEPGFVSDAANSLSGRRITTGQIFDASGNPIGPEITSGGGSLADRAQ
SYLADSPNIRNLPAKARYASADHVEAQYAVWMRENGVTDASVVINQNYVCGLPLGCQAAVPAIL
PRGSTMTVWYPGSGSPIVLRGVG

>SEQ ID NO:7 Sdd6/SCP273/No.2-1146 (SCP1.201 clade)


MVETRDKIIAAKSRSDAGLLAFQQATNGSIDSRPAEAIANLQRAKTHLDEAQRLVANSDAAVDNY
INAILGGASAATAQPSAVIPASKPSRFKPMRTDPAKADEIRPHVGKDRAVATLWDADGNRVLGLH
SADDDGPAATAAWKPPWRDYVRLRRHVEAHAAARMHQDGHKTMVMYINLPPCKYFDGCKLNL
EDILPKGSTLWMHRVFQNGGTKIYQFNGTGRAYV

>SEQ ID NO:8 SCP021/No.2-1160 (SCP1.201 clade)


MPIRLSGGGLNLYQYAPETNNWIDPLGCSGHRRRHEKMPPEGARLTTGNLFRHAQDEGISPILSSKN
DEFYYRLIKIYAGTGMLNANIRGIASHVEPKAGLILNEDGSGWKIGSLYINYPNGPCLDCRTLMPFI
LNDGSILYVTFPTLGLDGYSYGHFHGREPGFFREGTPCNLPHPE

>SEQ ID NO:9 SCP038/No.54 (SCP1.201 clade)


MADTPQPDSNPLPARDGQRLDREQAEAIRAQLPPTVKPGTGQKTHGCWVDEQGQPQSVTSGQDN
SAAAVWARLQALGIPLSGPPTATADVEQKVAIQMIQQGRQHVDVVINNEPCRGRFSCDTLVPIILP
EGSSLTVHGTNGFRKTYTGGAKPPWSR

>SEQ ID NO:10 SCP051/No.56 (SCP1.201 clade)


MRAGCGVPDGRTRSAISGKDEGFALALRTIRELGMTRGFPLRAADVEMKVASEMRANGITSATLV
INHVPCDDGMFSCDRMVPVLLPAGSTLTVFGAGGFRMTYHGGEQLPCPTP

>SEQ ID NO:11 Sdd59/SCP183/No.59 (SCP1.201 clade)

MLLTPPPRPAAPPTTRPKPLVARTGDAYPPGTEWALPLIVQPHPPVGGTVPVEGHVRALRPESQISH
VFHPGGGHWTEQARARLRVLPGFGWAVNLGHHVELQIAAWMTACGIHHAELVLNRPPCGERYG
LGCHQALPVLLPRGYRLTVSSTRGGPQPYQHHYEGKA

>SEQ ID NO:12 Sdd10/SCP018/No.60 (SCP1.201 clade)


MLDAALGAVRRIIAALGTSGAERASPGANGSERVDELAERLPPTVVPNTSAKTHGWWFTGQGAA
QELISGEGPDARAAYEALREEGYPRPGMPFVAMHVEIKLAAHMRRNDIEHATVVINNIPCPLVWG
CENLIGVVLPEGSSLTVHGSNGYERTFTGGRKPPWPR

>SEQ ID NO:13 SCPO11/No.61 (SCP1.201 clade)


MLGGVLPARSVMFPGHVEPDAHFGPNPERHHPALVEVPIVWAGRQEDRTSTWARRVQRGFPRYT
VGAKTAGMFYNAGSQSWELLSGVDHRGGLTRKASQHISRMLSSGFFDGKPLDTKSDHLRMLNYT
STHVETKAAIWARDSDQETIDVVTNRNYVCGESYDPDDVDEPPGCYQAVESVLREGQTMRVWTT
DPENRVITIHGKGM

>SEQ ID NO:14 SCP157/No.72 (SCP1.201 clade)


MAAGGGVSRPPATRDGAANPTRVPNPPEWLPGWLTEAARDLPRRQAKDPTSGVALINGERIPMRS
GRDPAAAADLKAAYKLIATTTDHLEAKLAARMRRDQVMHAEVLTNNPPCDYEPYGCEKILSRLL
PAGAQLSVYVRDDDDQVRLWRTYIGNGKAIA

>SEQ ID NO:15 SCP008/No.74 (SCP1.201 clade)


MTTGGGSDISRPPATRDTSATATEAPAQPELVPEGLTDAARDLPRRQAKDPTSGVALIGGERIPMR
SGRDPDAAADLKPAYKLIATTTDHLEAKLAARMRRDHITQAAVVTNNPPCDYTPYGCEKILSRLL
PAGARLAVYVRDDDGQVRHWRTYTGNGKAIA

>SEQ ID NO:16 SCP013/No.75 (SCP1.201 clade)


MIRARDRLTAVTASSRHPLVDQALQHVTAAIERLQVADRDAALAASALVAYGRTLGISLPVPPPVS
APTRGAAPVPSWIRQTGQDLPTRPDDHGPTHGQAFDSTGRPLSAEPWRSGRNIASTSDLRPIPGLK
GFPWTLTDHVESRAAQQMRRPGAPREVSLVVNKEPCTDDPYGCDRILRHIIPAGSRLTIYVRDPDA
PAGVRTVGQYEG

>SEQ ID NO:17 Sdd3/SCP170/No.63 (SCP1.201 clade)


MSASAQLNTYLAAIGNSTTTVEAQPEAAPPPAAAESLDSTPRLPDGGIDFHALAKRLGLLEARPTE
QPPFDPRRFNPACWQGLKPYDQAGTAEGNLFIAPGKRWNTRPMQASKLEVGPQSDLHPQWRSRK
APWHIEGKIAAYMRQKGFTDGCVYLNARPCSGPDGCARNLPDLLPVGSTLHVHARYIDRTGETRF
YYREYRGTGKALT

>SEQ ID NO:18 No.2-1158 (SCP1.201 clade)


MPIRLSGGLNLYQYAPETNNWIDPLGCSGYRRRHEKMPPEGARLTTGNLFRHAQDEGIPPIFSSEN
DEFYHRLIEIYAGTGVLNAYIRGIASHVEPKAGLILNEDGSGWKIGSLYINYPDGPCLGCRTLMPFIL
NDGSILYVTFPTLGLDGYSYGHFHGGVSGFFREGTPCNLRHPE

>SEQ ID NO:19 No.101m (MafB19 clade)

MCGWSELASYRAREGMPARGSADDTFTAARLQIDGQVFFGRNAHGRPVDIRVNAQTKTHAEAD
VFQQAKDAGATGTRAVLHVVRDFCRSCGATGGVGSLMRGLGVEELLVHSPSGIFTINAVRRPSTP
RPLG

>SEQ ID NO:20 No.2-1479 (Novel AID/APOBEC-like clade)

MRTRPAFGRCDCAGSDRGWEVAGGYTSEASHVRRSPTPDGNSLLGGVAQLCHAFFHCSPTPELSS
HPDELCLRVACDPAGRKCETKGVVVVAALRDRAGDLRFLSRYSNCPLSSHAEEYVVRDEELVRA
VMEMAPEDDARSSTKTPGSAGTLTLYQRLQPCHGSSDNRGPLWSCSDALVAGLHRELLGPRGVSL
RVAVSYTYRAHWDVRGFESERERRWWGPKVEAAREGIRVFAAAAKDGVTLEALNAEDWAFLVS
LCDEDVARDYAAAFGEGGEGFGSKGCGVFTAPAVAHRRAMDEFVAEQIRRHSASGPTNGRATNA
AG

>SEQ ID NO:21 No.2-1478 (Novel AID/APOBEC-like clade)

MADLDDVQDPLLDTALDSTKDEADDSVLSEIAVNDTSVDDGVEDPDHEHKKIAKAGDKVLGNKK
EFCGAFYHVPRSKSGCLDKQSCAIAKRGHDATPLTAVALVKYEQQESSEWAIKSVRRYTNCSDKM
KHAEEFFLMDIDCQLEARHKGEEGFLDFWNKKKWQITMYLTMQPCHLSTDTGGTKEDQSCCEV
MIKAKEKLGDNVEIVIKPTHLCQVGWYKGKPREKPKNAEKGVRKLFKTTGIELECMKEGDWKYL
LQYAQPEVENKLPDYDTSRRKTEDEKIGEELHNQQLEQLAPELAQLSVNEKRRK

>SEQ ID NO:22 No.2-39 (TM1506 clade)

MVYSMDQKNKVTEKLKEGGYSFVLYKDGEWSTSEKRGIAPIMELLKENKELLRGAYVADKVIGK
AAALLLIEGGISYLHAEIISEHAIEVLQNSNIEYEYQELVPYIVNRSGDGMCPMEETVLDVTDTKIAF
ELLQEKIKKMQAAMQAQNMK

>SEQ ID NO:23 No.2-1429 (XOO2897-like clade)

MISDAAVAGIASKMAEKYYSACKKLSRSIPISTLGVIGKPVPEYSCDGIVPYNSTDLGRMAYKARV
EAGFGIFGGRNVAVARVPGWDDPKTGDLVVGFSQGNGFHAEDHVLEQLTKKDISPKKITELYSER
QPCAACGPNLENHLSPGTEITWSVQWGSDLEMNSAFTELLGKLIQQQ

>SEQ ID NO:24 No.2-1442 (XOO2897-like clade)

MAPDSLVWFDPLGLIVLQQVPYNDHPLFGAVSEFIQGKSRSDLRGRNVAAVLLDDGTVIVRASEG
GGNHAERVLMGLSEVDPAKVVAVYTERSPCTGRINCHDLLDSSLGADVPVYYTHEMIRGQEGKT
AQQIEADRNQFCRGG

>SEQ ID NO:25 SpCas9

MDKKYSIGLDIGTNSVGWAVITDEYKVPSKKFKVLGNTDRHSIKKNLIGALLFDSGETAEATRLKR
TARRRYTRRKNRICYLQEIFSNEMAKVDDSFFHRLEESFLVEEDKKHERHPIFGNIVDEVAYHEKY
PTIYHLRKKLVDSTDKADLRLIYLALAHMIKFRGHFLIEGDLNPDNSDVDKLFIQLVQTYNQLFEE
NPINASGVDAKAILSARLSKSRRLENLIAQLPGEKKNGLFGNLIALSLGLTPNFKSNFDLAEDAKLQ
LSKDTYDDDLDNLLAQIGDQYADLFLAAKNLSDAILLSDILRVNTEITKAPLSASMIKRYDEHHQD
LTLLKALVRQQLPEKYKEIFFDQSKNGYAGYIDGGASQEEFYKFIKPILEKMDGTEELLVKLNRED
LLRKQRTFDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFNASLGTYH
DLLKIIKDKDFLDNEENEDILEDIVLTLTLFEDREMIEERLKTYAHLFDDKVMKQLKRRRYTGWGR
LSRKLINGIRDKQSGKTILDFLKSDGFANRNFMQLIHDDSLTFKEDIQKAQVSGQGDSLHEHIANLA
GSPAIKKGILQTVKVVDELVKVMGRHKPENIVIEMARENQTTQKGQKNSRERMKRIEEGIKELGSQ
ILKEHPVENTQLQNEKLYLYYLQNGRDMYVDQELDINRLSDYDVDHIVPQSFLKDDSIDNKVLTR
SDKNRGKSDNVPSEEVVKKMKNYWRQLLNAKLITQRKFDNLTKAERGGLSELDKAGFIKRQLVE
TRQITKHVAQILDSRMNTKYDENDKLIREVKVITLKSKLVSDFRKDFQFYKVREINNYHHAHDAY
LNAVVGTALIKKYPKLESEFVYGDYKVYDVRKMIAKSEQEIGKATAKYFFYSNIMNFFKTEITLAN
GEIRKRPLIETNGETGEIVWDKGRDFATVRKVLSMPQVNIVKKTEVQTGGFSKESILPKRNSDKLIA
RKKDWDPKKYGGFDSPTVAYSVLVVAKVEKGKSKKLKSVKELLGITIMERSSFEKNPIDFLEAKG
YKEVKKDLIIKLPKYSLFELENGRKRMLASAGELQKGNELALPSKYVNFLYLASHYEKLKGSPED
NEQKQLFVEQHKHYLDEIIEQISEFSKRVILADANLDKVLSAYNKHRDKPIREQAENIIHLFTLTNLG
APAAFKYFDTTIDRKRYTSTKEVLDATLIHQSITGLYETRIDLSQLGGD


>SEQ ID NO:26 nCas9(D10A)


MDKKYSIGLAIGTNSVGWAVITDEYKVPSKKFKVLGNTDRHSIKKNLIGALLFDSGETAEATRLKR
TARRRYTRRKNRICYLQEIFSNEMAKVDDSFFHRLEESFLVEEDKKHERHPIFGNIVDEVAYHEKY
PTIYHLRKKLVDSTDKADLRLIYLALAHMIKFRGHFLIEGDLNPDNSDVDKLFIQLVQTYNQLFEE
NPINASGVDAKAILSARLSKSRRLENLIAQLPGEKKNGLFGNLIALSLGLTPNFKSNFDLAEDAKLQ
LSKDTYDDDLDNLLAQIGDQYADLFLAAKNLSDAILLSDILRVNTEITKAPLSASMIKRYDEHHQD


LTLLKALVRQQLPEKYKEIFFDQSKNGYAGYIDGGASQEEFYKFIKPILEKMDGTEELLVKLNRED
LLRKQRTFDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFNASLGTYH
DLLKIIKDKDFLDNEENEDILEDIVLTLTLFEDREMIEERLKTYAHLFDDKVMKQLKRRRYTGWGR
LSRKLINGIRDKQSGKTILDFLKSDGFANRNFMQLIHDDSLTFKEDIQKAQVSGQGDSLHEHIANLA
GSPAIKKGILQTVKVVDELVKVMGRHKPENIVIEMARENQTTQKGQKNSRERMKRIEEGIKELGSQ
ILKEHPVENTQLQNEKLYLYYLQNGRDMYVDQELDINRLSDYDVDHIVPQSFLKDDSIDNKVLTR
SDKNRGKSDNVPSEEVVKKMKNYWRQLLNAKLITQRKFDNLTKAERGGLSELDKAGFIKRQLVE
TRQITKHVAQILDSRMNTKYDENDKLIREVKVITLKSKLVSDFRKDFQFYKVREINNYHHAHDAY
LNAVVGTALIKKYPKLESEFVYGDYKVYDVRKMIAKSEQEIGKATAKYFFYSNIMNFFKTEITLAN
GEIRKRPLIETNGETGEIVWDKGRDFATVRKVLSMPQVNIVKKTEVQTGGFSKESILPKRNSDKLIA
RKKDWDPKKYGGFDSPTVAYSVLVVAKVEKGKSKKLKSVKELLGITIMERSSFEKNPIDFLEAKG
YKEVKKDLIIKLPKYSLFELENGRKRMLASAGELQKGNELALPSKYVNFLYLASHYEKLKGSPED
NEQKQLFVEQHKHYLDEIIEQISEFSKRVILADANLDKVLSAYNKHRDKPIREQAENIIHLFTLTNLG
APAAFKYFDTTIDRKRYTSTKEVLDATLIHQSITGLYETRIDLSQLGGD


>SEQ ID NO:27 UGI


MTNLSDIIEKETGKQLVIQESILMLPEEVEEVIGNKPESDILVHTAYDESTDENVMLLTSDAPEYKP
WALVIQDSNGENKIKML


>SEQ ID NO:28 Ddd1/SCP177

MSLPEYDGTTTHGVLVLDDGTQIGFTSGNGDPRYTNYRNNGHVEQKSALYMRENNISNATVYHN
NTNGTCGYCNTMTATFLPEGATLTVVPPENAVANNSRAIDYVKTYTGTSNDPKISPRYKGN

>SEQ ID No:29 Ddd2

MEDFHTYHVGKCRLLVHNANCNQEKPVLPKYDGKTTEGVMVTPDGKQISFKSGNSSTPSYPQYK
AQSASHVEGKAALYMRENGINEATVFHNNPNGTCGFCDRQVPALLPKGAKLTVVPPSNSVANNV
RAIPVPKTYIGNSTVPKIK

>SEQ ID No:30 Ddd3/SCP001

MLSSSYNAFALTYGVLILDDGKQYSFNSGKPDPIYRNYIPASHVEGKAAIYMRENKIQSGTVYHNN
TDGTCPYCDKMLPTLLEKDSTLKVVPPQNATSSKKGWITNEKIYIGNDKIPKTAR

>SEQ ID No:31 Ddd4

MGGDEEEENLTSNNEKKKNANKQKIELPPYDGKTTYGVLILDDGKQYSFNSGKPAPIYRNYIPASH
VEGKAAIYMRENKIQSGTVYHNNTDGTCPYCDKMLPTLLEKDSTLKVVPPQNATSSKKGWITNEK
IYIGNDKIPKTAR

>SEQ ID No:32 Ddd6/SCP009

MALLREAYPSMEGATLPPFDGKTTIGLMFYTDASGQYQVKKLFSGEKVLSNYDATGHVEGKAALI
MRNEKITEAVVMHNHPSGTCNYCDKQVETLLPKNATLRVIPPENAKAPTSYWNDQPTTYRGDGK
DPKAPSKK

>SEQ ID No:33 Ddd7/SCP103

MIGLMGGLNLYQYAPNSIAWTDWWGLAGSYTLGSYQISAPQLPAYNGQTVGTFYYVNGAGGLE
SRTFSSGGPTPYPNYANAGHVEGQSALFMRDNGISDGLVFHNNPEGTCGFCVNMTETLLPENSKL
TVVPPEGAIPVKRGATGETRTFTGNSKSPKSPVKGEC

>SEQ ID No:34 Ddd8/SCP234

MQDNTNIIDNRPKLPDYDGKTTHGILVTPNSEHIPFSSGNPNPNYKNYIPASHVEGKSAIYMRENGI
TSGTIYYNNTDGTCPYCDKMLSTLLEEGSVLEVIPPINAKAPKPSWVDKPKTYIGNNKVPKPNK

>SEQ ID No:35 Ddd9/SCP003

MGKSLSESQATLSVAQRLLATIGEEGKTAGVLELDGELIPLVSGKSSLPNYAASGHVEGQAALIMR
DRGATSGRLLIDNPSGICGYCKSQVATLLPENATLQVGTPLGTVTPSSRWSASRTFTGNDRDPKPW
PR

>SEQ ID No:36 Ddd10/SCP004

MASPAVGTNAAGSSGKNVRMPRDYASELPEYDGKTTHGVLVTNEGKVIQLRSGGKEEPYTGYKA
VSASHVEGKAAIWIRENGSSGGTVYHNNTTGTCGYCNSQVKALLPEGVELKIVPPTNAVAKNAQA
RAVPTINVGNGTQPGRKQK

>SEQ ID No:37 Ddd11/SCP271

MQGTSSDTIAEMLNSASQPGRTAGVLDIDGELTPLTSGRPSLPNYIASGHVEGQAAMIMRQQQVQS
ATVYHDNPNGTCGYCYSQLPTLLPEGAALDVVPPAGTVPPSNRWHNGGPSFIGNSSEPKPWPR

>SEQ ID No:38 Ddd12/SCP005

MGVAGGAATNADAQALLGSIRQAGKTAGVLNIDGDLMPLVSRKSSLPNYAASGHVEGQAALIMR
ERGVSSAELLIDNPNGICSYCTSQVPTLLPEGAQLMVRPPLGTVPTQWWFNGRTFLGNAANPKPSP
W

>SEQ ID No:39 Ddd13/SCP006

MHNINGCGPSAVQQLLSANGEPGKTAGVLDLNGELTSLVSGKGELPNYAASGHVEGQAAMMMR
AEKATSATLYIDNPNGICGYCRSQIATLLPEGATLEVVTPLGTVEPTARWSSSKVFTGNERYPKGW
VE

>SEQ ID No:40 Ddd14/SCP007

MGSAVVGGGIAATGAKALTTGKKLTESPGTLNAAQRLLASIGEEGKTAGVLEVDGALFPLVSGKS
VLPNYAASGHVEGQAALLMQGMGATNGRLLIDNPNGICGYCTSQVPTLLPENAVLEVGTPLGTVT
PSARWSASKPFIGNDREPKPWPR

>SEQ ID No:41 No.2-1157 (SCP1.201 clade)

MDPIRLSGGLNLYQYAPETNNWIDPLGCSGHRRRHEKMPPEGAPLTTGNLFRHAQDEGIPPIFSRK
DDEFYHRLIEIYAGTGVLNAYIRGIASHVEPKAGLILNEDGNGWKIGSLYINYPDGPCPGCRRLMPF
ILNDGSILYVTFPTLGLDGYSYGHFHGGVSGFFREGTPCNLRHPE

>SEQ ID No:42 No.73/SCP158(SCP1.201 clade)

MPGGGEINRPPTTQADDVPQPVGSEWERTEPDALPGTVRAAVERLQPRPAGSTRPTLGVFNGEEIT
SGGGDRSLAADLDHDPLRGPPVTFYDHVESKAAARMRRTGSTESDLAIDNTVCGTNDRDQSYPW
TCDKILPAILPNGSRLRVWVTRDGGVTWWHRVYIGTGERITK

>SEQ ID No:43 No.2-1145/SCP315 (SCP1.201 clade)

MSPKKPTASSDLKAIGERLGLKPCEGLLGTLPAMKPNSGQRTRGRWHKHPDRELTSGANDRDWE
HVKDFWHHNIWSGTAEDTEPRWLAHLELKFAMTMRRTRTKSEPVDQVHEEITINHPDGPCPQCQL
LLPYFLEEGSSLTIHWPAGSATYIGRPYFDRPLRDVKPYINEEQQ

>SEQ ID No:44 No.2-1157/SCP020 (SCP1.201 clade)

MDPIRLSGGLNLYQYAPETNNWIDPLGCSGHRRRHEKMPPEGAPLTTGNLFRHAQDEGIPPIFSRK
DDEFYHRLIEIYAGTGVLNAYIRGIASHVEPKAGLILNEDGNGWKIGSLYINYPDGPCPGCRRLMPF
ILNDGSILYVTFPTLGLDGYSYGHFHGGVSGFFREGTPCNLRHPE

>SEQ ID No:45 No.2-1156 (SCP1.201 clade)

MDPIRLSGGLNLYQYAPETNNWIDPLGCSGYRRRHEKMPPEGARLTTGNLFRHAQDEGIPPIFSSE
NDEFYYRLIKIYAGTGILNANIRGIASHVEPKAGLILNEDGSGWKIGSLYINYPNGPCLDCRRLMPFI
LNEGSILYVTFPTLGLDGYSYGHFHGREPGFFREGTPCNLRHPE

>SEQ ID No:46 Sdd2 (SCP1.201 clade)

MAPDSLVWFDPLGLIVLQQVPYNDHPLFGAVSEFIQGKSRSDLRGRNVAAVLLDDGTVIVRASEG
GGNHAERVLMGLSEVDPAKVVAVYTERSPCTGRINCHDLLDSSLGADVPVYYTHEMIRGQEGKT
AQQIEADRNQFCRGG

>SEQ ID No:47 SCP008 (SCP1.201 clade)

MTTGGGSDISRPPATRDTSATATEAPAQPELVPEGLTDAARDLPRRQAKDPTSGVALIGGERIPMR
SGRDPDAAADLKPAYKLIATTTDHLEAKLAARMRRDHITQAAVVTNNPPCDYTPYGCEKILSRLL
PAGARLAVYVRDDDGQVRHWRTYTGNGKAIA

>SEQ ID No:48 SCP011 (SCP1.201 clade)

MLGGVLPARSVMFPGHVEPDAHFGPNPERHHPALVEVPIVWAGRQEDRTSTWARRVQRGFPRYT
VGAKTAGMFYNAGSQSWELLSGVDHRGGLTRKASQHISRMLSSGFFDGKPLDTKSDHLRMLNYT
STHVETKAAIWARDSDQETIDVVTNRNYVCGESYDPDDVDEPPGCYQAVESVLREGQTMRVWTT
DPENRVITIHGKGM

>SEQ ID No:49 SCP013 (SCP1.201 clade)

MIRARDRLTAVTASSRHPLVDQALQHVTAAIERLQVADRDAALAASALVAYGRTLGISLPVPPPVS
APTRGAAPVPSWIRQTGQDLPTRPDDHGPTHGQAFDSTGRPLSAEPWRSGRNIASTSDLRPIPGLK
GFPWTLTDHVESRAAQQMRRPGAPREVSLVVNKEPCTDDPYGCDRILRHIIPAGSRLTIYVRDPDA
PAGVRTVGQYEG

>SEQ ID No:50 miniSdd7

MEGGGPGAVPEGGDGPPAVPAEEVERLRGELPPPVVPGTGQKTHGRWIGPDGRVRAIVSGRDEDA
ALVHAQLAAKGIPDEPTRNSDVEQKLAAHMVANGIRHVTLVINHRPCRGFDDSCDTLVPIILPEGC
TLTVHGQTDKGMRVRVRYTGGARPWWS

>SEQ ID No:51 miniSdd4

MDPKKQNPACYQGLAPRQKGKPVRGNLFFPDGRRWNDVALESSRGEPAFDLNIIKPEYRSLSPAR
GHLEGNVAAWMRSTFHQEMVLYINESPCRKHGKGCLYTLEHFLPRGYVLHVWSRNDRGEWRGN
TFRGSGEAFTEGA

>SEQ ID No:52 miniSdd9

MCPIWVKNALQELVGRKETSGKVFDVDGKPIGPDIQSGYKDRELRRGVYETLRKSPFFQKHFPSTA
TWYVSLHVEAQYAVWMLRNRIKHATVVINNTYVCSDMNRLHDNCMTAVPHILPEGYTMTVWK
ADRTEVTLRGKAPKE

>SEQ ID No:53 miniSdd6

MPASKPSRFKPMRTDPAKADEIRPHVGKDRAVATLWDADGNRVLGLHSADDDGPAATAAWKPP
WRDYVRLRRHVEAHAAARMHQDGHKTMVMYINLPPCKYFDGCKLNLEDILPKGSTLWMHRVF
QNGGTKIYQFNGTGRAYV

>SEQ ID No:54 miniSdd10

MPGANGSERVDELAERLPPTVVPNTSAKTHGWWFTGQGAAQELISGEGPDARAAYEALREEGYP
RPGMPFVAMHVEIKLAAHMRRNDIEHATVVINNIPCPLVWGCENLIGVVLPEGSSLTVHGSNGYE
RTFTGGRKPPWPR

>SEQ ID No:55 miniSdd3

MPRRFNPACWQGLKPYDQAGTAEGNLFIAPGKRWNTRPMQASKLEVGPQSDLHPQWRSRKAPW
HIEGKIAAYMRQKGFTDGCVYLNARPCSGPDGCARNLPDLLPVGSTLHVHARYIDRTGETRFYYR
EYRGTGKALT

>SEQ ID NO:56 No.2-1241 (MafB19 clade)

MGLEGTPCDGFGALAARRKSLGLPAAGSEGDTSTLSLLRINGQSFEGINSSDQNPKTPITLDRVNAQ
TKTHAEAEAVQKAVNAGMAGKASHAEMWVDRDPCRACGIPGAGGLRSLARNLGCPITVHSPSGT
QVYTPTK

>SEQ ID NO:57 No.2-1231 (MafB19 clade)

MLGPPLDLNPANRAPEFGRCDGTSWIDSYRTINNATDLFGRPVWPNHRGTVAVARIDGDIYFGVN
SKAPGYSDADWNLAAGLRDQMALEHPELIRGESRGSRPLDAVFHAEANLLIRASRYVGSLVKRSI
EVQVDRPVCWSCEQALPKVGLELGDPYVTIREVRSGRASVMWQGEWLVWRKK

>SEQ ID NO:58 No.99 (MafB19 clade)

MGWVDPLGLVSGGAWDAISFFRDQNSLLSVVDEDLLAASGAKNAQNTVALLRVGDREFIGVNSR
IQNPKNPFTAGPINNITKFHAEGNAAQQAIDAGMVGKHRIAEMWVDRDLCHACGPSNGVGSLTRA
LGLDAIIVHTPAGTRKFNAPCAG

>SEQ ID NO:59 No.2-1430 (XOO2897-like clade)

MVKGLDGFVKTCTRTRGKLTARASTAVGGCPVGLVAYNSEEMSHWAYRYRTESEYFEGDHNVA
VAKVPGWNDPRTGDFIIANSKFSGHSETEILGKLEAKGFTPGQITALYTERQPCPACASVLTGSLKE
GTPVTWSVPYHPDYAKESRSLLDSYVRQANGQQRARPTTTQRLTEGNEAHD

>SEQ ID NO:60 No.2-1440 (XOO2897-like clade)

MRLGQSVDPRLLEMAKEARVTQAGISREAFASYNVATARVRVGTEIRYLDAGNSPGRLMHSEDW
LITQVEELRRVHGRESVALEQLFSERIPCGECLPKLERLFNAEVFYAVAKRGTRATDLMKAYGLR

>SEQ ID NO:61 No.2-1432 (XOO2897-like clade)

MPDPLGLAPAANDRAYVPNPLTWADPYGLACTGTTEPGSTDLSQAVIQERLRLGKKGNNFAAAR
YIDDNGVEQIAVAASSKGQFMHAERKLVRQYGDKITEVYSEFEPCIGTNQCRKTLGDMGIKYTYS
WAWTLSKDGVAANAARKAYVDQIFDDAEAGNWAAPWAD

>SEQ ID NO:62 No.2-1437 (XOO2897-like clade)

MNIDGDSYLVPGALAAMFLHKPGRGGKGKVGYGTTDLGQSVRLQRLIDKNRGMTNYAAARLDD
GDVIVGKSKKHVHAEEHLFQQAGKRKIVELYSEREPCSNKCEDLVKDIPFVSWSFKWNHPDRIKQ
DAIRDKANADLKDAVRSLFNSP

>SEQ ID NO:63 No.181 (AID/APOBEC clade)

MQGYIVDESGRVLDANGLPIASLPPADDLSKWANYTVESGLHDDLAALENRLDLLYRQQFGLPM
APPAWHLETQLAYRVATREVALRDSTLRLVMNNPGGVCDAVPLTGTGPDRQRQAVAGCIQAVK
MLLPAGTTMIIYYPDPDDPAELLEITVRGVGRWLD

>SEQ ID NO:64 rAPOBEC

MSSETGPVAVDPTLRRRIEPHEFEVFFDPRELRKETCLLYEINWGGRHSIWRHTSQNTNKHVEVNFI
EKFTTERYFCPNTRCSITWFLSWSPCGECSRAITEFLSRYPHVTLFIYIARLYHHADPRNRQGLRDLI
SSGVTIQIMTEQESGYCWRNFVNYSPSNEAHWPRYPHLWVRLYVLELYCIILGLPPCLNILRRKQP
QLTFFTIALQSCHYQRLPPHILWATGLK

>SEQ ID NO:65 DddA

GSYALGPYQISAPQLPAYNGQTVGTFYYVNDAGGLESKVFSSGGPTPYPNYANAGHVEGQSALF
MRDNGISEGLVFHNNPEGTCGFCVNMTETLLPENAKMTVVPPEGAIPVKRGATGETKVFTGNSNS
PKSPTKGGC

>SEQ ID NO:66 SCP090 (SCP1.201 clade)

MDGPHGTPVLDRIAKLREELPPPAVPGKGQKTDGRWFDGNGAVRDSVSGKDVDSEEAWRLLRES
GIPLPRPPVVAHAEMKVAAAMRRLNVRHAVLVITNVPCDERWSCENLLPAVLPVGCSLSVHGPG
YQRTFHGRTPKW

>SEQ ID NO:67 SCP015 (SCP1.201 clade)

MRPTTPPGPHARWRPDPPSAPHVAAIRRVGWPKKPQSDDDVRARGQLYHRDGTPWNASMLIASR
RGPASQRTDLKEPWASDPGYTTGWHIEGNTAALMVKHQQRDAVLYINQAVCGAEEPQDPKRCHS
NIVAMLSVGYALYVHSVQESGWLRRRVYKGTGEAIR

>SEQ ID NO:68 SCP278(SCP1.201 clade)

MQRHGGRVRLLSGENDDPHSWQQQAARFLRETFPDKGPGLAVLSRHVEIQLAVRLRHRPTNEVV
HEVLVIDRVVCGRDPRTQGREYTCDTVLPFVLDEGATLTVVEHDGARVTYRGRGRR

>SEQ ID NO:69 SCP287 (SCP1.201 clade)

MKILYIKSAEGYPSLMLKNNPRIPTNARSYTHVEGRAASIMRQSGIKSAKLTINNTNGVCDPCRGN
MEKSLLPDGGKLNVRYPDGQGGYTQRILI

>SEQ ID NO:70 SCP341 (SCP1.201 clade)

MREFGLPIEPPAWHLETQLAYRVSKREVALRENTLRLVMNNPGGVCDAVPAKDRGPDGQRQVVA
GCVQAIQMLLPAGTTMIIYYPDPANPAKLLQVTVRGVGRWLD

>SEQ ID NO:71 SCP353 (SCP1.201 clade)

MLQGWRLAVGDGSSDRELASGTKLASGQTDPSYTAAVQRARELGLARGGFVPDIARHIEIKEAST
MTAGETRTIVIGKDPCGIDPVTNVSCHPFLRYFLPPGATLIVYGPRGEPYRYEGKRTS

>SEQ ID NO:72 SCP357 (SCP1.201 clade)

MVGATTDSVPTQPGGRARSAPPRPPRPGKTHGRWCDSDGNAVVLESGKGGEYYEATRARGVAL
GLAKGIPNAEPSIARHVETQFVSRMIDQGIEYAEIEINRPVCGTTPKDQQ

>SEQ ID NO:73 SCP372 (SCP1.201 clade)

MQGYIVDESGRVLDANGLPIASLPPADDLSKWANYTVESGLHDDLAALENRLDLLYRQQFGLPM
APPAWHLETQLAYRVATREVALRDSTLRLVMNNPGGVCDAVPLTGTGPDRQRQAVAGCIQAVK
MLLPAGTTMIIYYPDPDDPAELLEITVRGVGRWLD

>SEQ ID NO:74 No.2-1128 (Toxin deam clade)

MQGYIVDESGRVLDANGLPIASLPPADDLSKWANYTVESGLHDDLAALENRLDLLYRQQFGLPM
APPAWHLETQLAYRVATREVALRDSTLRLVMNNPGGVCDAVPLTGTGPDRQRQAVAGCIQAVK
MLLPAGTTMIIYYPDPDDPAELLEITVRGVGRWLD

>SEQ ID NO:75 No.2-1114 (Toxin deam clade)

MGAPRTMGNMGVAQISIPGVQSKMAASSQIPDPTAAQRALGFVGEVNETFPSASVWTGGDTPYLL
NRKVDSEAKILNNIAAQLGDNTSASGTINLFTERPPCESCSNTIIKFQEKYPNIKINVMDSNGVIRPS
KR

>SEQ ID NO:76 No. 2-1223 (MafB19 clade)

MGQGFDGVQPANEFIKAWGEAMVAEAAGLGIVAGLGRFGLWGAKGATPVVTAEGRIGNSVFTD
VNQTARPAAQANPNQPTLIADRVDAKIAAKGTPHPNGNMADAHAEIGVIQKAFNEGKTVGSDMT
MNVVGKDVCGYCRGDIAAAASKSGLKSLTIQAKDDITGLPKTYYWEVGMKSIREKKI

>SEQ ID NO:77 No.2-1224 (MafB19 clade)

MYLPRGTSVTSKETVAKDPVSLAGQADNEAGILVDRNVIVGGTKGVSPVVTAEGKIGGKTFTDFN
QTARPASEANASQPTLISDRVTAKADASGKVLPNGNMADAHAEIGVIQQAYTAGKTMGASMELT
VSGKAVCGYCRGDIAAMAEKSGLTSLEVKEVATGKTLYWQPGMRALRERN

>SEQ ID NO:78 No.2-1228 (MafB19 clade)

MKKPTGSIVSPETTIVQESSKILDKKTHTSIPKVEAELIDKETGKIFKDTNQGNRPDYFLGDKSRPTLI
NDRIEAKVEKNPSKYLPNGNMASAHAEVGTIQQAFEDGITVGRDMNMKVTKEAVCGYCRGDIAA
MADKAGLKSLTVYEESTGKTLYWNPGMKSLKEKK

>SEQ ID NO:79 No.2-1229 (MafB19 clade)

MAGVLAPEVYLAKTPRVGDNSARGVGDGRSTTPKVTAEAEVDGVKFNDTNQNARPSEAANPNIP
TLISDDIQVKIDKNPDKPFPNGNMATAHAEVGAIQQAYDAGKTQGKNMTMRVTGEDVCDYCRSD
LRKAADKSGLNSLSVYEETTGRTLTWTRREDGTIGKVKIIEPEG

>SEQ ID NO:80 No.2-1230 (MafB19 clade)

MGVDRKTAQGYAETKQGMDTIVASVTPILGAAAAKQLSKVVDANIKVVAEGNVNGAKFSDTNQ
GARPSNLADVNKPTLIDGRIQAKIDKQNKPLPNGNMATAHAEVGVIQQAFEKGMSQGREMTMSV
SKEPVCGYCRSDIAAMADKAGLKSLTIYEETTGSVLYWQPGMKSLKIRD

>SEQ ID NO:81 No.2-1234 (MafB19 clade)

MDRQTAESYTETKQGLEIIAASVTPILGSVAAKQLSKIVDANLKVVARGNVDGARFSDTNQGVRP
SQLADFNKPTLINDVVQAKIDKRPDKNYPNGNMGTAHAEVGVIQQAFDKGMTQGREMAMSVGG
KEVCNYCLSDVRIMAEKAGLKSLTIYEEATGNVLFWQQGMKKIENRGPAK

>SEQ ID NO:82 No.2-1235 (MafB19 clade)

MTKSALGEIVIVVSDLVIPTNYVEILPVGKLSKVAKILKIGEDGTKSAGRLAEELAELQKVDIKFGK
TLPGAKAPITVTAESNIGGKHMFDTNQTARPEVNRTNTPTLAAGNAKIDPSNPNLTMKNAHAEIAL
IQRAYDAGLTKGETMQVLVRGKEVCDHCGQVMKTMYERSGLSKLIIHDTTSGTTTTYYKVIDAK
TKIATTKIEV

>SEQ ID NO:83 No.3-2107_cl21x07 (MafB19 clade)

MDDSYYMKQALLEAQKAGERGEVPVGAVVVCKDRIIARAHNLTETLTDVTAHAEMQAITAAAST
LGGKYLNECALYVTVEPCVMCAGAIAWAQTGKLVFGAEDEKRGYQRYAPQALHPKTMVVKGV
LADECAALMKNFFAAKRK

>SEQ ID NO:84 No.4-2130A_cl21x30 (MafB19 clade)

MTKSALGEIVIVVSDLVIPTNYVEILPVGKLSKVAKILKIGEDGTKSAGRLAEELAELQKVDIKFGK
TLPGAKAPITVTAESNIGGKHMFDTNQTARPEVNRTNTPTLAAGNAKIDPSNPNLTMKNAHAEIAL
IQRAYDAGLTKGETMQVLVRGKEVCDHCGQVMKTMYERSGLSKLIIHDTTSGTTTTYYKVIDAK
TKIATTKIEV

>SEQ ID NO:85 HsJAK2 (5'-3') Figure 15, 17, 18
AGGCAGGCCATTCCCA

>SEQ ID NO:86 HsJAK2 (3'-5') Figure 15, 17, 18
TCCGTCCGGTAAGGGT

>SEQ ID NO:87 HsSIRT6 (5'-3') Figure 15, 17, 18
TCGCCGTACGCGGACAAGGG

>SEQ ID NO:88 HsSIRT6 (3'-5') Figure 15, 17, 18
AGCGGCATGCGCCTGTTCCC

>SEQ ID NO:89 HsHEK2 (5'-3') Figure 16A
GAACACAAAGCATAGACTGCGGG

>SEQ ID NO:90 HsHEK2 (3'-5') Figure 16A
CTTGTGTTTCGTATCTGACGCCC

>SEQ ID NO:91 HsWFS1 (5'-3') Figure 16A
CAGCAGTATGGTGCGCTGTGCGG

>SEQ ID NO:92 HsWFS1 (3'-5') Figure 16A
GTCGTCATACCACGCGACACGCC

>SEQ ID NO:93 OsAAT Figure 21
ACAAGGATCCCAGCCCCGTGAAGG

>SEQ ID NO:94 OsACC1 or OsACC-T1 Figure 21
TCTCAGCATAGCACTCAATGCGGTCTGGG

>SEQ ID NO:95 OsCDC48-T1 Figure 21
TAGCACCCATGACAATGACATGG

>SEQ ID NO:96 OsCDC48-T2 Figure 21
GACCAGCCAGCGTCTGGCGCCGG

>SEQ ID NO:97 OsDEP1 Figure 21
CTAGCACATGAGAGAACAATATTGGG

>SEQ ID NO:98 OsODEV Figure 21
GCACACACACTAGTACCTCTGG

>SEQ ID NO:99 HsEMX1 Figure 22
ACAAAGTACAAACGGCAGAAGCTGGAGG

>SEQ ID NO: 100 HsHEK2 Figure 22
ACTGGAACACAAAGCATAGACTGCGGG

>SEQ ID NO: 101 HsWFS1 Figure 22
CCTGGCAGCAGTATGGTGCGCTGTGCGG

>SEQID NO:102 MmHPD-T1 Figure 30C
GCAACCAACCCGACCAAGAAATGCAGT

>SEQID NO:103 MmHPD-T2 Figure 30C
AGTCATTCAACGTCACAACCACCAGGT

>SEQID NO:104 GmALS1-T1 Figure 30D
TCTCCATCGACGCACCGCCGGGG

>SEQID NO:105 GmALS1-T2 Figure 30D
CAGGTCCCCCGCCGGATGATCGG

>SEQID NO:106 GmALS1-T3 Figure 30D
GATCCATTACTGGGAATCATCGG

>SEQ ID NO:107 GmPPO2 Figure 30D
AAGCGCTATATTGTGAAAAATGG

>SEQ ID NO:108 GmEPSPS Figure 30D
CAATGCGTCCTTTGACAGCAGCTGTGG

>SEQ ID NO:109 GmPPO2 wild-type Figure 30F
CATAAGCGCTATATTGTGAAAAATGGGGCA

>SEQ ID NO:110 GmPPO2 edited Figure 30F
CATAAGTGCTATATTGTGAAAAATGGGGCA

**Claims**

1. A cytosine deaminase, wherein the cytosine deaminase is capable of deaminating the cytosine base of deoxycytidine in DNA.

2. The cytosine deaminase according to claim 1, wherein the cytosine deaminase is derived from bacteria.

3. The cytosine deaminase according to claim 1 or 2, wherein the TM-score of AlphaFold2 three-dimensional structure between the cytosine deaminase and a reference cytosine deaminase is not less than 0.6, not less than 0.7, not less than 0.75, not less than 0.8, or not less than 0.85, and the cytosine deaminase comprises an amino acid sequence having 20-70%, 20-60%, 20-50%, 20-45%, 20-40%, 20-35% sequence identity or having at least 20%, at least 30%, at least 40%, at least 50%, at least 60%, at least 70%, at least 75%, at least 80%, at least 85%, at least 90%, at least 91%, at least 92%, 93%, at least 94%, at least 95%, at least 96%, at least 97%, at least 98%, or at least 99% sequence identity with the amino acid sequence of the reference cytosine deaminase; and the cytosine deaminase is capable of deaminating the cytosine base of deoxycytidine of DNA.

4. The cytosine deaminase according to claim 3, wherein the reference cytosine deaminase is:

   (a) rAPOBEC1 having a sequence shown in SEQ ID No: 64; or
   (b) DddA having a sequence shown in SEQ ID No: 65; or
   (c) Sdd7 having a sequence shown in SEQ ID No: 4.

5. The cytosine deaminase according to claim 4, wherein the TM-score of AlphaFold2 three-dimensional structure between the cytosine deaminase and rAPOBEC1 shown in SEQ ID No: 64 is not less than 0.6, not less than 0.7, not less than 0.75, not less than 0.8, not less than 0.85, and the cytosine deaminase comprises an amino acid sequence having 20-70%, 20-60%, 20-50%, 20-45%, 20-40%, 20-35% sequence identity or having at least 20%, at least 30%, at least 40%, at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99% sequence identity with SEQ ID No: 64; and the cytosine deaminase is capable of deaminating the cytosine base of deoxycytidine of DNA.

6.  The cytosine deaminase according to claim 4, wherein the TM-score of AlphaFold2 three-dimensional structure between the cytosine deaminase and DddA of SEQ ID No: 65 is not less than 0.6, not less than 0.7, not less than 0.75, not less than 0.8, not less than 0.85, and the cytosine deaminase comprises an amino acid sequence having 20-70%, 20-60%, 20-50%, 20-45%, 20-40%, 20-35% sequence identity or at least 20%, at least 30%, at least 40%, at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99% sequence identity with SEQ ID No: 65; and the cytosine deaminase is capable of deaminating the cytosine base of deoxycytidine in DNA.

7.  The cytosine deaminase according to claim 4, wherein the TM-score of AlphaFold2 three-dimensional structure between the cytosine deaminase and Sdd7 shown in SEQ ID No: 4 is not less than 0.6, not less than 0.7, not less than 0.75, not less than 0.8, not less than 0.85, and the cytosine deaminase comprises an amino acid sequence having 20-70%, 20-60%, 20-50%, 20-45%, 20-40%, 20-35% sequence identity or having at least 20%, at least 30%, at least 40%, at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99% sequence identity with SEQ ID No: 4; and the cytosine deaminase is capable of deaminating the cytosine base of deoxycytidine of DNA.

8.  The cytosine deaminase according to any one of claims 1 to 7, wherein the cytosine deaminase is from the AID/APOBEC clade, the SCP1.201 clade, the MafB19 clade, the Novel AID/APOBEC-like clade, the TM1506 clade, the XOO2897 clade or the Toxin deam clade.

9.  The cytosine deaminase according to claim 8, wherein the cytosine deaminase is from the AID/APOBEC clade and comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity with SEQ ID NO: 1 or 63.

10.  The cytosine deaminase according to claim 8, wherein the cytosine deaminase is from the SCP1.201 clade and comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity with any one of SEQ ID No: 28-40.

11.  The cytosine deaminase according to claim 10, wherein the cytosine deaminase is capable of deaminating the cytosine base of double-stranded DNA.

12.  The cytosine deaminase according to claim 10 or 11, wherein the cytosine deaminase comprises an amino acid sequence of any one of SEQ ID No: 28-40.

13.  The cytosine deaminase according to claim 12, wherein the cytosine deaminase comprises an amino acid sequence of any one of SEQ ID No: 28, 33, 34, 35.

14.  The cytosine deaminase according to claim 8, wherein the cytosine deaminase is from the SCP1.201 clade and comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity with any one of SEQ ID Nos: 2-18, 41-49.

15.  The cytosine deaminase according to claim 14, wherein the cytosine deaminase is capable of deaminating the cytosine base of single-stranded DNA.

16.  The cytosine deaminase according to claim 14 or 15, wherein the cytosine deaminase comprises an amino acid sequence of any one of SEQ ID No: 2-18, 41-49.

17.  The cytosine deaminase according to claim 16, wherein the cytosine deaminase comprises an amino acid sequence of any one of SEQ ID No: 2-7, 12, 17.

18. The cytosine deaminase according to any one of claims 1-17, wherein the cytosine deaminase is a truncated cytosine deaminase, and the truncated cytosine deaminase is capable of deaminating the cytosine base of deoxycytidine of DNA.

19. The cytosine deaminase according to claim 18, wherein the length of the truncated cytosine deaminase ranges from 130 to 160 amino acids.

20. The cytosine deaminase according to claim 19, wherein the truncated cytosine deaminase can be packaged entirely in one AAV particle.

21. The cytosine deaminase according to any one of claims 18-20, wherein the truncated cytosine deaminase comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity with any one of SEQ ID Nos: 50-55.

22. The cytosine deaminase according to claim 21, wherein the truncated cytosine deaminase is capable of deaminating the cytosine base of single-stranded DNA.

23. The cytosine deaminase according to claim 21 or 22, wherein the truncated cytosine deaminase consists of an amino acid sequence of any one of SEQ ID No: 50-55.

24. The cytosine deaminase according to claim 8, wherein the cytosine deaminase is from the MafB19 clade and comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity with any one of SEQ ID No: 19, 56, 57, 58.

25. The cytosine deaminase according to claim 8, wherein the cytosine deaminase is from the Novel AID/APOBEC-like clade and comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity with any one of SEQ ID No: 20, 21.

26. The cytosine deaminase according to claim 8, wherein the cytosine deaminase is from the TM1506 clade and comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity with SEQ ID No: 22.

27. A cytosine deaminase according to claim 8, wherein the cytosine deaminase is from the XOO2897 clade and comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity with any one of SEQ ID No: 23, 24, 59-62.

28. The cytosine deaminase according to claim 8, wherein the cytosine deaminase is from the Toxin deam clade and comprises an amino acid sequence having at least 50%, at least 60%, at least 70%, at least 75%, at least 80%, at least 85%, at least 90%, at least 91%, at least 92%, 93%, at least 94%, at least 95%, at least 96%, at least 97%, at least 98%, at least 99%, or even 100% sequence identity with SEQ ID No: 74 or 75.

29. Use of the cytosine deaminase according to any one of claims 1 to 28 for gene editing, such as base editing, in an organism or a cell of an organism.

30. A fusion protein comprising:

   (a) a nucleic acid targeting domain; and
   (b) a cytosine deamination domain, wherein the cytosine deamination domain comprises at least one cytosine deaminase polypeptide of any one of claims 1-28.

31. The fusion protein of claim 30, wherein the nucleic acid targeting domain is a TALE, ZFP or CRISPR effector protein domain.

32. The fusion protein of claim 31, wherein the CRISPR effector protein is at least one of Cas9, Cpf1, Cas3, Cas8a, Cas5, Cas8b, Cas8c, Cas10d, Cse1, Cse2, Csy1, Csy2, Csy3, GSU0054, Cas10, Csm2, Cmr5, Cas10, Csx11, Csx10, Csf1, Csn2, Cas4, C2c1 (Cas12b), C2c3, C2c2, Cas12c, Cas12d, Cas12e, Cas12f, Cas12g, Cas12h, Cas12i, Cas12j, Cas121, Cas12m, or other available CRISPR effector proteins.

33. The fusion protein of claim 32, wherein the Cas9 is a nuclease-inactivated Cas9, a Cas9 nickase, or a nuclease-active Cas9.

34. The fusion protein of claim 33, wherein the Cas9 is a nuclease-inactivated Cas9, and the nuclease-inactivated Cas9 comprises the amino acid sequence shown in SEQ ID NO: 26.

35. The fusion protein of any one of claims 30-34, wherein the nucleic acid targeting domain and the cytosine deamination domain are fused via a linker.

36. The fusion protein of any one of claims 30-35, wherein the fusion protein is co-expressed with a uracil DNA glycosylase inhibitor (UGI).

37. The fusion protein of any one of claims 30-35, wherein the fusion protein further comprises a uracil DNA glycosylase inhibitor (UGI), for example, the UGI is connected to other parts of the fusion protein through a linker, or the UGI is connected to other parts of the fusion protein through a self-cleaving peptide.

38. The fusion protein of any one of claims 30-37, wherein the fusion protein further comprises one or more nuclear localization sequences (NLS).

39. A base editing system for modifying a target nucleic acid region, comprising: i) a cytosine deaminase according to any one of claims 1 to 28 or a fusion protein according to any one of claims 30 to 38, and/or an expression construct containing a nucleotide sequence encoding the cytosine deaminase or the fusion protein.

40. The base editing system of claim 39, wherein the base editing system further comprises:

   ii) at least one guide RNA and/or at least one expression construct containing a nucleotide sequence encoding the at least one guide RNA; and/or
   iii) at least one uracil DNA glycosylation inhibitor (UGI) and/or at least one expression construct containing a nucleotide sequence encoding the uracil DNA glycosylation inhibitor (UGI); and/or
   iv) a nuclear localization sequence (NLS).

41. The base editing system of claim 40, wherein the at least one guide RNA can bind to the nucleic acid targeting domain of the fusion protein, and the guide RNA is directed to at least one target sequence within the target nucleic acid region.

42. The base editing system of claim 41, wherein the guide RNA is 15-100 nucleotides in length and comprises a sequence of at least 10, at least 15, or at least 20 consecutive nucleotides complementary to the target sequence.

43. The base editing system of claim 42, wherein the guide RNA comprises a sequence of 15 to 40 consecutive nucleotides complementary to the target sequence.

44. The base editing system of any one of claims 40-43, wherein the guide RNA is 15-50 nucleotides in length.

45. The base editing system of any one of claims 40-44, wherein the target nucleic acid is DNA.

46. The base editing system of any one of claims 40-45, wherein the target nucleic acid is in the genome of an organism.

47. The base editing system of claim 46, wherein the organism is a prokaryotic organism such as a bacterium; a eukaryotic organism such as a plant, a fungus, or a vertebrate.

48. The base editing system of claim 47, wherein the vertebrate is a mammal such as a human, a mouse, a rat, a monkey, a dog, a pig, a sheep, a cow, or a cat.

49. The base editing system of claim 47, wherein the plant is a crop plant, such as wheat, rice, corn, soybean, sunflower, sorghum, rapeseed, alfalfa, cotton, barley, millet, sugarcane, tomato, tobacco, cassava or potato.

50. A base editing method, comprising contacting the base editing system of any one of claims 39-49 with a target sequence of a nucleic acid molecule.

51. The base editing method of claim 50, wherein the nucleic acid molecule is a DNA molecule, for example, the nucleic acid molecule is a double-stranded DNA molecule or a single-stranded DNA molecule.

52. The base editing method of any one of claims 50-51, wherein the target sequence of a nucleic acid molecule comprises a sequence associated with a plant trait or expression.

53. The base editing method of any one of claims 50-51, wherein the target sequence of a nucleic acid molecule comprises a sequence or point mutation associated with a disease or condition.

54. The base editing method of any one of claims 50-53, wherein the base editing system achieves deamination by contacting with the target sequence of a nucleic acid molecule, and the deamination results in one or more nucleotides of the target sequence being substituted.

55. The base editing method of any one of claims 50-54, wherein the target sequence comprises a DNA sequence 5'-MCN-3', wherein M is A, T, C or G; N is A, T, C or G; wherein C in the middle of the 5'-MCN-3' sequence is deaminated.

56. The base editing method of any one of claims 50-55, wherein the deamination results in the introduction or removal of a splicing site.

57. The base editing method of any one of claims 50-55, wherein the deamination results in the introduction of a mutation in a gene promoter, the mutation resulting in an increase or decrease in transcription of a gene operably linked to the gene promoter.

58. The base editing method of any one of claims 50-55, wherein the deamination results in the introduction of a mutation in a gene repressor, the mutation resulting in an increase or decrease in transcription of a gene operably linked to the gene repressor.

59. The base editing method of any one of claims 50-58, wherein the contacting is performed in vivo or wherein the contacting is performed in vitro.

60. A method for producing at least one genetically modified cell, comprising introducing the base editing system of any one of claims 39-49 into at least one cell, thereby causing one or more nucleotides in the target nucleic acid region in the at least one cell being substituted, for example, the one or more nucleotide substitutions are C to T substitutions.

61. The method of claim 60, further comprising the step of screening for a cell having the desired one or more nucleotide substitutions from the at least one cell.

62. The method of claim 60 or 61, wherein the base editing system is introduced into the cell by a method selected from the following: calcium phosphate transfection, protoplast fusion, electroporation, liposome transfection, microinjection, viral infection (such as baculovirus, vaccinia virus, adenovirus, adeno-associated virus, lentivirus or other virus), gene gun technique, PEG-mediated protoplast transformation, Agrobacterium-mediated transformation.

63. The method of any one of claims 60-62, wherein the cell is from a mammal such as human, mouse, rat, monkey, dog, pig, sheep, cow, cat; poultry such as chicken, duck, goose; a plant, preferably a crop plant, such as wheat, rice, corn, soybean, sunflower, sorghum, rapeseed, alfalfa, cotton, barley, millet, sugarcane, tomato, tobacco, cassava or potato.

64. A protein clustering method, comprising:

    (1) obtaining the sequences of a plurality of candidate proteins from a database;
    (2) predicting the three-dimensional structure of each of the plurality of candidate proteins using a protein prediction program;
    (3) performing multiple structural alignment on the three-dimensional structures of the plurality of candidate proteins using a scoring function, thereby obtaining a structural similarity matrix;

(4) clustering the plurality of candidate proteins based on the structural similarity matrix using a phylogenetic tree construction method.

65. The protein clustering method of claim 64, wherein in step (1), the sequences of the plurality of candidate proteins are obtained through the annotation information in the database; or wherein in step (1), the sequences of the plurality of candidate proteins are obtained by searching the database based on sequence identity/similarity using the sequence of a reference protein.

66. The protein clustering method of claim 64 or 65, wherein the candidate protein is a deaminase, preferably a cytosine deaminase.

67. The protein clustering method of any one of claims 64-66, wherein the database is the InterPro database.

68. The protein clustering method of any one of claims 64-67, wherein the protein structure prediction program in step (2) is selected from AlphaFold2, RoseTT or other programs that can predict protein structure.

69. The protein clustering method of any one of claims 64-68, wherein the scoring function used in step (3) includes TM-score, RMSD, LDDT, GDT score, QSC, FAPE or other scoring functions that can score protein structure similarity.

70. The protein clustering method of any one of claims 64-69, wherein the phylogenetic tree construction method in step (4) is UPGMA.

71. The protein clustering method of any one of claims 64-70, wherein a clustering dendrogram of the plurality of candidate proteins is obtained in step (4).

72. A protein function prediction method based on three-dimensional structure, comprising clustering a plurality of candidate proteins according to the protein clustering method of any one of claims 64-71, and then predicting the function of the candidate proteins based on the clustering results.

73. The protein function prediction method of claim 72, wherein the plurality of candidate proteins include at least one reference protein with known function.

74. The protein function prediction method of claim 73, wherein the function of other candidate proteins in the clade or subclade where the reference protein is located is predicted by the position of the reference protein with known function in the cluster (dendrogram).

75. The protein function prediction method of claim 73 or 74, wherein the reference protein is a deaminase, such as a cytosine deaminase.

76. The protein function prediction method of claim 75, wherein the reference protein is a reference cytosine deaminase, and the reference cytosine deaminase is rAPOBEC1 having a sequence shown in SEQ ID No: 64 or DddA having a sequence shown in SEQ No: 65.

77. A method for identifying the minimal functional domain of a protein based on the three-dimensional structure, comprising:

a) comparing the structures of a plurality of candidate proteins clustered together by the protein clustering method of any one of claims 64-71, such as clustered in a same clade or subclade, to determine a conserved core structure;

b) identifying the conserved core structure as the minimal functional domain.

78. The method of claim 77, wherein the plurality of candidate proteins include at least one reference protein with known function.

79. The method of claim 78, wherein the reference protein is a deaminase, preferably the reference protein is a cytosine deaminase.

80. The method of claim 79, wherein the reference protein is a reference cytosine deaminase, wherein the reference cytosine deaminase is rAPOBEC1 having a sequence shown in SEQ ID No: 64 or DddA having a sequence shown in SEQ No: 65.

81. A cytosine deaminase identified by the protein function prediction method of any one of claims 75-76.

82. A truncated cytosine deaminase comprising or consisting of a minimal functional domain of cytosine deaminase identified by the method of any one of claims 79-80.

83. Use of the cytosine deaminase of claim 81 or the truncated cytosine deaminase of claim 82 for gene editing, such as base editing, in an organism or a cell of an organism.

84. A nucleic acid molecule encoding the cytosine deaminase of any one of claims 1-28, or the fusion protein of any one of claims 29-37.

85. A cell comprising the cytosine deaminase of any one of claims 1-28, or the fusion protein of any one of claims 30-38, or the base editing system of any one of claims 39-49, or the nucleic acid molecule of claim 84.

86. A kit containing a nucleic acid construct, wherein the nucleic acid construct comprises:

    (a) a nucleic acid sequence encoding a cytosine deaminase according to any one of claims 1-28; and
    (b) a heterologous promoter driving the expression of the sequence of (a).

87. A kit containing a nucleic acid construct, wherein the nucleic acid construct comprises:

    (a) a nucleic acid sequence encoding a fusion protein according to any one of claims 30-38; and
    (b) a heterologous promoter driving the expression of the sequence of (a).

88. The kit of any one of claims 86-87, further comprising an expression construct encoding a guide RNA backbone, wherein the construct comprises a cloning site that allows cloning a nucleic acid sequence identical or complementary to a target sequence into the guide RNA backbone.

89. A composition comprising a cytosine deaminase of any one of claims 1-28, or a fusion protein of any one of claims 30-38, or a base editing system of any one of claims 39-49, or a nucleic acid molecule of claim 84.

90. The composition of claim 89, wherein the cytosine deaminase, fusion protein, base editing system or nucleic acid molecule is packaged into a virus, a virus-like particle, a virosome, a liposome, a vesicle, an exosome, a liposome nanoparticle (LNP).

91. The composition of claim 90, wherein the virus is an adeno-associated virus (AAV) or a recombinant adeno-associated virus (rAAV).

92. The composition of claims 89-91, which is a pharmaceutical composition and further comprises a pharmaceutically acceptable carrier.

Figure 1

Figure 2

Figure 3

## OsACC-T1

| | -19GCC | -18CCT | -12GCA | -8TCC | -7CCT | -5TCT | -3TCA | 1GCA | 6GCA | 8ACT | 10TCA | 15GCG | 19TCT |
|---|---|---|---|---|---|---|---|---|---|---|---|---|---|
| PBE | 0.06 | 0 | 0.21 | 0 | 0 | 0.38 | 1.26 | 0 | 1.04 | 2.42 | 5.46 | 0.17 | 0 |
| NO.2-1479 | 0.06 | 0 | 0.25 | 0 | 0 | 0 | 0 | 0 | 15.25 | 0 | 0.40 | 0.15 | 0 |
| NO.76 | 0.06 | 0 | 0.19 | 0 | 0 | 0 | 0 | 5.67 | 29.58 | 2.98 | 5.96 | 0.61 | 0 |
| NO.2-1146 | 0.06 | 0 | 0.24 | 0 | 0 | 0 | 0.22 | 42.10 | 18.95 | 8.06 | 9.08 | 0.20 | 0 |
| NO.64 | 0.06 | 0 | 0.21 | 0 | 0 | 0 | 0 | 10.47 | 31.46 | 3.33 | 3.50 | 0.14 | 0 |
| NO.55 | 0.20 | 0 | 0.47 | 2.00 | 1.34 | 3.84 | 9.76 | 28.30 | 38.44 | 19.17 | 40.17 | 0.31 | 0 |
| NO.69 | 0.08 | 0 | 0.23 | 0 | 0 | 0.28 | 4.66 | 26.65 | 19.61 | 18.52 | 32.56 | 0.88 | 0 |
| NO.57 | 0.05 | 0 | 0.26 | 1.16 | 0.19 | 5.09 | 4.05 | 30.64 | 45.32 | 26.71 | 22.86 | 1.11 | 0 |
| NO.2-1429 | 0.07 | 0 | 0.21 | 0 | 0.02 | 0.58 | 2.93 | 23.11 | 52.19 | 45.02 | 11.50 | 0.15 | 0 |
| A3A | 0.05 | 0 | 0.45 | 0.51 | 0.69 | 3.51 | 6.68 | 8.10 | 13.73 | 12.51 | 14.57 | 0.60 | 0 |
| WT | 0.07 | 0 | 0.23 | 0 | 0 | 0 | 0 | 0 | 0.01 | 0 | 0 | 0.12 | 0 |

Figure 4

**OsCDC48-T2**

| | -4TCC | -3GCT | 3ACC | 4CCA | 7GCC | 8CCA | 10GCG | 13TCT | 17GCG | 19GCC |
|---|---|---|---|---|---|---|---|---|---|---|
| PBE | 0 | 0 | 4.47 | 10.68 | 1.94 | 0.87 | 0.01 | 5.17 | 0.12 | 0 |
| NO.2-1479 | 0 | 0 | 0.01 | 18.78 | 0.01 | 2.06 | 6.38 | 0 | 0.06 | 0 |
| NO.76 | 0 | 0 | 19.98 | 24.09 | 5.66 | 1.51 | 0.71 | 1.46 | 0.10 | 0.02 |
| NO.2-1146 | 0 | 0 | 28.16 | 36.31 | 12.50 | 22.22 | 0 | 0 | 0.12 | 0 |
| NO.64 | 0 | 0 | 40.13 | 42.18 | 8.70 | 2.15 | 0.01 | 0.02 | 0.19 | 0.03 |
| NO.55 | 1.45 | 1.45 | 43.18 | 47.88 | 35.91 | 32.61 | 7.99 | 18.59 | 1.51 | 0.03 |
| NO.69 | 0.02 | 0.32 | 52.18 | 59.50 | 24.22 | 36.48 | 24.73 | 2.59 | 0.10 | 0.01 |
| NO.57 | 0.68 | 0.01 | 55.20 | 60.29 | 46.15 | 44.78 | 3.64 | 44.34 | 0.98 | 0.01 |
| NO.2-1429 | 0 | 0.55 | 23.23 | 51.88 | 52.22 | 48.77 | 10.41 | 0.65 | 0.14 | 0.01 |
| A3A | 0.35 | 0.01 | 0.31 | 3.41 | 3.44 | 3.04 | 3.31 | 2.38 | 0.14 | 0.01 |
| WT | 0 | 0 | 0 | 0 | 0 | 0 | 0.01 | 0.01 | 0.12 | 0.02 |

Figure 5

**OsACC-T1**

Figure 6

## OsCDC48-T2

Figure 7

Figure 8

**domain sequence of deaminase
in InterPro database**

searching protein sequence
in NCBI database

**full length of gene
encoding deaminase**

scan the domain
by hmmscan

structure
prediction

**re-annotated deaminase**

**for structure clustering
(no editing activity generally)**

testing
activity

**synthesized deaminase included
complement secondary structure**

**for functional validation
and new deaminase discovery**

Figure 9

8 17  1  3 16  5  8  15 10 14  12  11  3  13  9  2  6  7  4  5 10

A_deamin (1)
AICARFT_IMPCHas (2)
APOBEC (3)
Bd3614-deam (4)
dCMP_cyt_deam (5)
FdhD-NarQ (6)
Inv-AAD (7)
LmjF365940-deam (8)
LpxI_C (9)
MafB19-deam (10)
Pput2613-deam (11)
SCP1.201-deam (12)
TM1506 (13)
Toxin-deaminase (14)
XOO_2897-deam (15)
YwqJ-deaminase (16)
JAB(outgroup) (17)

0
0.1
0.2
0.3
0.4
0.5
0.6
0.7
0.8
0.9
1

Figure 10

A_deamin (1)
AICARFT_IMPCHas (2)
APOBEC (3)
Bd3614-deam (4)
dCMP_cyt_deam (5)
FdhD-NarQ (6)
Inv-AAD (7)
LmjF365940-deam (8)
LpxI_C (9)
MafB19-deam (10)
Pput2613-deam (11)
SCP1.201-deam (12)
TM1506 (13)
Toxin-deaminase (14)
XOO_2897-deam (15)
YwqJ-deaminase (16)
JAB(outgroup) (17)

Figure 11A

Figure 11B

**A**

LmjF365940-clade1
**LmjF365940-clade2**

**B**

APOBEC-clade1
**APOBEC-clade2**

**C**

dCMP-clade1
**dCMP-clade2**

**D**

MafB19-clade1
**MafB19-clade2**

Figure 12

Figure 13A

**B**

Predicted core
structure of DddA

**C**

Canonical structure of
Ddd deaminase

**D**

Predicted core
structure of Sdd7

**E**

Canonical structure of
Sdd deaminase

Figure 13B-E

Figure 14

Figure 15

| | HsHEK2 | | | | | | | HsWFS1 | | | | | | |
|---|---|---|---|---|---|---|---|---|---|---|---|---|---|---|
| | GAA•A•AAAG•ATAGA•TG•GGG CTTGTGTTTCGTATCTGACGCCC | | | | | | | •AG•AGTATGGTG•G•TGTGCGG GTCGTCATACCACGCGACACGCC | | | | | | |
| HsHEK2 | 0.2 | 75.5 | 1.6 | 63.4 | 0.1 | 2.2 | 0.1 | 0.4 | 54.7 | 0.1 | 0.1 | 0.2 | 0.2 | 0.2 |
| HsWFS1 | 0.3 | 71.2 | 0.4 | 30.9 | 0.3 | 0.3 | 0.2 | 0.3 | 4.0 | 0.4 | 0.3 | 0.4 | 0.3 | 0.3 |
| | SCP015 | SCP016 (Sdd7) | SCP020 | SCP044 (Sdd9) | SCP090 | SCP183 (Sdd59) | SCP278 | SCP287 | SCP315 | SCP341 | SCP353 | SCP357 | SCP372 | Untreated |

Figure 16A

| | SCP015 | SCP016 (Sdd7) | SCP020 | SCP044 (Sdd9) | SCP090 | SCP183 (Sdd59) | SCP278 | SCP287 | SCP300 | SCP302 | SCP303 | SCP315 | SCP323 | SCP341 | SCP353 | SCP357 | SCP372 | SCP035 (DddA) | Untreated |
|---|---|---|---|---|---|---|---|---|---|---|---|---|---|---|---|---|---|---|---|
| HsJAK2 | 0 | 0.7 | 0 | 0.8 | 0 | 0.1 | 0 | 0 | 1.6 | 1.4 | 2.4 | 0 | 1.6 | 0 | 0.1 | 0.2 | 0 | 26.7 | 1.8 |
| HsSIRT6 | 0.3 | 0.7 | 0 | 0.8 | 0 | 0.2 | 0.2 | 0 | 1.2 | 1.0 | 1.2 | 0.2 | 1.1 | 0 | 0 | 0 | 0 | 6.3 | 0.9 |

Figure 16B

Figure 17

Figure 18

Figure 19

Figure 20

Figure 21

EP 4 491 723 A1

**A**

*HsEMX1*

**B**

*HsHEK2*

**C**

*HsWFS1*

Figure 22

Figure 23

Figure 24

Figure 25A

Figure 25B

Figure 26

Figure 27

Figure 28

Figure 29

**A**

Figure 30A

**B**

Figure 30B

Figure 30C

Figure 30D

**E**

| Repeat | Deaminase | Target gene | No. of transgenic plants | No. of mutants | Editing efficiency |
|--------|-----------|-------------|--------------------------|----------------|---------------------|
| Exp. 1 | mini-Sdd7 | GmPPO2 | 40 | 7 | 17.5% |
| | APOBEC3A | | 31 | 0 | 0.0% |
| Exp. 2 | mini-Sdd7 | GmPPO2 | 30 | 11 | 36.7% |
| | APOBEC3A | | 45 | 0 | 0.0% |
| Exp. 3 | mini-Sdd7 | GmPPO2 | 7 | 3 | 42.9% |
| | APOBEC3A | | 8 | 0 | 0.0% |

**G**

**F**

GmPPO2

wild-type: 5'- CATAAGCGCTATATTGTGAAAAATGGGGCA -3'
H K R Y I V K N G A

edited: 5'- CATAAGTGCTATATTGTGAAAAATGGGGCA -3'
H K C Y I V K N G A

Figure 30E-G

**A**

**B**

| Deaminase | Target gene | No. of mutants | No. of transgenic rice plants | C-to-T frequency(%) | Heterozygous/ Homozygous |
|---|---|---|---|---|---|
| mini-Sdd7 | | 222 | 298 | 74.50 | 64/145 |
| | *OsODEV* | | | | |
| APOBEC3A | | 127 | 279 | 45.52 | 28/75 |
| mini-Sdd7 | | 161 | 246 | 65.45 | 37/117 |
| | *OsACC1* | | | | |
| APOBEC3A | | 10 | 52 | 19.23 | 2/7 |
| mini-Sdd7 | | 185 | 220 | 84.09 | 10/170 |
| | *OsAAT* | | | | |
| APOBEC3A | | 82 | 102 | 80.39 | 9/70 |

Figure 31

Figure 32

<table>
<tr><td colspan="2">**INTERNATIONAL SEARCH REPORT**</td><td>International application No.<br>**PCT/CN2023/080052**</td></tr>
</table>

**A.    CLASSIFICATION OF SUBJECT MATTER**

C12N9/78(2006.01)i;   C07K19/00(2006.01)i;   C12N15/113(2010.01)i;   C12N7/00(2006.01)i;   C12N5/10(2006.01)i;
C12N15/79(2006.01)i

According to International Patent Classification (IPC) or to both national classification and IPC

**B.    FIELDS SEARCHED**

Minimum documentation searched (classification system followed by classification symbols)

IPC:  C12N C07K

Documentation searched other than minimum documentation to the extent that such documents are included in the fields searched

Electronic data base consulted during the international search (name of data base and, where practicable, search terms used)

CNABS; CNTXT; CJFD; WPABS; WPABSC; ENTXTC; ENTXT; DWPI; CNKI; 万方, WANFANG; ISI Web of Science;
STN; GenBank; EMBL; 中国专利生物序列检索系统, China Patent Biological Sequence Search System: 中国科学院遗传与
发育生物学研究所, 高彩霞, 胞嘧啶脱氨酶, CRISPR效应蛋白, 尿嘧啶DNA糖基化酶抑制, 核定位序列, 向导RNA, 腺相关
病毒, 接头, 基于序列1, 4, 26, 64, 65的检索, search based on sequence 1, 4, 26, 64, 65, INSTITUTE OF GENETICS AND
DEVELOPMENTAL BIOLOGY, CHINESE ACADEMY OF SCIENCES, AID, CAS9, UGI, NLS, sgRNA, AAV, rAAV, linker

**C.    DOCUMENTS CONSIDERED TO BE RELEVANT**

| Category* | Citation of document, with indication, where appropriate, of the relevant passages | Relevant to claim No. |
|---|---|---|
| X | CN 102482639 A (MEDICAL RESEARCH COUNCIL) 30 May 2012 (2012-05-30)<br>claims 100-109, description paragraphs [0053], [0085] | 1, 3-9 |
| Y | CN 102482639 A (MEDICAL RESEARCH COUNCIL) 30 May 2012 (2012-05-30)<br>claims 100-109, description paragraphs [0053], [0085] | 29-63, 84-92 |
| Y | CN 109295053 A (SHANGHAI INSTITUTES FOR BIOLOGICAL SCIENCES, CHINESE<br>ACADEMY OF SCIENCES) 01 February 2019 (2019-02-01)<br>claims 10-15, description paragraphs [0077], [0081]-[0082] | 29-63, 84-92 |

☐ Further documents are listed in the continuation of Box C.       ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **23 May 2023** | **05 June 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2023/080052** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter.*1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

## INTERNATIONAL SEARCH REPORT

| International application No. |
| --- |
| **PCT/CN2023/080052** |

**Box No. III    Observations where unity of invention is lacking (Continuation of item 3 of first sheet)**

This International Searching Authority found multiple inventions in this international application, as follows:

Invention 1: the solutions relating to the cytosine deaminase shown in SEQ ID NO: 1 in claims 1, 3-9, 29-63 and 84-92

Invention 2: the solutions relating to the cytosine deaminase shown in SEQ ID NO: 63 in claims 1, 3-9, 29-63 and 84-92

Inventions 3-15: the solutions relating to the cytosine deaminase shown in SEQ ID NOs: 28-40 in claims 1-8, 10-13, 29-63 and 84-92

Inventions 16-41: the solutions relating to the cytosine deaminase shown in SEQ ID NOs: 2-18 and 41-49 in claims 1-8, 14-17, 29-63 and 84-92

Inventions 42-47: the solutions relating to the cytosine deaminase shown in SEQ ID NOs: 50-55 in claims 1-8, 18-23, 29-63 and 84-92

Inventions 48-51: the solutions relating to the cytosine deaminase shown in SEQ ID NOs: 19, 56, 57 and 58 in claims 1-8, 24, 29-63 and 84-92

Inventions 52-53: the solutions relating to the cytosine deaminase shown in SEQ ID NOs: 20 and 21 in claims 1-8, 25, 29-63 and 84-92

Invention 54: the solutions relating to the cytosine deaminase shown in SEQ ID NO: 22 in claims 1-8, 26, 29-63 and 84-92

Inventions 55-60: the solutions relating to the cytosine deaminase shown in SEQ ID NOs: 23, 24 and 59-62 in claims 1-8, 27, 29-63 and 84-92

Inventions 61-62: the solutions relating to the cytosine deaminase shown in SEQ ID NO: 74 or 75 in claims 1-8, 28-63 and 84-92

Invention 63: the solutions relating to a protein clustering method in claims 64-71

Invention 64: the solutions relating to a protein function prediction method based on a three-dimensional structure in claims 72-76

Invention 65: the solutions relating to a method for identifying a minimal functional domain of a protein on the basis of a three-dimensional structure in claims 77-80

Invention 66: the solution relating to a cytosine deaminase identified using the protein function prediction method of either of claims 75-76 in claim 81

Invention 67: the solutions relating to a truncated cytosine deaminase in claims 82-83

The present International Authority is of the opinion that the claims comprise 67 inventions. Inventions 1-62 and inventions 63-67 do not have a same or corresponding special technical feature that defines a contribution which the inventions make over the prior art. The same or corresponding technical feature of the 67 inventions is cytosine deaminase. However, the prior art (e.g., CN 102482639 A) discloses an activation-induced cytidine deaminase (AID) mutant. Therefore, the 67 inventions do not have a same or corresponding special technical feature that defines a contribution which the inventions make over the prior art, do not have any technical relationship therebetween, do not fall within a single general inventive concept, and thus do not meet the requirement of unity of invention and do not comply with PCT Rule 13.1.

Form PCT/ISA/210 (continuation of first sheet) (July 2022)

## INTERNATIONAL SEARCH REPORT

| | |
|---|---|
| International application No. | |
| | **PCT/CN2023/080052** |

**Box No. III   Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **The solutions relating to the cytosine deaminase shown in SEQ ID NO: 1 in claims 1, 3-9, 29-63 and 84-92**

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

| INTERNATIONAL SEARCH REPORT | | | | International application No. | | | |
|---|---|---|---|---|---|---|---|
| Information on patent family members | | | | PCT/CN2023/080052 | | | |

| Patent document cited in search report | | | Publication date (day/month/year) | Patent family member(s) | | | Publication date (day/month/year) |
|---|---|---|---|---|---|---|---|
| CN | 102482639 | A | 30 May 2012 | JP | 2012522501 | A | 27 September 2012 |
| | | | | JP | 5683566 | B2 | 11 March 2015 |
| | | | | US | 2012151613 | A1 | 14 June 2012 |
| | | | | US | 9683226 | B2 | 20 June 2017 |
| | | | | EP | 2414507 | A1 | 08 February 2012 |
| | | | | EP | 2414507 | B1 | 02 July 2014 |
| | | | | WO | 2010113039 | A1 | 07 October 2010 |
| | | | | CA | 2757178 | A1 | 07 October 2010 |
| | | | | CA | 2757178 | C | 19 May 2020 |
| | | | | RU | 2011144569 | A | 10 May 2013 |
| | | | | RU | 2537264 | C2 | 27 December 2014 |
| | | | | AU | 2010230985 | A1 | 06 October 2011 |
| | | | | AU | 2010230985 | B2 | 24 September 2015 |
| | | | | CN | 102482639 | B | 06 January 2016 |
| CN | 109295053 | A | 01 February 2019 | EP | 3712272 | A1 | 23 September 2020 |
| | | | | EP | 3712272 | A4 | 13 October 2021 |
| | | | | US | 2021355508 | A1 | 18 November 2021 |
| | | | | WO | 2019020007 | A1 | 31 January 2019 |
| | | | | CA | 3106738 | A1 | 31 January 2019 |

Form PCT/ISA/210 (patent family annex) (July 2022)

## REFERENCES CITED IN THE DESCRIPTION

**Patent documents cited in the description**

- WO 2015089465 A1
- US 2014070135 W
- WO 2016205711 A1
- US 2016038181 W
- WO 2018141835 A1
- EP 2018052491 W
- WO 2020191234 A1
- US 2020023713 W
- WO 2020191233 A1
- US 2020023712 W
- WO 2019079347 A1
- US 2018056146 W
- WO 2021155065 A1
- US 2021015580 W

**Non-patent literature cited in the description**

- **IYER, L. M.** ; **ZHANG, D.** ; **ROGOZIN, I. B.** ; **ARAVIND, L.** Evolution of the deaminase fold and multiple origins of eukaryotic editing and mutagenic nucleic acid deaminases from bacterial toxin systems.. *Nucleic acids research*, 2011, vol. 39 (22), 9473-9497
- Computational Molecular Biology. Oxford University Press, 1988
- Biocomputing: Informatics and Genome Projects. Academic Press, 1993
- Computer Analysis of Sequence Data. Humana Press, 1994
- **VON HEINJE, G.** Sequence Analysis in Molecular Biology. Academic Press, 1987
- Sequence Analysis Primer. M Stockton Press, 1991
- **CARRILLO, H.** ; **LIPMAN, D.** *SIAM J Applied Math*, 1988, vol. 48, 1073
- **WATSON et al.** Molecular Biology of the Gene. The Benjamin/Cummings Pub. co., 1987, 224
- **JOHN JUMPER**. Highly Accurate Protein Structure Prediction with AlphaFold. *Nature*, 2021, vol. 596 (7873), 583-89
- **C. P. KURTZMAN** ; **JACK W. FELL** ; **T. BOEKHOUT**. The Yeasts: A Taxonomic Study. Elsevier, 2011
- **NAKAMURA Y et al.** Codon usage tabulated from the international DNA sequence databases: status for the year 2000. *Nucl. Acids Res.*, 2000, vol. 28, 292
- **KOBLAN, L. W** ; **DOMAN, J. L** ; **WILSON, C.** ; **LEVY, J. M.** ; **TAY, T.** ; **NEWBY, G. A** ; **MAIANTI, J. P** ; **RAGURAM, A.** ; **LIU, D. R.** Improving cytidine and adenine base editors by expression optimization and ancestral reconstruction. *Nat. Biotechnol.*, 2018, vol. 36, 843-846
- **ZHANG, YANG** ; **JEFFREY SKOLNICK.** Scoring function for automated assessment of protein structure template quality.. *Proteins*, 2004, vol. 57 (4), 702-710
- **ZONG, Y. et al.** *Nat. Biotechnol.*, 2017, vol. 35, 438-440
- **ZHANG, YANG** ; **JEFFREY SKOLNICK.** Scoring function for automated assessment of protein structure template quality. *Proteins*, 2004, vol. 57 (4), 702-710
- **B. Y. MOK** ; **M. H. DE MORAES** ; **J. ZENG** ; **D. E. BOSCH** ; **A. V. KOTRYS** ; **A. RAGURAM** ; **F. HSU** ; **M. C. RADEY** ; **S. B. PETERSON** ; **V. K. MOOTHA**. A bacterial cytidine deaminase toxin enables CRISPR-free mitochondrial base editing.. *Nature*, 2020, vol. 583, 631-637
- **B. Y. MOK** ; **A. V. KOTRYS** ; **A. RAGURAM** ; **T. P. HUANG** ; **V. K. MOOTHA** ; **D. R. LIU**. CRISPR-free base editors with enhanced activity and expanded targeting scope in mitochondrial and nuclear DNA.. *Nat. Biotechnol.*, vol. 40, 1378-1387
- **L. W. KOBLAN** ; **J. L. DOMAN** ; **C. WILSON** ; **J. M. LEVY** ; **T. TAY** ; **G. A. NEWBY** ; **J. P. MAIANTI** ; **A. RAGURAM** ; **D. R. LIU**. Improving cytidine and adenine base editors by expression optimization and ancestral reconstruction.. *Nat. Biotechnol.*, 2018, vol. 36, 843-846
- **Y. ZONG** ; **Q. SONG** ; **C. LI** ; **S. JIN** ; **D. ZHANG** ; **Y. WANG** ; **J.-L. QIU** ; **C. GAO**. Efficient C-to-T base editing in plants using a fusion of nCas9 and human APOBEC3A.. *Nat. Biotechnol.*, 2018, vol. 36, 950-953
- **Q. LIN** ; **Z. ZHU** ; **G. LIU** ; **C. SUN** ; **D. LIN** ; **C. XUE** ; **S. LI** ; **D. ZHANG** ; **C. GAO** ; **Y. WANG**. Genome editing in plants with MAD7 nuclease.. *J. Genet. Genomics*, 2021, vol. 48, 444-451

- **XI XIANG** ; **KUNLI QU** ; **XUE LIANG** ; **XIAOGUANG PAN** ; **JUN WANG** ; **PENG HAN** ; **ZHANYING DONG** ; **LIJUN LIU** ; **JIAYAN ZHONG** ; **TAO MA**. Massively parallel quantification of CRISPR editing in cells by TRAP-seq enables better design of Cas9, ABE, CBE gRNAs of high efficiency and accuracy. *bioRxiv*, 20 May 2020, 103614